# EUROPEAN PATENT APPLICATION

(11) **EP 2 589 294 A1**
(43) Date of publication of application: **08.05.2013**
(21) Application number: 11187513.4
(22) Date of filing: 02.11.2011
(51) Int. Cl.: A01P 5/00, A01N 43/08, A01N 43/10, A01N 43/18, A01N 43/36, A01N 43/40, A01N 43/56, A01N 43/60, A01N 43/707, A01N 43/76, A01N 43/78, A01N 43/80, A01N 43/828, A01N 43/84, A61K 31/435

(54) **Compounds with nematicidal activity**

(71) Applicant: Bayer CropScience AG, 40789 Monheim (DE)
(72) Inventor: The designation of the inventor has not yet been filed

(57) **Abstract**

The present invention relates to the use of predominantly known pyridyl carboxamide derivatives as nematicides, compositions containing such compounds and methods for the control of nematodes.

## Description

The present invention relates to the use of predominantly known pyridyl ethyl carboxamide derivatives as nematicides, compositions containing such compounds and methods for the control of nematodes.

Nematodes cause a substantial loss in agricultural product including food and industrial crops and are combated with chemical compounds having nematicidal activity. To be useful in agriculture these compounds should have a high activity, a broad spectrum activity against different strains of nematodes and should not be toxic to non-target organisms.

The compounds described below are predominantly known from WO 2004/074280 A1 (later, e.g. in the Tables, referred to as P9), WO2005/0058833 A2 (P10), WO 2005/085238 A1 (P11), WO 2005/103006 A1 (P12), WO 2006/122955 A1 (P13), WO 2006/008194 A1 (P14) and WO 2006/008193 A1 (P15). There it is stated that they can be used as fungicides.

Surprisingly it has now been found that these compounds exhibit nematicidal activity and can therefore be used to control nematodes.

It was also found that the compounds of formula (I) exhibit activity against bacteria and viruses and can be used as bactericides and as virucides.

Accordingly, the present invention relates to the use of a compound of formula (I) 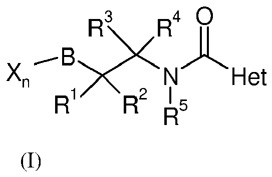 wherein
- B: represents 2-pyridyl, 3-pyridyl, or 4-pyridyl,
- X: is selected from the group consisting of halogen, nitro, cyano, hydroxy, amino, -SH, -SF₅, -CHO, -OCHO, -NHCHO, -COOH, -CONH₂, -CONH(OH), -OCONH₂, (hydroxyimino)-C₁-C₆-alkyl, C₁-C₈-alkyl, C₁-C₈-halogenoalkyl having 1 to 5 halogen atoms, C₂-C₈-alkenyl, C₂-C₈-alkynyl, C₁-C₈-alkylamino, di-(C₁-C₈-alkyl)amino, C₁-C₈-alkoxy, C₁-C₈-halogenoalkoxy having 1 to 5 halogen atoms,, C₂-C₈-alkenyloxy, C₂-C₈-halogenoalkenyloxy having 1 to 5 halogen atoms, C₃-C₈-alkynyloxy, C₃-C₈-halogenoalkynyloxy having 1 to 5 halogen atoms, C₃-C₈-cycloalkyl, C₃-C₈-halogenocycloalkyl having 1 to 5 halogen atoms, C₁-C₈-alkylcarbonyl, C₁-C₈-halogenoalkylcarbonyl having 1 to 5 halogen atoms, -CONH(C₁-C₈-alkyl), -CON(C₁-C₈-alkyl)₂, -CONH(OC₁-C₈-alkyl), -CON(OC₁-C₈-alkyl)(C₁-C₈-alkyl), C₁-C₈-alkoxycarbonyl, C₁-C₈-halogenoalkoxycarbonyl having 1 to 5 halogen atoms, C₁-C₈- alkylcarbonyloxy, C₁-C₈-halogenoalkylcarbonyloxy having 1 to 5 halogen atoms, C₁-C₈-alkylcarbonylamino, C₁-C₈-halogenoalkylcarbonylamino having 1 to 5 halogen atoms, -OCONH(C₁-C₈-alkyl), -OCON(C₁-C₈-alkyl)₂, -OCONH(OC₁-C₈-alkyl), -OCO(OC₁-C₈-alkyl), -S-C₁-C₈-alkyl, -S-C₁-C₈-halogenoalkyl having 1 to 5 halogen atoms, -S(O)-C₁-C₈-alkyl, -S(O)-C₁-C₈-halogenoalkyl having 1 to 5 halogen atoms, -S(O)₂-C₁-C₈-alkyl, -S(O)₂-C₁-C₈-halogenoalkyl having 1 to 5 halogen atoms, (C₁-C₆-alkoxyimino)-C₁-C₆-alkyl, (C₂-C₆-alkenyloxyimino)-C₁-C₆-alkyl, (C₃-C₆-alkynyloxyimino)-C₁-C₆-alkyl, (benzyloxyimino)-C₁-C₆-alkyl, benzyloxy, -S-benzyl, benzylamino, phenoxy, -S-phenyl and phenylamino,
- n: is 1, 2, 3 or 4 and if n is 2, 3, or 4, then the substituents X may be the same or different,
- R¹ and R²: are the same or different and are selected from the group consisting of hydrogen, halogen, cyano, hydroxy, amino, -SH, -CHO, -OCHO, -NHCHO, -COOH, -CONH₂, -CONH(OH), -OCONH₂, a (hydroxyimino)-C₁-C₆-alkyl group, C₁-C₆-alkyl, C₂-C₆-alkenyl, C₂-C₆-alkynyl, C₁-C₆-alkylamino, di-(C₁-C₆-alkyl)amino, C₁-C₆-alkoxy, C₁-C₆-halogenoalkyl having 1 to 5 halogen atoms, C₁-C₆-halogenoalkoxy having 1 to 5 halogen atoms,, C₂-C₆-alkenyloxy, C₂-C₆-halogenoalkenyloxy having 1 to 5 halogen atoms, C₃-C₆-alkynyloxy, C₃-C₆-halogenoalkynyloxy having 1 to 5 halogen atoms, C₃-C₆-cycloalkyl, C₃-C₆-halogenocycloalkyl having 1 to 5 halogen atoms, C₃-C₆-cycloalkyl-C₁-C₆-alkyl, C₃-C₆-halogenocycloalkyl-C₁-C₆-alkyl having 1 to 5 halogen atoms, C₁-C₆-alkylcarbonyl, C₁-C₆-halogenoalkylcarbonyl having 1 to 5 halogen atoms, -CONH(C₁-C₆-alkyl), -CON(C₁-C₆-alkyl)₂, -CONH(OC₁-C₆-alkyl), -CON(OC₁-C₆-alkyl)(C₁-C₆-alkyl), C₁-C₆-alkoxycarbonyl, a C₁-C₆-halogenoalkoxycarbonyl having 1 to 5 halogen atoms, -OC(O)-C₁-C₆-alkyl, -OC(O)-C₁-C₆-halogenoalkyl having 1 to 5 halogen atoms, -NHC(O)-C₁-C₆-alkyl, -NHC(O)-C₁-C₆-halogenoalkyl having 1 to 5 halogen atoms, -OCONH(C₁-C₆-alkyl), -OCON(C₁-C₆-alkyl)₂, -OCONH(OC₁-C₆-alkyl), OCO(OC₁-C₆-alkyl), -S-C₁-C₆-alkyl, -S-C₁-C₆-halogenoalkyl having 1 to 5 halogen atoms, -S(O)-C₁-C₆-alkyl, -S(O)-C₁-C₆-halogenoalkyl having 1 to 5 halogen atoms, -S(O)₂-C₁-C₆-alkyl, -S(O)₂-C₁-C₆-halogenoalkyl having 1 to 5 halogen atoms, benzyl, benzyloxy, -S-benzyl, -S(O)-benzyl, -S(O)₂-benzyl, benzylamino, phenoxy, -S-phenyl, -S(O)-phenyl, -S(O)₂-phenyl, phenylamino, phenylcarbonylamino, 2,6-dichlorophenyl-carbonylamino, 2-chlorophenyl-carbonylamino and phenyl,
- R³ and R⁴: are the same or different and are selected from the group consisting of hydrogen, halogen, cyano, hydroxy, amino, -SH, -CHO, -COOH, -CONH₂, -CONH(OH), -OCONH₂, (hydroxyimino)-C₁-C₆-alkyl, C₁-C₆-alkyl, C₂-C₆-alkenyl, C₂-C₆-alkynyl, C₁-C₆-alkylamino, di-(C₁-C₆-alkyl)amino, C₁-C₆-alkoxy, hydroxy-C₁-C₆-alkyl, C₁-C₆-alkoxy-C₁-C₆-alkyl, C₁-C₆-halogenoalkyl having 1 to 5 halogen atoms, C₁-C₆-halogenoalkoxy having 1 to 5 halogen atoms, C₂-C₆-alkenyloxy, C₂-C₆-halogenoalkenyloxy having 1 to 5 halogen atoms, C₃-C₆-alkynyloxy, C₃-C₆-halogenoalkynyloxy having 1 to 5 halogen atoms, C₃-C₆-cycloalkyl, C₃-C₆-halogenocycloalkyl having 1 to 5 halogen atoms, C₁-C₆-alkylcarbonyl, C₁-C₆-halogenoalkylcarbonyl having 1 to 5 halogen atoms, -CONH(C₁- C₆-alkyl), -CON(C₁-C₆-alkyl)₂, -CONH(OC₁-C₆-alkyl), -CON(OC₁-C₆-alkyl)(C₁-C₆-alkyl), C₁-C₆-alkoxycarbonyl, C₁-C₆-halogenoalkoxycarbonyl having 1 to 5 halogen atoms, -OC(O)-C₁-C₆-alkyl, -OC(O)-C₁-C₆-halogenoalkyl having 1 to 5 halogen atoms, -NHC(O)-C₁-C₆-alkyl, -NHC(O)-C₁-C₆-halogenoalkyl having 1 to 5 halogen atoms, -OCONH(C₁-C₆-alkyl), -OCON(C₁-C₆-alkyl)₂, -OCONH(OC₁-C₆-alkyl), OCO(OC₁-C₆-alkyl), -S-C₁-C₆-alkyl, -S-C₁-C₆-halogenoalkyl having 1 to 5 halogen atoms, -S(O)-C₁-C₆-alkyl, -S(O)-C₁-C₆-halogenoalkyl having 1 to 5 halogen atoms, -S(O)₂-C₁-C₆-alkyl, -S(O)₂-C₁-C₆-halogenoalkyl having 1 to 5 halogen atoms, benzyl, benzyloxy, -S-benzyl, -S(O)-benzyl, -S(O)₂-benzyl, benzylamino, phenoxy, -S-phenyl, -S(O)-phenyl, -S(O)₂-phenyl, phenylamino, phenylcarbonylamino, 2-chlorophenyl-carbonylamino, 2,6-dichlorophenyl-carbonylamino and phenyl,
- R⁵: is selected from the group consisting of hydrogen, cyano, -CHO, -OH, C₁-C₆-alkyl, C₁-C₆-halogenoalkyl having 1 to 5 halogen atoms, C₁-C₆-alkoxy, C₁-C₆-halogenoalkoxy having 1 to 5 halogen atoms, C₃-C₇-cycloalkyl, C₃-C₇-halogenocycloalkyl having 1 to 5 halogen atoms, C₃-C₇-cycloalkyl-C₁-C₆-alkyl, -CONH(C₁-C₆-alkyl), C₂-C₆-alkenyl, C₂-C₆-alkynyl, C₁-C₆-alkoxy-C₁-C₆-alkyl, C₃-C₇-cycloalkyl-C₁-C₆-alkyl, cyano-C₁-C₆-alkyl, amino-C₁-C₆-alkyl, C₁-C₆-alkylamino-C₁-C₆-alkyl, di-(C₁-C₆-alkyl)amino-C₁-C₆-alkyl, C₁-C₆-alkylcarbonyl, C₁-C₆-halogenoalkylcarbonyl having 1 to 5 halogen atoms, C₁-C₆-alkoxycarbonyl, C₁-C₆-benzyloxycarbonyl, C₁-C₆-alkoxy-C₁-C₆-alkylcarbonyl, -S-C₁-C₆-alkyl, -S-C₁-C₆-halogenoalkyl having 1 to 5 halogen atoms, -S(O)₂-C₁-C₆-alkyl, and -S(O)₂-C₁-C₆-halogenoalkyl having 1 to 5 halogen atoms,
with the proviso, that when B = 2-pyridyl, then
Het may not be pyrazinyl (Het ≠ Het-29), and
when B = 2-pyridyl and Het = pyridyl or pyrazolyl, then
at least one of R¹, R², R³, R⁴ and R⁵ is not a hydrogen atom, and
R¹ and R² may also together with the carbon atom to which they are bonded form a 3-, 4-, 5- or 6-membered carbocycle, or
R² and R³ may together with the carbon atoms to which they are bonded form a 3-, 4-, 5-, 6- or 7-membered carbocycle, and
with the further proviso, that when B = 3-pyridyl, then
R¹ and R² may also together with the carbon atom to which they are bonded form a 3-, 4-, 5- or 6-membered carbocycle, or
R³ and R⁴ may also together with the carbon atoms to which they are bonded form a 3-, 4-, 5- or 6-membered carbocycle, and
with the further proviso, that when B = 4-pyridyl, then
R¹ and R² may also together with the carbon atom to which they are bonded form a 3-, 4-, 5- or 6-membered carbocycle, or
R³ and R⁴ may also together with the carbon atoms to which they are bonded form a 3-, 4-, 5- or 6-membered carbocycle;

According to the present invention, "Het" may be a five-membered non-fused heterocyclic ring. Specific examples include the following five-membered rings:
Het represents a heterocycle of the formula (Het-1) 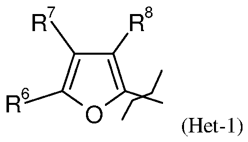 in which
   - R⁶ and R⁷: may be the same or different and are selected from the group consisting of hydrogen, halogen, amino, nitro, C₁-C₄-alkyl and C₁-C₄-halogenoalkyl having 1 to 5 halogen atoms, and
   - R⁸: is selected from the group consisting of hydrogen, halogen, nitro, C₁-C₄-alkyl and C₁-C₄-halogenoalkyl having 1 to 5 halogen atoms, or
Het represents a heterocycle of the formula (Het-2) 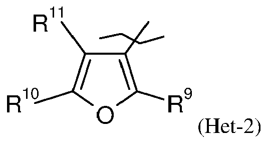 in which
   - R⁹: is selected from the group consisting of hydrogen, halogen, C₁-C₄-alkyl and C₁-C₄-halogenoalkyl having 1 to 5 halogen atoms, and
   - R¹⁰ and R¹¹: may be the same or different and are selected from the group consisting of hydrogen, halogen, amino, C₁-C₄-alkyl, C₁-C₄-halogenoalkyl having 1 to 5 halogen atoms and phenyl (optionally substituted by halogen or C₁-C₄-alkyl), or
Het represents a heterocycle of the formula (Het-3) 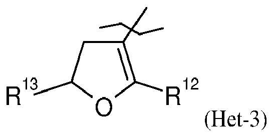 in which
   - R¹²: is selected from the group consisting of halogen, C₁-C₄-alkyl and C₁-C₄-halogenoalkyl having 1 to 5 halogen atoms, and
   - R¹³: is selected from the group consisting of hydrogen, C₁-C₄-alkyl and C₁-C₄-halogenoalkyl having 1 to 5 halogen atoms, or
Het represents a heterocycle of the formula (Het-4) 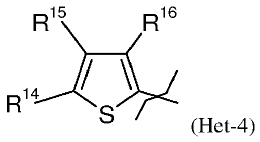 in which
   - R¹⁴ and R¹⁵: may be the same or different and are selected from the group consisting of hydrogen, halogen, C₁-C₄-alkyl, C₁-C₄-halogenoalkyl having 1 to 5 halogen atoms, -S-C₁-C₄-alkyl, -S(O)₂-C₁-C₄-alkyl, phenyl optionally substituted by halogen or C₁-C₄-alkyl and pyridyl (optionally substituted by halogen or C₁-C₄-alkyl), and
   - R¹⁶: is selected from the group consisting of hydrogen, halogen, cyano, C₁-C₄-alkyl, C₁-C₄-halogenoalkyl having 1 to 5 halogen atoms and C₁-C₄-halogenoalkoxy having 1 to 5 halogen atoms, or
Het represents a heterocycle of the formula (Het-5) 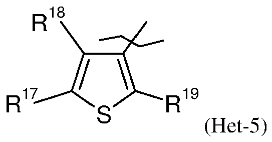 in which
   - R¹⁷ and R¹⁸: may be the same or different and are selected from the group consisting of hydrogen, halogen, C₁-C₄-alkyl, C₁-C₄-alkyloxy and C₁-C₄-halogenoalkyl having 1 to 5 halogen atoms, and
   - R¹⁹: is selected from the group consisting of hydrogen, halogen, C₁-C₄-alkyl and C₁-C₄-halogenoalkyl having 1 to 5 atoms, or
Het represents a heterocycle of the formula (Het-6) 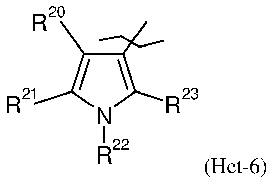 in which
   - R²⁰: is selected from the group consisting of hydrogen, halogen, cyano, C₁-C₄-alkyl and C₁-C₄-halogenoalkyl having 1 to 5 halogen atoms, and
   - R²¹ and R²³: may be the same or different and are selected from the group consisting of hydrogen, halogen, C₁-C₄-alkyl and C₁-C₄-halogenoalky having 1 to 5 halogen atoms, and
   - R²²: is selected from the group consisting of hydrogen, cyano, C₁-C₄-alkyl, C₁-C₄-halogenoalkyl having 1 to 5 halogen atoms, C₁-C₄-alkoxy-C₁-C₄-alkyl, hydroxyl-C₁-C₄-alkyl, -S(O)₂-C₁-C₄-alkyl, -S(O)₂-N(C₁-C₄-alkyl)₂, C₁-C₆-alkylcarbonyl, -S(O)₂-phenyl (optionally substituted by halogen or C₁-C₄-alkyl) and benzoyl (optionally substituted by halogen or C₁-C₄-alkyl), or
Het represent a heterocycle of the formula (Het-7) 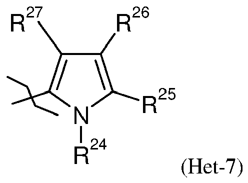 in which
   - R²⁴: is selected from the group consisting of hydrogen, cyano, C₁-C₄-alkyl, C₁-C₄-halogenoalkyl having 1 to 5 halogen atoms, C₁-C₄-alkoxy-C₁-C₄-alkyl, hydroxy-C₁-C₄-alkyl, -S(O)₂-C₁-C₄-alkyl, -S(O)₂-N(C₁-C₄-alkyl)₂, C₁-C₆-alkylcarbonyl, -S(O)₂-phenyl (optionally substituted by halogen or C₁-C₄-alkyl) and benzoyl (optionally substituted by halogen or a C₁-C₄-alkyl), and
   - R²⁵, R²⁶ and R²⁷: may be the same or different and are selected from the group consisting of hydrogen, halogen, cyano, C₁-C₄-alkyl, C₁-C₄-halogenalkyl having 1 to 5 halogen atoms and C₁-C₄-alkylcarbonyl, or
Het represents a heterocycle of the formula (Het-8) 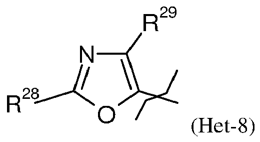 in which
   - R²⁸: is selected from the group consisting of hydrogen and C₁-C₄-alkyl, and
   - R²⁹: is selected from the group consisting of halogen, C₁-C₄-alkyl and C₁-C₄-halogenoalkyl having 1 to 5 halogen atoms, or
Het represent a heterocycle of the formula (Het-9) 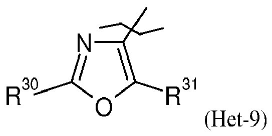 in which
   - R³⁰: is selected from the group consisting of hydrogen and C₁-C₄-alkyl, and
   - R³¹: is selected from the group consisting of halogen, C₁-C₄-alkyl, C₁-C₄-halogenoalkyl having 1 to 5 halogen atoms and phenyl (optionally substituted by halogen or C₁-C₄-alkyl), or
Het represent a heterocycle of the formula (Het-10) 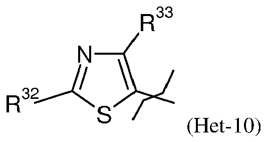 in which
   - R³²: is selected from the group consisting of hydrogen, halogen, amino, cyano, C₁-C₄-alkylamino, di-(C₁-C₄-alkyl)amino, C₁-C₄-alkyl, C₁-C₄-halogenoalkyl having 1 to 5 halogen atoms and phenyl (optionally substituted by halogen or C₁-C₄-alkyl), and
   - R³³: is selected from the group consisting of halogen, C₁-C₄-alkyl and C₁-C₄-halogenoalkyl having 1 to 5 halogen atoms, or
Het represents a heterocycle of the formula (Het-11) 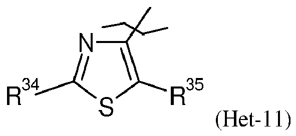 in which
   - R³⁴: is selected from the group consisting of hydrogen, halogen, amino, cyano, C₁-C₄-alkylamino, di-(C₁-C₄-alkyl)amino, C₁-C₄-alkyl and C₁-C₄-halogenoalkyl having 1 to 5 halogen atoms, and
   - R³⁵: is selected from the group consisting of halogen, C₁-C₄-alkyl and C₁-C₄-halogenoalkyl having 1 to 5 halogen atoms, or
Het represents a heterocycle of the formula (Het-12) 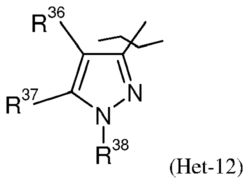 in which
   - R³⁶: is selected from the group consisting of hydrogen, halogen, cyano, nitro, C₁-C₄-alkyl, C₁-C₄-halogenoalkyl having 1 to 5 halogen atoms, C₃-C₆-cycloalkyl, C₁-C₄-alkoxy, C₁-C₄-halogenoalkoxy having 1 to 5 halogen atoms, -S-C₁-C₄-alkyl, -S-C₁-C₄-halogenoalkyl having 1 to 5 halogen atoms, aminocarbonyl and aminocarbonyl-C₁-C₄-alkyl, and
   - R³⁷: ist selected from the group consisting of hydrogen, halogen, cyano, nitro, C₁-C₄-alkyl, C₁-C₄-alkoxy and -S-C₁-C₄-alkyl, and
   - R³⁸: is selected from the group consisting of hydrogen, phenyl, C₁-C₄-alkyl, C₁-C₄-halogenoalkyl having 1 to 5 halogen atoms, hydroxy-C₁-C₄-alkyl, C₂-C₆-alkenyl, C₃-C₆-cycloalkyl, C₁-C₄-alkylthio-C₁-C₄-alkyl, C₁-C₄-halogenoalkylthio-C₁-C₄-alkyl having 1 to 5 halogen atoms, C₁-C₄-alkoxy-C₁-C₄-alkyl and C₁-C₄-halogenoalkoxy-C₁-C₄-alkyl having 1 to 5 halogen atoms, or
Het represents a heterocycle of the formula (Het-13) 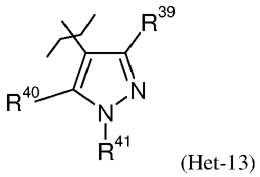 in which
   - R³⁹: is selected from the group consisting of hydrogen, halogen, cyano, nitro, C₁-C₄-alkyl, C₁-C₄-halogenoalkyl having 1 to 5 halogen atoms, C₃-C₆-cycloalkyl, C₁-C₄-alkoxy, C₁-C₄-halogenoalkoxy having 1 to 5 halogen atoms, -S-C₁-C₄-alkyl, -S-C₁-C₄-halogenoalkyl having 1 to 5 atoms, aminocarbonyl and aminocarbonyl-C₁-C₄-alkyl, and
   - R⁴⁰: is selected from the group consisting of hydrogen, halogen, cyano, C₁-C₄-alkyl, C₁-C₄-alkoxy, C₁-C₄-halogenoalkoxy having 1 to 5 halogen atoms and -S-C₁-C₄-alkyl, and
   - R⁴¹: is selected from the group consisting of hydrogen, C₁-C₄-alkyl, C₁-C₄-halogenoalkyl having 1 to 5 halogen atoms, hydroxy-C₁-C₄-alkyl, C₂-C₆-alkenyl, C₃-C₆-cycloalkyl, C₁-C₄-alkylthio-C₁-C₄-alkyl, C₁-C₄-halogenoalkylthio-C₁-C₄-alkyl having 1 to 5 halogen atoms, C₁-C₄-alkoxy-C₁-C₄-alkyl, C₁-C₄- halogenoalkoxy-C₁-C₄-alkyl having 1 to 5 halogen atoms and phenyl (optionally substituted by halogen, C₁-C₄-alkyl, C₁-C₄-alkoxy-C₁-C₄-alkyl or nitro), or
Het represents a heterocycle of the formula (Het-14) 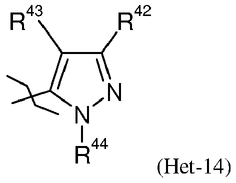 in which
   - R⁴²: is selected from the group consisting of hydrogen, halogen, cyano, nitro, C₁-C₄-alkyl, C₁-C₄-halogenoalkyl having 1 to 5 halogen atoms, C₃-C₆-cycloalkyl, C₁-C₄-alkoxy, C₁-C₄-halogenoalkoxy having 1 to 5 halogen atoms, -S-C₁-C₄-alkyl, -S-C₁-C₄-halogenoalkyl having 1 to 5 halogen atoms, aminocarbonyl and aminocarbonyl-C₁-C₄-alkyl, and
   - R⁴³: is selected from the group consisting of hydrogen, halogen, cyano, C₁-C₄-alkyl, C₁-C₄-alkoxy, -S-C₁-C₄-alkyl and C₁-C₄-halogenoalkyl having 1 to 5 halogen atoms, and
   - R⁴⁴: is selected from the group consisting of hydrogen, phenyl, benzyl, C₁-C₄-alkyl, C₁-C₄-halogenoalkyl having 1 to 5 halogen atoms, hydroxy-C₁-C₄-alkyl, C₂-C₆-alkenyl, C₃-C₆-cycloalkyl, C₁-C₄-alkylthio-C₁-C₄-alkyl, C₁-C₄-halogenoalkylthio-C₁-C₄-alkyl having 1 to 5 halogen atoms, C₁-C₄-alkoxy-C₁-C₄-alkyl and C₁-C₄-halogenoalkoxy-C₁-C₄-alkyl having 1 to 5 halogen atoms, or
Het represents a heterocycle of the formula (Het-15) 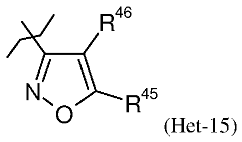 in which
   - R⁴⁵ and R⁴⁶: may be the same or different and are selected from the group consisting of hydrogen, halogen, C₁-C₄-alkyl and C₁-C₄-halogenoalkyl having 1 to 5 halogen atoms, or
Het represents a heterocycle of the formula (Het-16) 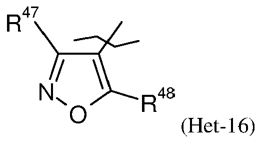 in which
   - R⁴⁷ and R⁴⁸: may be the same or different and are selected from the group consisting of hydrogen, halogen, C₁-C₄-alkyl, C₁-C₄-halogenoalkyl having 1 to 5 halogen atoms, phenyl (optionally substituted by halogen or a C₁-C₄-alkyl), and heterocyclyl like pyridyl, pyrimidinyl and thiadiazolyl (each optionally substituted by halogen or C₁-C₄-alkyl), or
Het represents a heterocycle of the formula (Het-17) 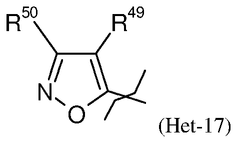 in which
   - R⁴⁹ and R⁵⁰: may be the same or different and are selected from the group consisting of hydrogen, halogen, C₁-C₄-alkyl and C₁-C₄-halogenoalkyl having 1 to 5 halogen atoms, or
Het represents a heterocycle of the formula (Het-18) 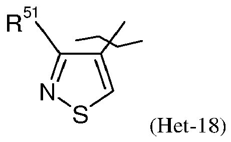 in which
   - R⁵¹: is selected from the group consisting of halogen, C₁-C₄-alkyl and C₁-C₄-halogenoalkyl having 1 to 5 halogen atoms, or
Het represents a heterocycle of the formula (Het-19) 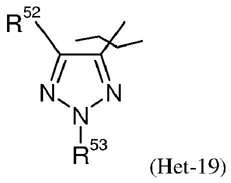 in which
   - R⁵²: is selected from the group consisting of halogen, C₁-C₄-alkyl and C₁-C₄-halogenoalkyl having 1 to 5 halogen atoms, and
   - R⁵³: is selected from the group consisting of hydrogen, C₁-C₄-alkyl, C₁-C₄-halogenoalkyl having 1 to 5 halogen atoms and phenyl (optionally substituted by halogen or C₁-C₄-alkyl), or
Het represents a heterocycle of the formula (Het-20) 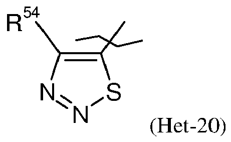 in which
   - R⁵⁴: is selected from the group consisting of halogen, C₁-C₄-alkyl and C₁-C₄-halogenoalkyl having 1 to 5 halogen atoms.

According to the present invention, "Het" may be a six-membered non-fused heterocyclic ring. Specific examples include the following six-membered rings:
Het represents a heterocycle of the formula (Het-21) 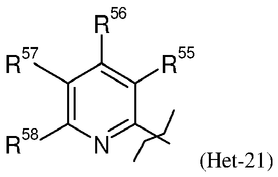 in which
   - R⁵⁵: is selected from the group consisting of hydrogen, halogen, hydroxy, cyano, C₁-C₄-alkyl, C₁-C₄-halogenoalkyl having 1 to 5 halogen atoms, C₁-C₄-alkoxy, -S-C₁-C₄-alkyl, -S-C₁-C₄-halogenoalkyl having 1 to 5 halogen atoms and C₁-C₄-halogenoalkoxy having 1 to 5 halogen atoms, and
   - R⁵⁶, R⁵⁷ and R⁵⁸,: which may be the same or different, are selected from the group consisting of hydrogen, halogen, cyano, C₁-C₄-alkyl, C₁-C₄-halogenoalkyl having 1 to 5 halogen atoms, C₁-C₄-alkoxy, -S-C₁-C₄-alkyl, C₁-C₄-halogenoalkoxy having 1 to 5 halogen atoms, -S(O)-C₁-C₄-alkyl and -S(O)₂-C₁-C₄-alkyl, or
Het represents a heterocycle of the formula (Het-22) 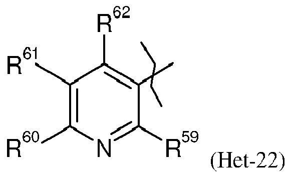 in which
   - R⁵⁹: is selected from the group consisting of hydrogen, halogen, hydroxy, cyano, C₁-C₄-alkyl, C₁-C₄-halogenoalkyl having 1 to 5 halogen atoms, C₁-C₄ alkoxy, -S-C₁-C₅-alkyl, -S-C₂-C₅-alkenyl, -S-C₁-C₄-halogenoalkyl having 1 to 5 halogen atoms, C₁-C₄-halogenoalkoxy having 1 to 5 halogen atoms, phenyloxy (optionally substituted by halogen or C₁-C₄-alkyl) and -S-phenyl (optionally substituted by halogen or C₁-C₄-alkyl), and
   - R⁶⁰, R⁶¹ and R⁶²,: which may the same or different, are selected from the group consisting of hydrogen, halogen, cyano, C₁-C₄-alkyl, C₁-C₄-halogenoalkyl having 1 to 5 halogen atoms, C₁-C₄-alkoxy, -S-C₁-C₄-alkyl, C₁-C₄-halogenoalkoxy having 1 to 5 halogen atoms, -S(O)-C₁-C₄-alkyl, -S(O)₂-C₁-C₄-alkyl, N-morpholine optionally substituted by halogen or C₁-C₄-alkyl, and thienyl (optionally substituted by halogen or a C₁-C₄-alkyl), or
Het represents a heterocycle of the formula (Het-23) 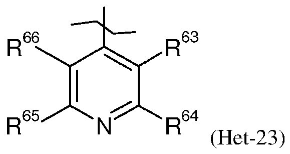 in which
   - R⁶³, R⁶⁴, R⁶⁵ and R⁶⁶,: which may be the same or different, are selected from the group consisting of hydrogen, halogen, hydroxy, cyano, C₁-C₄-alkyl, C₁-C₄-halogenoalkyl having 1 to 5 halogen atoms, C₁-C₄-alkoxy, -S-C₁-C₄-alkyl, -S-C₁-C₄-halogenoalkyl having 1 to 5 halogen atoms, C₁-C₄-halogenoalkoxy having 1 to 5 halogen atoms, -S(O)-C₁-C₄-alkyl and -S(O)₂-C₁-C₄-alkylsulphonyl, or
Het represents a heterocycle of the formula (Het-24) 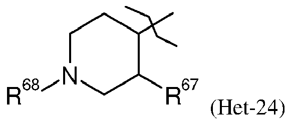 in which
   - R⁶⁷: is selected from the group consisting of hydrogen, halogen, C₁-C₄-alkyl and C₁-C₄-halogenoalkyl having 1 to 5 halogen atoms, and
   - R⁶⁸: is selected from the group consisting of hydrogen, C₁-C₄-alkyl, C₁-C₄-halogenoalkyl having 1 to 5 halogen atoms, C₁-C₆-alkoxycarbonyl, benzyl (optionally substituted by 1 to 3 halogen atoms), benzyloxycarbonyl (optionally substituted by 1 to 3 halogen atoms), and heterocyclyl like pyridyl and pyrimidinyl (each optionally substituted by halogen, C₁-C₄-alkyl, C₁-C₄-halogenoalkyl having 1 to 5 halogen atoms), or
Het represents a heterocycle of the formula (Het-25) 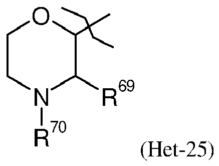 in which
   - R⁶⁹: is selected from the group consisting of hydrogen, halogen, hydroxy, cyano, C₁-C₄-alkyl, C₁-C₄-halogenoalkyl having 1 to 5 halogen atoms, C₁-C₄-alkoxy, -S-C₁-C₄-alkyl, -S-C₁-C₄-halogenoalkyl having 1 to 5 halogen atoms and C₁-C₄-halogenoalkoxy having 1 to 5 halogen atoms, and
   - R⁷⁰: is selected from the group consisting of hydrogen, C₁-C₄-alkyl, C₁-C₄-halogenoalkyl having 1 to 5 halogen atoms, and benzyl, or
Het represents a heterocycle of the formula (Het-26) 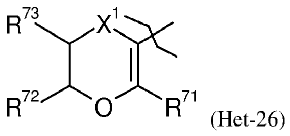 in which
   - X¹: is selected from the group consisting of sulphur, -SO-, -SO₂- and -CH₂-, and
   - R⁷¹: is selected from the group consisting of C₁-C₄-alkyl and C₁-C₄-halogenoalkyl having 1 to 5 halogen atoms, and
   - R⁷² and R⁷³: may be the same or different and are selected from the group consisting of hydrogen and C₁-C₄-alkyl, or
Het represents a heterocycle of the formula (Het-27) 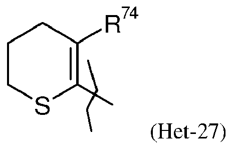 in which
   - R⁷⁴: is selected from the group consisting of C₁-C₄-alkyl and C₁-C₄-halogenoalkyl having 1 to 5 halogen atoms, or
Het represents a heterocycle of the formula (Het-28) 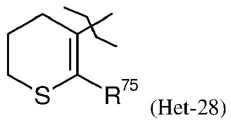 in which
   - R⁷⁵: is selected from the group consisting of C₁-C₄-alkyl and C₁-C₄-halogenoalkyl having 1 to 5 halogen atoms, or
Het represents a heterocycle of the formula (Het-29) 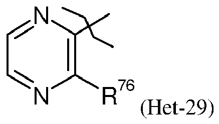 in which
   - R⁷⁶: is selected from the group consisting of hydrogen, halogen, C₁-C₄-alkyl and C₁-C₄-halogenoalkyl having 1 to 5 halogen atoms
as well as salts, N-oxides, metal complexes and metalloid complexes of compounds of formula (I), against nematodes.

In formulae (Het-1) to (Het-29) 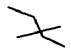 depicts the bond which connects Het to the C(O)NR⁵-moiety in the compounds of formula (I).

Any of the compounds according to the invention can exist in one or more optical or chiral isomer forms depending on the number of asymmetric centres in the compound. The invention thus relates equally to all the optical isomers and to their racemic or scalemic mixtures (the term "scalemic" denotes a mixture of enantiomers in different proportions), and to the mixtures of all the possible stereoisomers, in all proportions. The diastereoisomers and/or the optical isomers can be separated according to the methods which are known *per se* by the man ordinary skilled in the art.

The invention also relates to the use of salts, N-oxides, metal complexes and metalloid complexes of compounds of formula (I).

A preferred embodiment of the present invention is the use of compounds of formula (Ia) 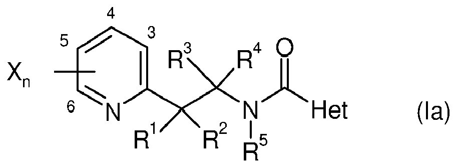 wherein R¹, R², R³, R⁴, R⁵, Het, X and n have the meanings given above, for the control of nematodes.

Another preferred embodiment of the present invention is the use of compounds of formula (Ib) 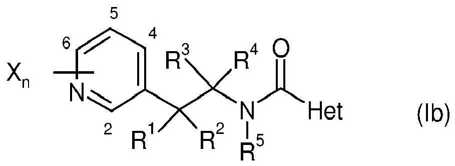 wherein R¹, R², R³, R⁴, R⁵, Het, X and n have the meanings given above, for the control of nematodes.

Another preferred embodiment of the present invention is the use of compounds of formula (Ic) 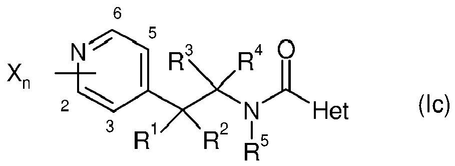 wherein R¹, R², R³, R⁴, R⁵, Het, X and n have the meanings given above, for the control of nematodes.

Compounds of formula (I) can for example be prepared by reacting a compound of formula (II) 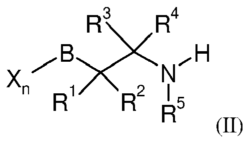 wherein X, n, B, R¹, R², R³, R⁴ and R⁵ are defined as above,
with a compound of formula (III) 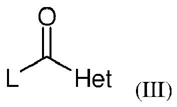 wherein Het is defined as above, and

L is a leaving group chosen as being a halogen atom, a hydroxyl group, an optionally substituted alkyl group, an optionally substituted benzyl group or a group of formula 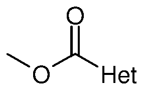 wherein Het is defined as above,
usually in the presence of a base, a condensing agent and in the presence of a solvent.

This process and other processes for the preparation of the compounds of formula (I) as well as intermediates for the preparation of compounds of formula (I) are described in more detail in WO 2001/011965 A1 (P1), WO 2005/058828 A1 (P2), WO2005/014545 A2 (P3), WO 2005/103004 A1 (P4), WO 2006/122952 A1 (P5), EP 2 289 880 A1 (P6), WO 2006/008191 A1 (P7), WO 2006/008192 A1 (P8), WO 2004/074280 A1 (P9), WO 2005/058833 A2 (P10), WO 2005/085238 A1 (P11), WO 2005/103006 A1 (P12), WO 2006/122955 A1 (P13), WO 2006/008194 A1 (P14), WO 2006/008193 A1 (P15) and WO 2006/067103 A2 (P16).

In compounds of formula (I), which are preferably used, R¹, R², R³, R⁴, R⁵, Het, B, X and n have the following meanings.
- B: represents 2-pyridyl, 3-pyridyl, or 4-pyridyl;
- X: is selected from the group consisting of halogen, nitro, C₁-C₄-alkyl, C₁-C₄-halogenoalkyl having 1 to 5 halogen atoms, C₂-C₄-alkenyl, C₂-C₄-alkynyl, C₁-C₄-alkylamino, di-(C₁-C₄-alkyl)amino, C₁-C₄-alkoxy, C₁-C₄-halogenoalkoxy having 1 to 5 halogen atoms, C₂-C₄-alkenyloxy, C₂-C₄-halogenoalkenyloxy having 1 to 5 halogen atoms, C₃-C₄-alkynyloxy, C₃-C₄-halogenoalkynyloxy having 1 to 5 halogen atoms, C₃-C₆-cycloalkyl, C₃-C₆-halogenocycloalkyl having 1 to 5 halogen atoms, C₁-C₄-alkylcarbonyl, C₁-C₄-halogenoalkylcarbonyl having 1 to 5 halogen atoms, -CONH(C₁-C₄-alkyl), -CON(C₁-C₄-alkyl)₂, -CONH(OC₁-C₄-alkyl), -CON(OC₁-C₄-alkyl)(C₁-C₄-alkyl), C₁-C₄-alkoxycarbonyl, C₁-C₄-halogenoalkoxycarbonyl having 1 to 5 halogen atoms, C₁-C₄-alkylcarbonyloxy, C₁-C₄-halogenoalkylcarbonyloxy having 1 to 5 halogen atoms, C₁-C₄-alkylcarbonylamino, C₁-C₄-halogenoalkylcarbonylamino having 1 to 5 halogen atoms, -OCONH(C₁-C₄-alkyl), -OCON(C₁-C₄-alkyl)₂, -OCONH(OC₁-C₄-alkyl), -OCO(OC₁-C₄-alkyl), -S-C₁-C₄-alkyl, -S-C₁-C₄-halogenoalkyl having 1 to 5 halogen atoms, -S(O)-C₁-C₄-alkyl, -S(O)-C₁-C₄-halogenoalkyl having 1 to 5 halogen atoms, -S(O)₂-C₁-C₄-alkyl, -S(O)₂-C₁-C₄-halogenoalkyl having 1 to 5 halogen atoms, (C₁-C₄-alkoxyimino)-C₁-C₆-alkyl, (C₂-C₆-alkenyloxyimino)-C₁-C₆-alkyl, (C₃-C₆-alkynyloxyimino)-C₁-C₆-alkyl, (benzyloxyimino)-C₁-C₆-alkyl, benzyloxy, -S-benzyl, benzylamino, phenoxy, -S-phenyl and phenylamino;
- n: is 1, 2, 3 or 4 and if n is 2, 3, or 4, then the substituents X may be the same or different;
- R¹ and R²: are the same or different and are selected from the group consisting of hydrogen, halogen, cyano, hydroxy, amino, -CHO, -OCHO, -NHCHO, -COOH, -CONH₂, C₁-C₄-alkyl, C₂-C₄-alkenyl, C₂-C₄-alkynyl, C₁-C₄-alkylamino, di-(C₁-C₄-alkyl)amino, C₁-C₄-alkoxy, C₁-C₄-halogenoalkyl having 1 to 5 halogen atoms, C₁-C₄-halogenoalkoxy having 1 to 5 halogen atoms, C₂-C₄-alkenyloxy, C₂-C₄-halogenoalkenyloxy having 1 to 5 halogen atoms, C₃-C₄-alkynyloxy, C₃-C₄-halogenoalkynyloxy having 1 to 5 halogen atoms, C₃-C₆-cycloalkyl, C₃-C₆-halogenocycloalkyl having 1 to 5 halogen atoms, C₃-C₆-cycloalkyl-C₁-C₃-alkyl, C₃-C₆-halogenocycloalkyl-C₁-C₃-alkyl having 1 to 5 halogen atoms, C₁-C₄-alkylcarbonyl, C₁-C₄-halogenoalkylcarbonyl having 1 to 5 halogen atoms, -CONH(C₁-C₄-alkyl), -CON(C₁-C₄-alkyl)₂, -CONH(OC₁-C₄-alkyl), -CON(OC₁-C₄-alkyl)(C₁-C₄-alkyl), C₁-C₄-alkoxycarbonyl, a C₁-C₄-halogenoalkoxycarbonyl having 1 to 5 halogen atoms, -OC(O)-C₁-C₄-alkyl, -OC(O)-C₁-C₄-halogenoalkyl having 1 to 5 halogen atoms, -NHC(O)-C₁-C₄-alkyl, -NHC(O)-C₁-C₄-halogenoalkyl having 1 to 5 halogen atoms, -OCONH(C₁-C₄-alkyl), -OCON(C₁-C₄-alkyl)₂, -OCONH(OC₁-C₄-alkyl), OCO(OC₁-C₄-alkyl), -S-C₁-C₄-alkyl, -S-C₁-C₄-halogenoalkyl having 1 to 5 halogen atoms, -S(O)-C₁-C₄-alkyl, -S(O)-C₁-C₄-halogenoalkyl having 1 to 5 halogen atoms, -S(O)₂-C₁-C₄-alkyl, -S(O)₂-C₁-C₄-halogenoalkyl having 1 to 5 halogen atoms, benzyl, benzyloxy, -S-benzyl, -S(O)-benzyl, -S(O)₂-benzyl, benzylamino, phenoxy, -S-phenyl, -S(O)-phenyl, -S(O)₂-phenyl, phenylamino, phenylcarbonylamino, 2,6-dichlorophenyl-carbonylamino, 2-chlorophenyl-carbonylamino and phenyl;
- R³ and R⁴: are the same or different and are selected from the group consisting of hydrogen, halogen, cyano, hydroxy, amino, -CHO, -COOH, -CONH₂, C₁-C₄-alkyl, C₂-C₄-alkenyl, C₂-C₄-alkynyl, C₁-C₄-alkylamino, di-(C₁-C₄-alkyl)amino, C₁-C₄-alkoxy, hydroxy-C₁-C₄-alkyl, C₁-C₆-alkoxy-C₁-C₄-alkyl, C₁-C₄-halogenoalkyl having 1 to 5 halogen atoms, C₁-C₄-halogenoalkoxy having 1 to 5 halogen atoms, C₂-C₄-alkenyloxy, C₂-C₄-halogenoalkenyloxy having 1 to 5 halogen atoms, C₃-C₄-alkynyloxy, C₃-C₄-halogenoalkynyloxy having 1 to 5 halogen atoms, C₃-C₆-cycloalkyl, C₃-C₆-halogenocycloalkyl having 1 to 5 halogen atoms, C₁-C₄-alkylcarbonyl, C₁-C₄-halogenoalkylcarbonyl having 1 to 5 halogen atoms, -CONH(C₁-C₄-alkyl), -CON(C₁-C₄-alkyl)₂, -CONH(OC₁-C₄-alkyl), -CON(OC₁-C₄-alkyl)(C₁-C₄-alkyl), C₁-C₄-alkoxycarbonyl, C₁-C₄-halogenoalkoxycarbonyl having 1 to 5 halogen atoms, -OC(O)-C₁-C₄-alkyl, -OC(O)-C₁-C₄-halogenoalkyl having 1 to 5 halogen atoms, -NHC(O)-C₁-C₄-alkyl, -NHC(O)-C₁-C₄-halogenoalkyl having 1 to 5 halogen atoms, -OCONH(C₁-C₄-alkyl), -OCON(C₁-C₄-alkyl)₂, -OCONH(OC₁-C₄-alkyl), OCO(OC₁-C₄-alkyl), -S-C₁-C₄-alkyl, -S-C₁-C₄-halogenoalkyl having 1 to 5 halogen atoms, -S(O)-C₁-C₄-alkyl, -S(O)-C₁-C₄-halogenoalkyl having 1 to 5 halogen atoms, -S(O)₂-C₁-C₄-alkyl, -S(O)₂-C₁-C₄-halogenoalkyl having 1 to 5 halogen atoms, benzyl, benzyloxy, -S-benzyl, -S(O)-benzyl, -S(O)₂-benzyl, benzylamino, phenoxy, -S-phenyl, -S(O)-phenyl, -S(O)₂-phenyl, phenylamino, phenylcarbonylamino, 2-chlorophenyl-carbonylamino, 2,6-dichlorophenyl-carbonylamino and phenyl;
- R⁵: is selected from the group consisting of hydrogen, -CHO, -OH, C₁-C₄-alkyl, C₁-C₄-halogenoalkyl having 1 to 5 halogen atoms, C₁-C₄-alkoxy, C₁-C₄-halogenoalkoxy having 1 to 5 halogen atoms, C₃-C₆-cycloalkyl, C₃-C₆-halogenocycloalkyl having 1 to 5 halogen atoms, C₃-C₆-cycloalkyl-C₁-C₄-alkyl, -CONH(C₁-C₄-alkyl), C₂-C₄-alkenyl, C₂-C₄-alkynyl, C₁-C₄-alkoxy-C₁-C₄-alkyl, C₃-C₆-cycloalkyl-C₁-C₃-alkyl, cyano-C₁-C₄-alkyl, amino-C₁-C₆-alkyl, C₁-C₄-alkylamino-C₁-C₄-alkyl, di-(C₁-C₄-alkyl)amino-C₁-C₄-alkyl, C₁-C₄-alkylcarbonyl, C₁-C₄-halogenoalkylcarbonyl having 1 to 5 halogen atoms, C₁-C₄-alkoxycarbonyl, C₁-C₄-benzyloxycarbonyl, C₁-C₄-alkoxy-C₁-C₄-alkylcarbonyl, -S-C₁- C₄-alkyl, -S-C₁-C₄-halogenoalkyl having 1 to 5 halogen atoms, -S(O)₂-C₁-C₄-alkyl, and -S(O)₂-C₁-C₄-halogenoalkyl having 1 to 5 halogen atoms;
with the proviso, that when B = 2-pyridyl, then
Het may not be pyrazinyl (Het ≠ Het-29), and
when B = 2-pyridyl and Het = pyridyl or pyrazolyl, then
at least one of R¹, R², R³, R⁴ and R⁵ is not a hydrogen atom, and
R¹ and R² may also together with the carbon atom to which they are bonded form a 3-, 5- or 6-membered carbocycle, or
R² and R³ may also together with the carbon atoms to which they are bonded form a 3-, 5-, 6- or 7-membered carbocycle, and
with the further proviso, that when B = 3-pyridyl, then
R¹ and R² may also together with the carbon atom to which they are bonded form a 3-, 5- or 6-membered carbocycle, or
R³ and R⁴ may also together with the carbon atoms to which they are bonded form a 3-, 5- or 6-membered carbocycle, and
with the further proviso, that when B = 4-pyridyl, then
R¹ and R² may also together with the carbon atom to which they are bonded form a 3-, 5- or 6-membered carbocycle, or
R³ and R⁴ may also together with the carbon atoms to which they are bonded form a 3-, 5- or 6-membered carbocycle;
Het represents a heterocycle of the formula (Het-1) 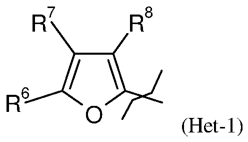 in which
   - R⁶ and R⁷: may be the same or different and are selected from the group consisting of hydrogen, halogen, nitro, C₁-C₄-alkyl and C₁-C₄-halogenoalkyl having 1 to 5 halogen atoms, and
   - R⁸: is selected from the group consisting of hydrogen, halogen, C₁-C₄-alkyl and C₁-C₄-halogenoalkyl having 1 to 5 halogen atoms, or
Het represents a heterocycle of the formula (Het-2) 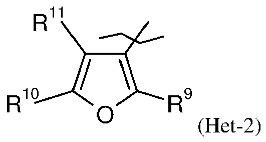 in which
   - R⁹: is selected from the group consisting of hydrogen, halogen, C₁-C₄-alkyl and C₁-C₄-halogenoalkyl having 1 to 5 halogen atoms, and
   - R¹⁰ and R¹¹: may be the same or different and are selected from the group consisting of hydrogen, halogen, C₁-C₄-alkyl and C₁-C₄-halogenoalkyl having 1 to 5 halogen atoms, phenyl optionally substituted by halogen or C₁-C₄-alkyl), or
Het represents a heterocycle of the formula (Het-4) 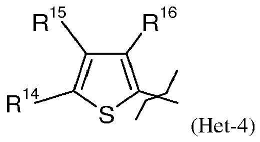 in which
   - R¹⁴ and R¹⁵: may be the same or different and are selected from the group consisting of hydrogen, halogen, C₁-C₄-alkyl, C₁-C₄-halogenoalkyl having 1 to 5 halogen atoms, -S-C₁-C₄-alkyl, -S(O)₂-C₁-C₄-alkyl, phenyl (optionally substituted by halogen or C₁-C₄-alkyl) and pyridyl (optionally substituted by halogen or C₁-C₄-alkyl), and
   - R¹⁶: is selected from the group consisting of hydrogen, halogen, cyano, C₁-C₄-alkyl, C₁-C₄-halogenoalkyl having 1 to 5 halogen atoms and C₁-C₄-halogenoalkoxy having 1 to 5 halogen atoms, or
Het represents a heterocycle of the formula (Het-5) 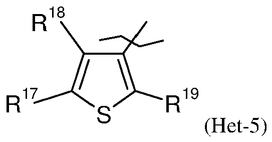 in which
   - R¹⁷ and R¹⁸: may be the same or different and are selected from the group consisting of hydrogen, halogen, C₁-C₄-alkyl, C₁-C₄-alkyloxy and C₁-C₄-halogenoalkyl having 1 to 5 halogen atoms, and
   - R¹⁹: is selected from the group consisting of hydrogen, halogen, C₁-C₄-alkyl and C₁-C₄-halogenoalkyl having 1 to 5 atoms, or
Het represents a heterocycle of the formula (Het-6) 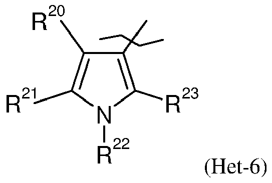 in which
   - R²⁰: is selected from the group consisting of hydrogen, halogen, cyano, C₁-C₄-alkyl and C₁-C₄-halogenoalkyl having 1 to 5 halogen atoms, and
   - R²¹ and R²³: may be the same or different and are selected from the group consisting of hydrogen, halogen, C₁-C₄-alkyl and C₁-C₄-halogenoalky having 1 to 5 halogen atoms, and
   - R²²: is selected from the group consisting of hydrogen, cyano, C₁-C₄-alkyl, C₁-C₄-halogenoalkyl having 1 to 5 halogen atoms, C₁-C₄-alkoxy-C₁-C₄-alkyl, or
Het represent a heterocycle of the formula (Het-7) 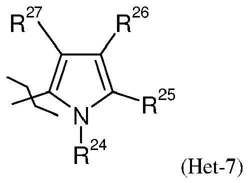 in which
   - R²⁴: is selected from the group consisting of hydrogen, cyano, C₁-C₄-alkyl, C₁-C₄-halogenoalkyl having 1 to 5 halogen atoms, C₁-C₄-alkoxy-C₁-C₄-alkyl, C₁-C₆-alkylcarbonyl, or benzoyl (optionally substituted by halogen or a C₁-C₄-alkyl), and
   - R²⁵, R²⁶ and R²⁷: may be the same or different and are selected from the group consisting of hydrogen, halogen, cyano, C₁-C₄-alkyl, C₁-C₄-halogenalkyl having 1 to 5 halogen atoms and C₁-C₄-alkylcarbonyl, or
Het represent a heterocycle of the formula (Het-9) 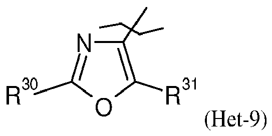 in which
   - R³⁰: is selected from the group consisting of hydrogen and C₁-C₄-alkyl, and
   - R³¹: is selected from the group consisting of halogen, C₁-C₄-alkyl, C₁-C₄-halogenoalkyl having 1 to 5 halogen atoms and phenyl (optionally substituted by halogen or C₁-C₄-alkyl), or
Het represent a heterocycle of the formula (Het-10) 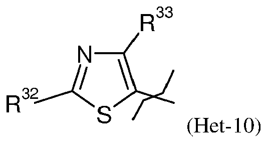 in which
   - R³²: is selected from the group consisting of hydrogen, halogen, C₁-C₄-alkylamino, di-(C₁-C₄-alkyl)amino, C₁-C₄-alkyl, C₁-C₄-halogenoalkyl having 1 to 5 halogen atoms and phenyl (optionally substituted by halogen or C₁-C₄-alkyl), and
   - R³³: is selected from the group consisting of halogen, C₁-C₄-alkyl and C₁-C₄-halogenoalkyl having 1 to 5 halogen atoms, or
Het represents a heterocycle of the formula (Het-11) 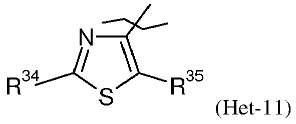 in which
   - R³⁴: is selected from the group consisting of hydrogen, halogen, C₁-C₄-alkylamino, di-(C₁-C₄-alkyl)amino, C₁-C₄-alkyl and C₁-C₄-halogenoalkyl having 1 to 5 halogen atoms, and
   - R³⁵: is selected from the group consisting of halogen, C₁-C₄-alkyl and C₁-C₄-halogenoalkyl having 1 to 5 halogen atoms, or
Het represents a heterocycle of the formula (Het-12) 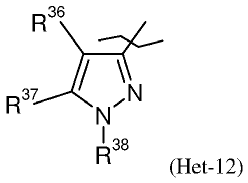 in which
   - R³⁶: is selected from the group consisting of hydrogen, halogen, cyano, nitro, C₁-C₄-alkyl, C₁-C₄-halogenoalkyl having 1 to 5 halogen atoms, C₃-C₆-cycloalkyl, C₁-C₄-alkoxy, C₁-C₄-halogenoalkoxy having 1 to 5 halogen atoms, -S-C₁-C₄-alkyl and -S-C₁-C₄-halogenoalkyl having 1 to 5 halogen atoms, and
   - R³⁷: ist selected from the group consisting of hydrogen, halogen, cyano, nitro, C₁-C₄-alkyl, C₁-C₄-alkoxy and -S-C₁-C₄-alkyl, and
   - R³⁸: is selected from the group consisting of phenyl, C₁-C₄-alkyl, C₁-C₄-halogenoalkyl having 1 to 5 halogen atoms, hydroxy-C₁-C₄-alkyl, C₂-C₆-alkenyl, C₃-C₆-cycloalkyl, C₁-C₄-alkylthio-C₁-C₄-alkyl, C₁-C₄-halogenoalkylthio-C₁-C₄-alkyl having 1 to 5 halogen atoms, C₁-C₄-alkoxy-C₁-C₄-alkyl and C₁-C₄-halogenoalkoxy-C₁-C₄-alkyl having 1 to 5 halogen atoms, or
Het represents a heterocycle of the formula (Het-13) 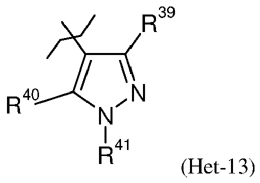 in which
   - R³⁹: is selected from the group consisting of hydrogen, halogen, cyano, nitro, C₁-C₄-alkyl, C₁-C₄-halogenoalkyl having 1 to 5 halogen atoms, C₃-C₆-cycloalkyl, C₁-C₄-alkoxy, C₁-C₄-halogenoalkoxy having 1 to 5 halogen atoms, -S-C₁-C₄-alkyl, -S-C₁-C₄-halogenoalkyl having 1 to 5 atoms, aminocarbonyl and aminocarbonyl-C₁-C₄-alkyl, and
   - R⁴⁰: is selected from the group consisting of hydrogen, halogen, cyano, C₁-C₄-alkyl, C₁-C₄-alkoxy, C₁-C₄-halogenoalkoxy having 1 to 5 halogen atoms and -S-C₁-C₄-alkyl, and
   - R⁴¹: is selected from the group consisting of hydrogen, C₁-C₄-alkyl, C₁-C₄-halogenoalkyl having 1 to 5 halogen atoms, hydroxy-C₁-C₄-alkyl, C₂-C₆-alkenyl, C₃-C₆-cycloalkyl, C₁-C₄-alkylthio-C₁-C₄-alkyl, C₁-C₄-halogenoalkylthio-C₁-C₄-alkyl having 1 to 5 halogen atoms, C₁-C₄-alkoxy-C₁-C₄-alkyl, C₁-C₄-halogenoalkoxy-C₁-C₄-alkyl having 1 to 5 halogen atoms and phenyl optionally substituted by halogen, C₁-C₄-alkyl, C₁-C₄-alkoxy-C₁-C₄-alkyl or nitro, or
Het represents a heterocycle of the formula (Het-14) 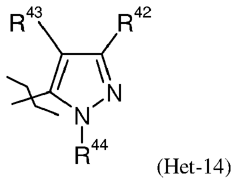 in which
   - R⁴²: is selected from the group consisting of hydrogen, halogen, cyano, C₁-C₄-alkyl, C₁-C₄-halogenoalkyl having 1 to 5 halogen atoms, C₃-C₆-cycloalkyl, C₁-C₄-alkoxy, C₁-C₄-halogenoalkoxy having 1 to 5 halogen atoms, -S-C₁-C₄-alkyl, -S-C₁-C₄-halogenoalkyl having 1 to 5 halogen atoms, aminocarbonyl and aminocarbonyl-C₁-C₄-alkyl, and
   - R⁴³: is selected from the group consisting of hydrogen, halogen, cyano, C₁-C₄-alkyl, C₁-C₄-alkoxy, -S-C₁-C₄-alkyl and C₁-C₄-halogenoalkyl having 1 to 5 halogen atoms, and
   - R⁴⁴: is selected from the group consisting of phenyl, benzyl, C₁-C₄-alkyl, C₁-C₄-halogenoalkyl having 1 to 5 halogen atoms, hydroxy-C₁-C₄-alkyl, C₂-C₆-alkenyl, C₃-C₆-cycloalkyl, C₁-C₄-alkylthio-C₁-C₄-alkyl, C₁-C₄-halogenoalkylthio-C₁-C₄-alkyl having 1 to 5 halogen atoms, C₁-C₄-alkoxy-C₁-C₄-alkyl and C₁-C₄-halogenoalkoxy-C₁-C₄-alkyl having 1 to 5 halogen atoms, or
Het represents a heterocycle of the formula (Het-15) 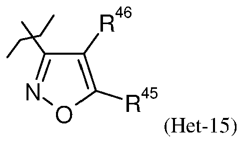 in which
   - R⁴⁵ and R⁴⁶: may be the same or different and are selected from the group consisting of hydrogen, halogen, C₁-C₄-alkyl and C₁-C₄-halogenoalkyl having 1 to 5 halogen atoms, or
Het represents a heterocycle of the formula (Het-16) 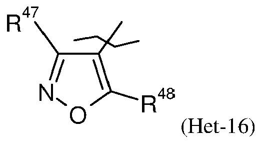 in which
   - R⁴⁷ and R⁴⁸: may be the same or different and are selected from the group consisting of hydrogen, halogen, C₁-C₄-alkyl, C₁-C₄-halogenoalkyl having 1 to 5 halogen atoms, phenyl (optionally substituted by halogen or a C₁-C₄-alkyl), or heterocyclyl like pyridyl, pyrimidinyl and thiadiazolyl (optionally substituted by halogen or C₁-C₄-alkyl), or
Het represents a heterocycle of the formula (Het-17) 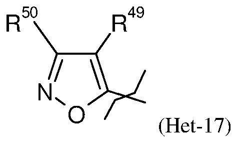 in which
   - R⁴⁹ and R⁵⁰: may be the same or different and are selected from the group consisting of hydrogen, halogen, C₁-C₄-alkyl and C₁-C₄-halogenoalkyl having 1 to 5 halogen atoms, or
Het represents a heterocycle of the formula (Het-19) 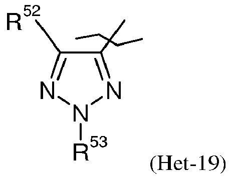 in which
   - R⁵²: is selected from the group consisting of halogen, C₁-C₄-alkyl and C₁-C₄-halogenoalkyl having 1 to 5 halogen atoms, and
   - R⁵³: is selected from the group consisting of C₁-C₄-alkyl, C₁-C₄-halogenoalkyl having 1 to 5 halogen atoms and phenyl (optionally substituted by halogen or C₁-C₄-alkyl), or
Het represents a heterocycle of the formula (Het-20) 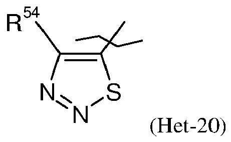 in which
   - R⁵⁴: is selected from the group consisting of halogen, C₁-C₄-alkyl and C₁-C₄-halogenoalkyl having 1 to 5 halogen atoms, or
Het represents a heterocycle of the formula (Het-21) 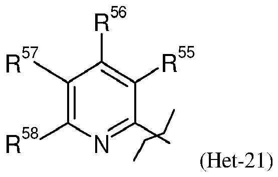 in which
   - R⁵⁵: is selected from the group consisting of hydrogen, halogen, cyano, C₁-C₄-alkyl, C₁-C₄-halogenoalkyl having 1 to 5 halogen atoms, C₁-C₄-alkoxy, -S-C₁-C₄-alkyl, -S-C₁-C₄-halogenoalkyl having 1 to 5 halogen atoms and C₁-C₄-halogenoalkoxy having 1 to 5 halogen atoms, and
   - R⁵⁶, R⁵⁷ and R⁵¹,: which may be the same or different, are selected from the group consisting of hydrogen, halogen, cyano, C₁-C₄-alkyl, C₁-C₄-halogenoalkyl having 1 to 5 halogen atoms, C₁-C₄-alkoxy, -S-C₁-C₄-alkyl, C₁-C₄-halogenoalkoxy having 1 to 5 halogen atoms, -S(O)-C₁-C₄-alkyl and -S(O)₂-C₁-C₄-alkyl, or
Het represents a heterocycle of the formula (Het-22) 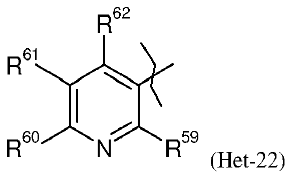 in which
   - R⁵⁹: is selected from the group consisting of hydrogen, halogen, hydroxy, cyano, C₁-C₄-alkyl, C₁-C₄-halogenoalkyl having 1 to 5 halogen atoms, C₁-C₄ alkoxy, -S-C₁-C₅-alkyl, -S-C₂-C₅-alkenyl, -S-C₁-C₄-halogenoalkyl having 1 to 5 halogen atoms, C₁-C₄-halogenoalkoxy having 1 to 5 halogen atoms, phenyloxy (optionally substituted by halogen or C₁-C₄-alkyl) and -S-phenyl (optionally substituted by halogen or C₁-C₄-alkyl), and
   - R⁶⁰, R⁶¹ and R⁶²,: which may the same or different, are selected from the group consisting of hydrogen, halogen, cyano, C₁-C₄-alkyl, C₁-C₄-halogenoalkyl having 1 to 5 halogen atoms, C₁-C₄-alkoxy, -S-C₁-C₄-alkyl, C₁-C₄-halogenoalkoxy having 1 to 5 halogen atoms, -S(O)-C₁-C₄-alkyl, -S(O)₂-C₁-C₄-alkyl, N-morpholine (optionally substituted by halogen or C₁-C₄-alkyl) and thienyl (optionally substituted by halogen or a C₁-C₄-alkyl), or
Het represents a heterocycle of the formula (Het-23) 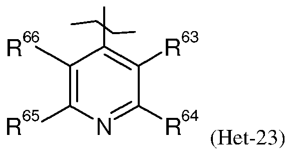 in which
   - R⁶³, R⁶⁴, R⁶⁵ and R⁶⁶,: which may be the same or different, are selected from the group consisting of hydrogen, halogen, hydroxy, cyano, C₁-C₄-alkyl, C₁-C₄-halogenoalkyl having 1 to 5 halogen atoms, C₁-C₄-alkoxy, -S-C₁-C₄-alkyl, -S-C₁-C₄-halogenoalkyl having 1 to 5 halogen atoms, C₁-C₄-halogenoalkoxy having 1 to 5 halogen atoms, -S(O)-C₁-C₄-alkyl and -S(O)₂-C₁-C₄-alkylsulphonyl, or
Het represents a heterocycle of the formula (Het-24) 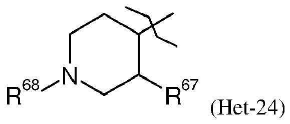 in which
   - R⁶⁷: is selected from the group consisting of hydrogen, halogen, C₁-C₄-alkyl and C₁-C₄-halogenoalkyl having 1 to 5 halogen atoms, and
   - R⁶⁸: is selected from the group consisting of C₁-C₄-alkyl, C₁-C₄-halogenoalkyl having 1 to 5 halogen atoms, C₁-C₆-alkoxycarbonyl, benzyl (optionally substituted by 1 to 3 halogen atoms), benzyloxycarbonyl (optionally substituted by 1 to 3 halogen atoms) and heterocyclyl like pyrimidinyl, (optionally substituted by halogen, C₁-C₄-alkyl, C₁-C₄-halogenoalkyl having 1 to 5 halogen atoms), or
Het represents a heterocycle of the formula (Het-25) 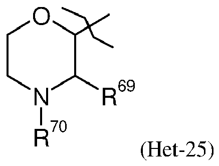 in which
   - R⁶⁹: is selected from the group consisting of hydrogen, C₁-C₄-alkyl and C₁-C₄-halogenoalkyl having 1 to 5 halogen atoms, and
   - R⁷⁰: is selected from the group consisting of hydrogen, C₁-C₄-alkyl, C₁-C₄-halogenoalkyl having 1 to 5 halogen atoms and benzyl, or
Het represents a heterocycle of the formula (Het-26) 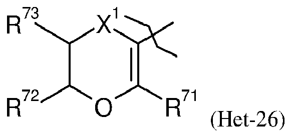 in which
   - X¹: is selected from the group consisting of sulphur, -SO-, or -SO₂-, and
   - R⁷¹: is selected from the group consisting of C₁-C₄-alkyl and C₁-C₄-halogenoalkyl having 1 to 5 halogen atoms, and
   - R⁷² and R⁷³: may be the same or different and are selected from the group consisting of hydrogen and C₁-C₄-alkyl, or
Het represents a heterocycle of the formula (Het-29) 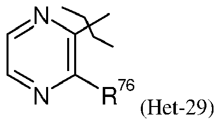 in which
   - R⁷⁶: is selected from the group consisting of hydrogen, halogen, C₁-C₄-alkyl and C₁-C₄-halogenoalkyl having 1 to 5 halogen atoms,
   as well as its salts, N-oxydes, metallic complexes, metalloidic complexes and optically active isomers.

In compounds of formula (I), which are particularly preferably used, R¹, R², R³, R⁴, R⁵, Het, B, X and n have the following meanings.
- B: represents 2-pyridyl, 3-pyridyl, or 4-pyridyl;
- X: is selected from the group consisting of halogen, C₁-C₄-alkyl, C₁-C₄-halogenoalkyl having 1 to 5 halogen atoms, C₁-C₄-alkoxy and (C₁-C₄-alkoxyimino)-C₁-C₆-alkyl;
- n: is 1, 2, 3 or 4 and if n is 2, 3, or 4, then the substituents X may be the same or different;
- R¹ and: R² are the same or different and are selected from the group consisting of hydrogen, halogen, cyano, -COOH, C₁-C₄-alkyl, C₂-C₄-alkenyl, C₂-C₄-alkynyl, C₃-C₆-cycloalkyl-C₁-C₃-alkyl, C₁-C₄₋alkoxycarbonyl, -NHC(O)-C₁-C₄-alkyl, 2,6-dichlorophenyl-carbonylamino, 2-chlorophenyl-carbonylamino and phenyl, or
- R³ and R⁴: are the same or different and are selected from the group consisting of hydrogen, C₁-C₄-alkyl, C₁-C₄-halogenoalkyl having 1 to 5 halogen atoms and phenyl;
- R⁵: is selected from the group consisting of hydrogen and C₃-C₆-cycloalkyl;
with the proviso, that when B = 2-pyridyl, then
Het may not be pyrazinyl (Het ≠ Het-29), and
when B = 2-pyridyl and Het = pyridyl or pyrazolyl, then
at least one of R¹, R², R³, R⁴ and R⁵ is not a hydrogen atom, and
R¹ and R² may also together with the carbon atom to which they are bonded form a 5-membered carbocycle, or
R² and R³ may together with the carbon atoms to which they are bonded form a 3-, 6- or 7-membered carbocycle;
Het represents a heterocycle of the formula (Het-1) 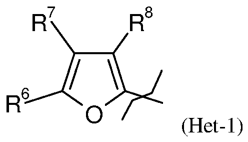 in which
   - R⁶ and R⁷: may be the same or different and are selected from the group consisting of hydrogen and nitro, and
   - R⁸: is selected from the group consisting of hydrogen, halogen and C₁-C₄-alkyl, or
Het represents a heterocycle of the formula (Het-2) 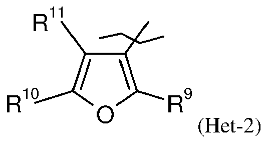 in which
   - R⁹: is selected from the group consisting of hydrogen, halogen, C₁-C₄-alkyl and C₁-C₄-halogenoalkyl having 1 to 5 halogen atoms, and
   - R¹⁰ and R¹¹: may be the same or different and are selected from the group consisting of hydrogen, C₁-C₄₋alkyl and phenyl (optionally substituted by halogen), or
Het represents a heterocycle of the formula (Het-4) 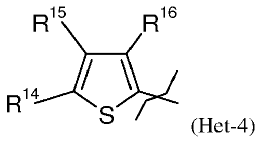 in which
   - R¹⁴ and R¹⁵: may be the same or different and are selected from the group consisting of hydrogen, halogen, C₁-C₄-halogenoalkyl having 1 to 5 halogen atoms, -S-C₁-C₄-alkyl, -S(O)₂-C₁-C₄-alkyl, phenyl and pyridyl, and
   - R¹⁶: is selected from the group consisting of halogen, C₁-C₄-alkyl, C₁-C₄-halogenoalkyl having 1 to 5 halogen atoms and C₁-C₄-halogenoalkoxy having 1 to 5 halogen atoms, or
Het represents a heterocycle of the formula (Het-5) 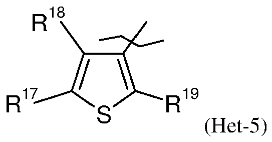 in which
   - R¹⁷ and R¹⁸: may be the same or different and are selected from the group consisting of hydrogen, halogen, C₁-C₄-alkyl and C₁-C₄-alkyloxy, and
   - R¹⁹: is selected from the group consisting of hydrogen, halogen and C₁-C₄-halogenoalkyl having 1 to 5 atoms, or
Het represents a heterocycle of the formula (Het-6) 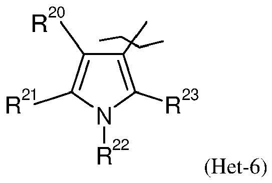 in which
   - R²⁰: is selected from the group consisting of C₁-C₄-halogenoalkyl having 1 to 5 halogen atoms, and
   - R²¹ and R²³: may be the same or different and are selected from the group consisting of hydrogen and C₁-C₄-alkyl, and
   - R²²: is selected from the group consisting of hydrogen and C₁-C₄-alkyl, or
Het represent a heterocycle of the formula (Het-7) 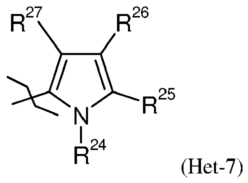 in which
   - R²⁴: is selected from the group consisting of hydrogen, C₁-C₄-alkyl and C₁-C₆-alkylcarbonyl, and
   - R²⁵, R²⁶ and R²⁷: may be the same or different and are selected from the group consisting of hydrogen, halogen and C₁-C₄-alkyl, or
Het represent a heterocycle of the formula (Het-9) 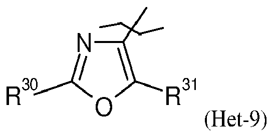 in which
   - R³⁰: is selected from the group consisting of C₁-C₄-alkyl, and
   - R³¹: is selected from the group consisting of C₁-C₄-halogenoalkyl having 1 to 5 halogen atoms and phenyl, or
Het represent a heterocycle of the formula (Het-10) 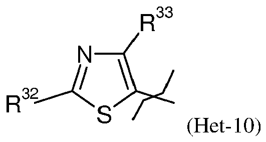 in which
   - R³²: is selected from the group consisting of C₁-C₄-alkyl and phenyl, and
   - R³³: is selected from the group consisting of C₁-C₄-alkyl and C₁-C₄-halogenoalkyl having 1 to 5 halogen atoms, or
Het represents a heterocycle of the formula (Het-11) 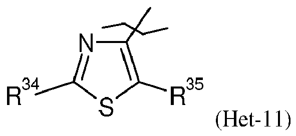 in which
   - R³⁴: is selected from the group consisting of C₁-C₄-alkyl, and
   - R³⁵: is selected from the group consisting of C₁-C₄-halogenoalkyl having 1 to 5 halogen atoms, or
Het represents a heterocycle of the formula (Het-12) 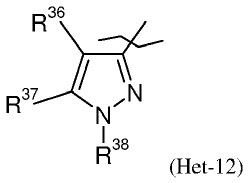 in which
   - R³⁶: is selected from the group consisting of C₁-C₄-halogenoalkyl having 1 to 5 halogen atoms, and
   - R¹⁷: ist selected from the group consisting of hydrogen, and
   - R³⁸: is selected from the group consisting of C₁-C₄-alkyl, or
Het represents a heterocycle of the formula (Het-13) 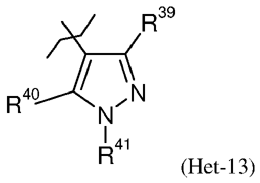 in which
   - R³⁹: is selected from the group consisting of C₁-C₄-alkyl, C₁-C₄-halogenoalkyl having 1 to 5 halogen atoms and C₁-C₄-alkoxy, and
   - R⁴°: is selected from the group consisting of halogen, and
   - R⁴¹: is selected from the group consisting of C₁-C₄-alkyl, or
Het represents a heterocycle of the formula (Het-14) 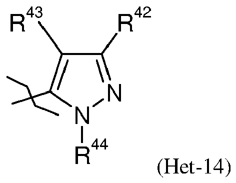 in which
   - R⁴²: is selected from the group consisting of C₁-C₄-alkyl and C₁-C₄-halogenoalkyl having 1 to 5 halogen atoms, and
   - R⁴³: is selected from the group consisting of halogen and C₁-C₄-alkyl, and
   - R⁴⁴: is selected from the group consisting of C₁-C₄-alkyl, or
Het represents a heterocycle of the formula (Het-15) 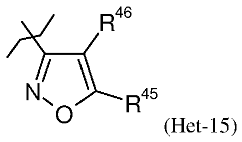 in which
   - R⁴⁵ and R⁴⁶: may be the same or different and are selected from the group consisting of hydrogen and C₁-C₄-alkyl, or
Het represents a heterocycle of the formula (Het-16) 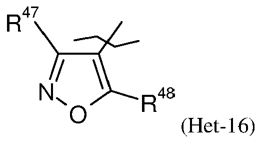 in which
   - R⁴⁷ and R⁴⁸: may be the same or different and are selected from the group consisting of C₁-C₄-alkyl, phenyl (optionally substituted by halogen), and heterocyclyl like thiadiazolyl, or
Het represents a heterocycle of the formula (Het-17) 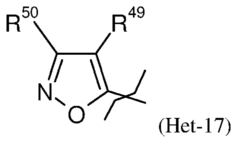 in which
   - R⁴⁹ and R⁵⁰: may be hydrogen, or
Het represents a heterocycle of the formula (Het-19) 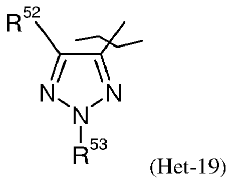 in which
   - R⁵²: is selected from the group consisting of C₁-C₄-alkyl and C₁-C₄-halogenoalkyl having 1 to 5 halogen atoms, and
   - R⁵³: is selected from the group consisting of C₁-C₄-alkyl and phenyl, or
Het represents a heterocycle of the formula (Het-20) 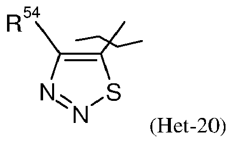 in which
   - R⁵⁴: is selected from the group consisting of C₁-C₄-alkyl, or
Het represents a heterocycle of the formula (Het-21) 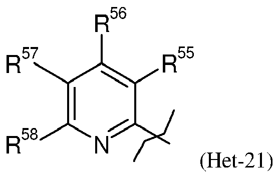 in which
   - R⁵⁵: is selected from the group consisting of hydrogen, halogen and C₁-C₄-halogenoalkyl having 1 to 5 halogen atoms, and
   - R⁵⁶, R⁵⁷ and R⁵⁸,: which may be the same or different, are selected from the group consisting of hydrogen, halogen and C₁-C₄-alkyl, or
Het represents a heterocycle of the formula (Het-22) 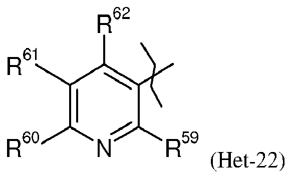 in which
   - R⁵⁹: is selected from the group consisting of hydrogen, halogen, C₁-C₄-alkyl, C₁-C₄-halogenoalkyl having 1 to 5 halogen atoms and -S-C₁-C₅-alkyl, and
   - R⁶⁰, R⁶¹ and R⁶²,: which may the same or different, are selected from the group consisting of hydrogen, halogen and C₁-C₄-alkyl, or
Het represents a heterocycle of the formula (Het-23) 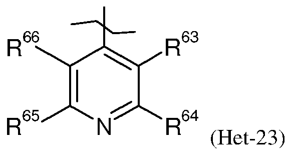 in which
   - R⁶³, R⁶⁴, R⁶⁵ and R⁶⁶,: which may be the same or different, are selected from the group consisting of hydrogen and halogen, or
Het represents a heterocycle of the formula (Het-24) 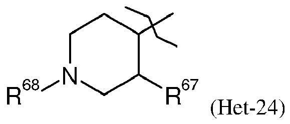 in which
   - R⁶⁷: is hydrogen, and
   - R⁶⁸: is selected from the group consisting of benzyloxycarbonyl and heterocyclyl like pyrimidinyl, (optionally substituted by C₁-C₄-halogenoalkyl having 1 to 5 halogen atoms), or
Het represents a heterocycle of the formula (Het-25) 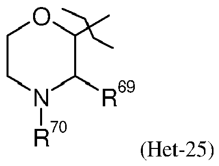 in which
   - R⁶⁹: is hydrogen, and
   - R⁷⁰: is benzyl, or
Het represents a heterocycle of the formula (Het-26) 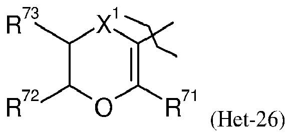 in which
   - X¹: is sulphur, and
   - R⁷¹: is selected from the group consisting of C₁-C₄-alkyl and C₁-C₄-halogenoalkyl having 1 to 5 halogen atoms, and
   - k⁷² and R⁷³: may be the same or different and are selected from the group consisting of hydrogen and C₁-C₄-alkyl;
as well as its salts, N-oxydes, metallic complexes, metalloidic complexes and optically active isomers.

A "nematicide" as used herein means that the compound is capable of controlling nematodes.

"Controlling nematodes" as used in the present invention means killing nematodes or preventing nematodes to develop or to grow. Controlling nematodes as used herein also encompasses controlling nematode progeny (development of viable cysts and/or egg masses). The compounds described herein, may be used to keep an organism healthy and may be used curatively, preventively or systematically to control nematodes.

The "organism" as mentioned in the above paragraphs may be a plant. When using the compounds described herein, to keep a plant healthy, the controlling of nematodes as used herein encompasses the reduction of damage to plants and/or encompasses increased yield.

Alternatively, the organisms as mentioned above may be a human or an animal. When using the compounds described herein to keep a human or animal healthy, the use encompasses therapeutic use and veterinarian use with the aim to prevent or to cure damage by nematodes.

"Nematodes" as used herein encompass all species of the order Nematoda and in particular species that are parasitic or cause health problems to plant or to fungi (for example species of the orders *Aphelenchida,* Meloidogyne, *Tylenchida* and others) or to humans and animals (for example species of the orders *Ascaradida, Oxyurida, Strongylida, Stronglyloides* and *Trichocephalida*).

Preferably, "nematodes" as used herein, refer to plant nematodes meaning plant parasitic nematodes that cause damage to plants. Plant nematodes encompass plant parasitic nematodes and nematodes living in the soil. Plant parasitic nematodes include, but are not limited to, ectoparasites such as *Xiphinema spp., Longidorus spp.,* and *Trichodorus spp.;* semiparasites such as *Tylenchulus spp.;* migratory endoparasites such as *Pratylenchus spp., Radopholus spp.,* and *Scutellonerna spp.;* sedentary parasites such as *Heterodera spp., Globoderal spp.,* and *Meloidogyne spp.,* and stem and leaf endoparasites such as *Ditylenchus spp., Aphelenchoides spp.,* and *Hirshmaniella spp..* The compounds described herein are distinguished especially for their effective control of halmful root parasitic soil nematodes such as, cystforming nematodes of the genera *Heterodera* or *Globodera,* and/or root knot nematodes of the genus *Meloidogyne.* Harmful species of these genera are for example *Meloidogyne incognata, Heterodera glycines* (soybean cyst nematode), *Globodera pallida* and *Globodera rostochiensis* (potato cyst nematode), which species are effectively controlled with the compounds described herein. However, the use of the compounds described herein is in no way restricted to these genera or species, but also extends in the same manner to other nematodes.

The compounds described herein may have a broad spectrum activity against various genera and/or strains and/or species of nematodes including but not limited to e.g. *Aglenchus agricola, Anguina tritici, Aphelenchoides arachidis, Aphelenchoides fragaria* and the stem and leaf endoparasites *Aphelenchoides spp.* in general, *Belonolaimus gracilis, Belonolaimus longicaudatus, Belonolaimus nortoni, Bursaphelenchus eremus, Bursaphelenchus xylophilus and Bursaphelenchus spp. in general, Cacopaurus pestis, Criconemella curvata, Criconemella onoensis, Criconemella ornata, Criconemella rusium, Criconemella xenoplax (= Mesocriconema xenoplax)* and *Criconemella spp.* in general, *Criconemoides femiae, Criconemoides onoense, Criconemoides ornatum* and *Criconemoides spp.* in general, *Ditylenchus destructor, Ditylenchus dipsaci, Ditylenchus myceliophagus* and the stem and leaf endoparasites *Ditylenchus spp.* in general, *Dolichodorus heterocephalus, Globodera pallida (=Heterodera pallida), Globodera rostochiensis* (potato cyst nematode), *Globodera solanacearum, Globodera tabacum, Globodera virginia* and the sedentary, cyst forming parasites *Globodera spp.* in general" *Helicotylenchus digonicus, Helicotylenchus dihystera, Helicotylenchus erythrine, Helicotylenchus multicinctus, Helicotylenchus nannus, Helicotylenchus pseudorobustus* and *Helicotylenchus spp.* in general, *Hemicriconemoides, Hemicycliophora arenaria, Hemicycliophora nudata, Hemicycliophora parvana, Heterodera avenae, Heterodera cruciferae, Heterodera glycines* (soybean cyst nematode), *Heterodera oryzae, Heterodera schachtii, Heterodera zeae* and the sedentary, cyst forming parasites *Heterodera spp.* in general, *Hirschmaniella gracilis, Hirschmaniella* oryzae *Hirschmaniella spinicaudata* and the stem and leaf endoparasites *Hirschmaniella spp.* in general, *Hoplolaimus aegyptii, Hoplolaimus californicus, Hoplolaimus columbus, Hoplolaimus galeatus, Hoplolaimus indicus, Hoplolaimus magnistylus, Hoplolaimus pararobustus, Longidorus africanus, Longidorus breviannulatus, Longidorus elongatus, Longidorus laevicapitatus, Longidorus vineacola* and the ectoparasites *Longidorus spp.* in general, *Meloidogyne acronea, Meloidogyne africana, Meloidogyne arenaria, Meloidogyne arenaria thamesi, Meloidogyne artiella, Meloidogyne chitwoodi, Meloidogyne coffeicola, Meloidogyne ethiopica, Meloidogyne exigua, Meloidogyne graminicola, Meloidogyne graminis, Meloidogyne hapla, Meloidogyne incognita, Meloidogyne incognita acrita, Meloidogyne javanica, Meloidogyne kikuyensis, Meloidogyne naasi, Meloidogyne paranaensis, Meloidogyne thamesi* and the sedentary parasites *Meloidogyne spp.* in general, *Meloinema spp., Nacobbus aberrans, Neotylenchus vigissi, Paraphelenchus pseudoparietinus, Paratrichodorus allius, Paratrichodorus lobatus, Paratrichodorus minor, Paratrichodorus nanus, Paratrichodorus porosus, Paratrichodorus teres* and *Paratrichodorus spp.* in general, *Paratylenchus hamatus, Paratylenchus minutus, Paratylenchus projectus and Paratylenchus spp.* in general, *Pratylenchus agilis, Pratylenchus alleni, Pratylenchus andinus, Pratylenchus brachyurus, Pratylenchus cerealis, Pratylenchus coffeae, Pratylenchus crenatus, Pratylenchus delattrei, Pratylenchus giibbicaudatus, Pratylenchus goodeyi, Pratylenchus hamatus, Pratylenchus hexincisus, Pratylenchus loosi, Pratylenchus neglectus, Pratylenchus penetrans, Pratylenchus pratensis, Pratylenchus scribneri, Pratylenchus teres, Pratylenchus thornei, Pratylenchus vulnus, Pratylenchus zeae* and the migratory endoparasites *Pratylenchus spp.* in general, *Pseudohalenchus minutus, Psilenchus magnidens, Psilenchus tumidus, Punctodera chalcoensis, Quinisulcius acutus, Radopholus citrophilus, Radopholus similis,* the migratory endoparasites *Radopholus spp.* in general, *Rotylenchulus borealis, Rotylenchulus parvus, Rotylenchulus reniformis* and *Rotylenchulus spp.* in general, *Rotylenchus laurentinus, Rotylenchus macrodoratus, Rotylenchus robustus, Rotylenchus uniformis* and *Rotylenchus spp.* in general, *Scutellonema brachyurum, Scutellonema bradys, Scutellonema clathricaudatum* and the migratory endoparasites *Scutellonema spp.* in general, *Subanguina radiciola, Tetylenchus nicotianae, Trichodorus cylindricus, Trichodorus minor, Trichodorus primitivus, Trichodorus proximus, Trichodorus similis, Trichodorus sparsus* and *the ectoparasites Trichodorus spp.* in general, *Tylenchorhynchus agri, Tylenchorhynchus brassicae, Tylenchorhynchus clarus, Tylenchorhynchus claytoni, Tylenchorhynchus digitatus, Tylenchorhynchus ebriensis, Tylenchorhynchus maximus, Tylenchorhynchus nudus, Tylenchorhynchus vulgaris and Tylenchorhynchus spp.* in general, *Tylenchulus semipenetrans and the semiparasites Tylenchulus spp. in general, Xiphinema americanum, Xiphinema brevicolle, Xiphinema* Nematicides comprising as an active ingredient a compound of formula (Ia) or (Ib) are suitable for the control of nematodes in soil in the fields of fruit trees, vegetables, other crops and ornamental plants.

Examples of nematodes to which a nematicide of the present invention is applicable include, but are not limited to, nematodes of the genus Meloidogyne such as the southern root-knot nematode (Meloidogyne incognita), Javanese root-knot nematode (Meloidogyne avanica), northern root-knot nematode (Meloidogyne hapla), and peanut root-knot nematode (Meloidogyne arenaria); nematodes of the genus Ditylenchus such as the potato rot nematode (Ditylenchus destructor) and bulb and stem nematode (Ditylenchus dipsaci); nematodes of the genus Pratylenchus such as the cob root-lesion nematode (Pratylenchus penetrans), chrysanthemum root-lesion nematode (Pratylenchus fallax), coffee root-lesion nematode (Pratylenchus coffeae), tea root-lesion nematode (Pratylenchus loosi), and walnut root-lesion nematode (Pratylenchus vulnus); nematodes of the genus Globodera such as the golden nematode (Globodera rostochiensis) and potato cyst nematode (Globodera pallida); nematodes of the genus Heterodera such as the soybean cyst nematode (Heterodera glycines) and sugar beet cyst nematode (Heterodera schachtii); nematodes of the genus Aphelenchoides such as the rice white-tip nematode (Aphelenchoides besseyi), chrysanthemum foliar nematode (Aphelenchoides ritzemabosi), and strawberry nematode (Aphelenchoides fragariae); nematodes of the genus Aphelenchus such as the mycophagous nematode (Aphelenchus avenae); nematodes of the genus Radopholus such as the burrowing nematode (Radopholus similis); nematodes of the genus Tylenchulus such as the citrus nematode (Tylenchulus semipenetrans); nematodes of the genus Rotylenchulus such as the reniform nematode (Rotylenchulus reniformis); nematodes that occur in trees, such as the pine wood nematode (Bursaphelenchus xylophilus), and the like. Furthermore, a nematocidal composition of the present invention is also effective against animal parasitic nematodes such as ascarid, oxyurid, anisakis, filaria, Wuchereria bancrofti, Onchocerca volvulus, and Gnathostoma.

Plants for which a nematicide of the present invention can be used are not particularly limited; for example, plants such as cereals (for example, rice, barley, wheat, rye, oat, corn, kaoliang 5 and the like), beans (soybean, azuki, bean, broad bean, peas, peanuts and the like), fruit trees/fruits (apples, citruses, pears, grapes, peaches, Japanese apricots, cherries, walnuts, almonds, bananas, strawberries and the like), vegetables (cabbage, tomato, spinach, broccoli, lettuce, onion, Welsh onion, pepper and the like), root crops (carrot, potato, sweet potato, radish, lotus root, turnip and the like), industrial crops (cotton, hemp, paper mulberry, mitsumata, rape, beet, hop, sugarcane, sugar beet, olive, rubber, coffee, tobacco, tea and the like), pepos (pumpkin, cucumber, watermelon, melon and the like), pasture plants (orchard grass, sorghum, thimosy, clover, alfalfa and the like), lawn grasses (mascarene grass, bent grass and the like), crops for flavorings etc. (lavender, rosemary, thyme, parsley, pepper, ginger and the like), and flower plants (chrysanthemum, rose, orchids and the like) can be mentioned.

Alternatively, "nematodes" as used herein, refer to nematodes which cause damage to humans or animals. Specific nematode species harmful to humans or animals are *Ascaris suum, Trichinella spiralis, Trichuris suis* (pig), *Ascaris lumbricoides, Trichinella sp.* (human), *Ostertagia ostertagi, Haemonchus placei, Cooperia oncophora, Dictyocaulus viviparus, Fasciola hepatica* (cattle), *Haemonchus contortus, Nematodirus battus* (sheep), *Strongyloides sp.* (horse), *Ancylostoma caninum, Toxocara canis* (dog), *Toxocara cati, Taenia taeniaeformis* (cat). Moreover, many known nematicides are equally active as anthelmintic and are used to control human and animal parasitic worms, which do not necessarily belong to the group of nematoda. Therefore, it is envisaged by the present invention that the compounds described herein may also be used as anthelmintic.

A further aspect of the invention are nematicidal compositions, comprising an effective amount of at least one compound as defined herein and at least one of the following: surfactant, solid or liquid diluent, characterized in that the surfactant or the diluent is normally used in nematicidal compositions. In an embodiment, said composition comprises at least two compounds as defined herein.

A related aspect of the invention is a method for preparing a nematicidal composition as described herein, comprising the step of mixing at least one compound as described herein with a surfactant or diluent normally used in nematicidal compositions. In an embodiment, said method comprises mixing least two compounds as defined herein with a surfactant or diluent normally used in nematicidal compositions.

In particular, the present invention relates to nematicidal composition developed to be used inagriculture or horticulture. These nematicidal compositions may be prepared in a manner known per se. The present invention further provides formulations, and application forms prepared from them, as crop protection agents and/or pesticidal agents, such as drench, drip and spray liquors, comprising at least one of the active compounds of the invention. The application forms may comprise further crop protection agents and/or pesticidal agents, and/or activity-enhancing adjuvants such as penetrants, examples being vegetable oils such as, for example, rapeseed oil, sunflower oil, mineral oils such as, for example, liquid paraffins, alkyl esters of vegetable fatty acids, such as rapeseed oil or soybean oil methyl esters, or alkanol alkoxylates, and/or spreaders such as, for example, alkylsiloxanes and/or salts, examples being organic or inorganic ammonium or phosphonium salts, examples being ammonium sulphate or diammonium hydrogen phosphate, and/or retention promoters such as dioctyl sulphosuccinate or hydroxypropylguar polymers and/or humectants such as glycerol and/or fertilizers such as ammonium, potassium or phosphorous fertilizers, for example.

Examples of typical formulations include water-soluble liquids (SL), emulsifiable concentrates (EC), emulsions in water (EW), suspension concentrates (SC, SE, FS, OD), water-dispersible granules (WG), granules (GR) and capsule concentrates (CS); these and other possible types of formulation are described, for example, by Crop Life International and in Pesticide Specifications, Manual on development and use of FAO and WHO specifications for pesticides, FAO Plant Production and Protection Papers - 173, prepared by the FAO/WHO Joint Meeting on Pesticide Specifications, 2004,ISBN: 9251048576. The formulations may comprise active agrochemical compounds other than one or more active compounds of the invention.

The formulations or application forms in question preferably comprise auxiliaries, such as extenders, solvents, spontaneity promoters, carriers, emulsifiers, dispersants, frost protectants, biocides, thickeners and/or other auxiliaries, such as adjuvants, for example. An adjuvant in this context is a component which enhances the biological effect of the formulation, without the component itself having a biological effect. Examples of adjuvants are agents which promote the retention, spreading, attachment to the leaf surface, or penetration.

These formulations are produced in a known manner, for example by mixing the active compounds with auxiliaries such as, for example, extenders, solvents and/or solid carriers and/or further auxiliaries, such as, for example, surfactants. The formulations are prepared either in suitable plants or else before or during the application.

Suitable for use as auxiliaries are substances which are suitable for imparting to the formulation of the active compound or the application forms prepared from these formulations (such as, e.g., usable crop protection agents, such as spray liquors or seed dressings) particular properties such as certain physical, technical and/or biological properties.

Suitable extenders are, for example, water, polar and nonpolar organic chemical liquids, for example from the classes of the aromatic and non-aromatic hydrocarbons (such as paraffins, alkylbenzenes, alkylnaphthalenes, chlorobenzenes), the alcohols and polyols (which, if appropriate, may also be substituted, etherified and/or esterified), the ketones (such as acetone, cyclohexanone), esters (including fats and oils) and (poly)ethers, the unsubstituted and substituted amines, amides, lactams (such as Nalkylpyrrolidones) and lactones, the sulphones and sulphoxides (such as dimethyl sulphoxide).

If the extender used is water, it is also possible to employ, for example, organic solvents as auxiliary solvents. Essentially, suitable liquid solvents are: aromatics such as xylene, toluene or alkylnaphthalenes, chlorinated aromatics and chlorinated aliphatic hydrocarbons such as chlorobenzenes, chloroethylenes or methylene chloride, aliphatic hydrocarbons such as cyclohexane or paraffins, for example petroleum fractions, mineral and vegetable oils, alcohols such as butanol or glycol and also their ethers and esters, ketones such as acetone, methyl ethyl ketone, methyl isobutyl ketone or cyclohexanone, strongly polar solvents such as dimethylformamide and dimethyl sulphoxide, and also water.

In principle it is possible to use all suitable solvents. Suitable solvents are, for example, aromatic hydrocarbons, such as xylene, toluene or alkylnaphthalenes, for example, chlorinated aromatic or aliphatic hydrocarbons, such as chlorobenzene, chloroethylene or methylene chloride, for example, aliphatic hydrocarbons, such as cyclohexane, for example, paraffins, petroleum fractions, mineral and vegetable oils, alcohols, such as methanol, ethanol, isopropanol, butanol or glycol, for example, and also their ethers and esters, ketones such as acetone, methyl ethyl ketone, methyl isobutyl ketone or cyclohexanone, for example, strongly polar solvents, such as dimethyl sulphoxide, and water.

All suitable carriers may in principle be used. Suitable carriers are in particular: for example, ammonium salts and ground natural minerals such as kaolins, clays, talc, chalk, quartz, attapulgite, montmorillonite or diatomaceous earth, and ground synthetic minerals, such as finely divided silica, alumina and natural or synthetic silicates, resins, waxes and/or solid fertilizers. Mixtures of such carriers may likewise be used. Carriers suitable for granules include the following: for example, crushed and fractionated natural minerals such as calcite, marble, pumice, sepiolite, dolomite, and also synthetic granules of inorganic and organic meals, and also granules of organic material such as sawdust, paper, coconut shells, maize cobs and tobacco stalks.

Liquefied gaseous extenders or solvents may also be used. Particularly suitable are those extenders or carriers which at standard temperature and under standard pressure are gaseous, examples being aerosol propellants, such as halogenated hydrocarbons, and also butane, propane, nitrogen and carbon dioxide. Examples of emulsifiers and/or foam-formers, dispersants or wetting agents having ionic or nonionic properties, or mixtures of these surface-active substances, are salts of polyacrylic acid, salts of lignosulphonic acid, salts of phenolsulphonic acid or naphthalenesulphonic acid, polycondensates of ethylene oxide with fatty alcohols or with fatty acids or with fatty amines, with substituted phenols (preferably alkylphenols or arylphenols), salts of sulphosuccinic esters, taurine derivatives 5 (preferably alkyltaurates), phosphoric esters of polyethoxylated alcohols or phenols, fatty acid esters of polyols, and derivatives of the compounds containing sulphates, sulphonates and phosphates, examples being Alkylaryl polyglycol ethers, alkylsulphonates, alkyl sulphates, arylsulphonates, protein hydrolysates, lignin-sulphite waste liquors and methylcellulose. The presence of a surface-active substance is advantageous if one of the active compounds and/or one of the inert carriers is not soluble in water and if application takes place in water.

Further auxiliaries that may be present in the formulations and in the application forms derived from them include colorants such as inorganic pigments, examples being iron oxide, titanium oxide, Prussian Blue, and organic dyes, such as alizarin dyes, azo dyes and metal phthalocyanine dyes, and nutrients and trace nutrients, such as salts of iron, manganese, boron, copper, cobalt, molybdenum and zinc.

Stabilizers, such as low-temperature stabilizers, preservatives, antioxidants, light stabilizers or other agents which improve chemical and/or physical stability may also be present. Additionally present may be foam-formers or defoamers.

Furthermore, the formulations and application forms derived from them may also comprise, as additional auxiliaries, stickers such as carboxymethylcellulose, natural and synthetic polymers in powder, granule or latex form, such as gum arabic, polyvinyl alcohol, polyvinyl acetate, and also natural phospholipids, such as cephalins and lecithins, and synthetic phospholipids. Further possible auxiliaries include mineral and vegetable oils.

There may possibly be further auxiliaries present in the formulations and the application forms derived from them. Examples of such additives include fragrances, protective colloids, binders, adhesives, thickeners, thixotropic substances, penetrants, retention promoters, stabilizers, sequestrants, complexing agents, humectants and spreaders. Generally speaking, the active compounds may be combined with any solid or liquid additive commonly used for formulation purposes.

Suitable retention promoters include all those substances which reduce the dynamic surface tension, such as dioctyl sulphosuccinate, or increase the viscoelasticity, such as hydroxypropylguar polymers, for example.

Suitable penetrants in the present context include all those substances which are typically used in order to enhance the penetration of active agrochemical compounds into plants. Penetrants in this context are defined in that, from the (generally aqueous) application liquor and/or from the spray coating, they areable to penetrate the cuticle of the plant and thereby increase the mobility of the active compounds in thecuticle. This property can be determined using the method described in the literature (Baur et al., 1997, Pesticide Science 51, 131-152). Examples include alcohol alkoxylates such as coconut fatty ethoxylate (10) or isotridecyl ethoxylate (12), fatty acid esters such as rapeseed or soybean oil methyl esters, fatty amine alkoxylates such as tallowamine ethoxylate (15), or ammonium and/or phosphonium salts such as ammonium sulphate or diammonium hydrogen phosphate, for example.

The formulations preferably comprise between 0.00000001% and 98% by weight of active compound or, with particular preference, between 0.01% and 95% by weight of active compound, more preferably between 0.5% and 90% by weight of active compound, based on the weight of the formulation.

The active compound content of the application forms (crop protection products) prepared from the formulations may vary within wide ranges. The active compound concentration of the application forms may be situated typically between 0.00000001% and 95% by weight of active compound, preferably between 0.00001 % and 1% by weight, based on the weight of the application form. Application takes place in a customary manner adapted to the application forms.

The compounds are applied in a customary manner appropriate for the use forms.

All plants and plant parts can be treated in accordance with the invention. Plants are understood here to mean all plants and plant populations, such as desired and undesired wild plants or crop plants (including naturally occurring crop plants). Crop plants may be plants which can be obtained by conventional breeding and optimization methods or by biotechnological and genetic engineering methods or combinations of these methods, including the transgenic plants and including the plant varieties which are protectable and non-protectable by plant breeders' rights. Parts of plants shall be understood to mean all above-ground and below-ground parts and organs of plants, such as shoot, leaf, flower and root, examples including leaves, needles, stems, trunks, flowers, fruit bodies, fruits and seeds, and also roots, tubers and rhizomes. The plant parts also include harvested material and vegetative and generative propagation material, for example cuttings, tubers, rhizomes, slips and seeds.

The inventive treatment of the plants and plant parts with the active ingredients is effected directly or by allowing them to act on the surroundings, habitat or storage space thereof by the customary treatment methods, for example by dipping, spraying, evaporating, fogging, scattering, painting on, injecting, and, in the case of propagation material, especially in the case of seeds, also by applying one or more coats.

As already mentioned above, it is possible to treat all plants and their parts in accordance with the invention. In a preferred embodiment, wild plant species and plant cultivars, or those obtained by conventional biological breeding methods, such as crossing or protoplast fusion, and also parts thereof, are treated. In a further preferred embodiment, transgenic plants and plant cultivars obtained by genetic engineering, if appropriate in combination with conventional methods (Genetically Modified Organisms), and parts thereof are treated. The terms "parts" or "parts of plants" or "plant parts" have been explained above.

More preferably, plants of the plant cultivars which are each commercially available or in use are treated in accordance with the invention. Plant cultivars are understood to mean plants having new properties ("traits") and which have been obtained by conventional breeding, by mutagenesis or by recombinant DNA techniques. They may be cultivars, biotypes and genotypes.

Depending on the plant species or plant cultivars, and the location and growth conditions (soils, climate, vegetation period, diet) thereof, the inventive treatment may also result in superadditive ("synergistic") effects. For example, possibilities include reduced application rates and/or broadening of the activity spectrum and/or an increase in the activity of the compounds and compositions usable in accordance with the invention, better plant growth, increased tolerance to high or low temperatures, increased tolerance to drought or to levels of water or soil salinity, enhanced flowering performance, easier harvesting, accelerated ripening, higher yields, higher quality and/or higher nutritional value of the harvested products, increased storage life and/or processibility of the harvested products, which exceed the effects normally to be expected.

The transgenic plants or plant cultivars (those obtained by genetic engineering) which are to be treated with preference in accordance with the invention include all plants which, through the genetic modification, received genetic material which imparts particular advantageous useful properties ("traits") to these plants. Examples of such properties are better plant growth, increased tolerance to high or low temperatures, increased tolerance to drought or to levels of water or soil salinity, enhanced flowering performance, easier harvesting, accelerated ripening, higher yields, higher quality and/or a higher nutritional value of the harvested products, better storage life and/or processibility of the harvested products. Further and particularly emphasized examples of such properties are an improved defence of the plants against animal and microbial pests, such as against insects, mites, phytopathogenic fungi, bacteria and/or viruses, and also increased tolerance of the plants to certain herbicidally active ingredients. Examples of transgenic plants include the important crop plants, such as cereals (wheat, rice), maize, soya, potatoes, sugar beet, tomatoes, peas and other vegetable types, cotton, tobacco, oilseed rape, and also fruit plants (with the fruits of apples, pears, citrus fruits and grapes), particular emphasis being given to maize, soya, potatoes, cotton, tobacco and oilseed rape. Traits that are particularly emphasized are improved defence of the plants against insects, arachnids, nematodes, slugs and snails by toxins formed in the plants, especially those formed in the plants by the genetic material from Bacillus thuringiensis (for example by the genes CryIA(a), CryIA(b), CryIA(c), CryIIA, CryIIIA, CryIIIB2, Cry9c, Cry2Ab, Cry3Bb and CryIF, and also combinations thereof) (referred to hereinafter as "Bt plants"). Traits that are also particularly emphasized are the improved defence of plants against fungi, bacteria and viruses by systemic acquired resistance (SAR), systemin, phytoalexins, elicitors and also resistance genes and correspondingly expressed proteins and toxins. Traits that are additionally particularly emphasized are the increased tolerance of the plants to certain active herbicidal ingredients, for example imidazolinones, sulphonylureas, glyphosate or phosphinothricin (for example the "PAT" gene). The genes which impart the desired traits in question may also be present in combinations with one another in the transgenic plants. Examples of "Bt plants" include maize varieties, cotton varieties, soya varieties and potato varieties which are sold under the trade names YIELD GARD® (for example maize, cotton, soya), KnockOut® (for example maize), StarLink® (for example maize), Bollgard® (cotton), Nucotn® (cotton) and NewLeaf® (potato). Examples of herbicide-tolerant plants include maize varieties, cotton varieties and soya varieties which are sold under the trade names Roundup Ready® (tolerance to glyphosate, for example maize, cotton, soya), Liberty Link® (tolerance to phosphinothricin, for example oilseed rape), IMI® (tolerance to imidazolinones) and STS® (tolerance to sulphonylureas, for example maize). Herbicide-resistant plants (plants bred in a conventional manner for herbicide tolerance) which may be mentioned include the varieties sold under the name Clearfield® (for example maize). Of course, these statements also apply to plant cultivars which have these genetic traits or genetic traits which are still to be developed and will be developed and/or marketed in the future.

The plants listed can be treated in accordance with the invention in a particularly advantageous manner with the compounds of the general formula (I) and/or the active ingredient mixtures according to the invention. The preferred ranges stated above for the active ingredients or mixtures also apply to the treatment of these plants. Particular emphasis is given to the treatment of plants with the compounds or mixtures specifically mentioned in the present text.

The inventive active ingredient may be present in its commercially available formulations and in the use forms, prepared from these formulations, as a mixture with other active ingredients, such as insecticides, attractants, sterilants, bactericides, acaricides, nematicides, fungicides, growth regulators, herbicides, safeners, fertilizers or semiochemicals. The mixtures thus obtained have a broadened spectrum of activity.

Mixtures with fungicides are particularly advantageous. Examples of suitable fungicide mixing partners can be selected from the list consisting of
(1) Inhibitors of the ergosterol biosynthesis, for example aldimorph, azaconazole, bitertanol, bromuconazole, cyproconazole, diclobutrazole, difenoconazole, diniconazole, diniconazole-M, dodemorph, dodemorph acetate, epoxiconazole, etaconazole, fenarimol, fenbuconazole, fenhexamid, fenpropidin, fenpropimorph, fluquinconazole, flurprimidol, flusilazole, flutriafol, furconazole, furconazole-cis, hexaconazole, imazalil, imazalil sulfate, imibenconazole, ipconazole, metconazole, myclobutanil, naftifine, nuarimol, oxpoconazole, paclobutrazol, pefurazoate, penconazole, piperalin, prochloraz, propiconazole, prothioconazole, pyributicarb, pyrifenox, quinconazole, simeconazole, spiroxamine, tebuconazole, terbinafine, tetraconazole, triadimefon, triadimenol, tridemorph, triflumizole, triforine, triticonazole, uniconazole, uniconazole-p, viniconazole, voriconazole, 1-(4- chlorophenyl)-2-(1H-1,2,4-triazol-1-yl)cycloheptanol, methyl 1-(2,2-dimethyl-2,3-dihydro-1H-inden-1-yl)-1H-imidazole-5-carboxylate, N'-{5-(difluoromethyl)-2-methyl-4-[3-(trimethylsilyl)propoxy]phenyl}-N-ethyl-N-methylimidoformamide, N-ethyl-N-methyl-N'-{2-methyl-5-(trifluoromethyl)-4-[3-(trimethylsilyl)propoxy]phenyl}imidoformamide and O-[1-(4-methoxyphenoxy)- 3,3-dimethylbutan-2-yl]1H-imidazole-1-carbothioate.
(2) inhibitors of the respiratory chain at complex I or II, for example bixafen, boscalid, carboxin, diflumetorim, fenfuram, fluopyram, flutolanil, fluxapyroxad, furametpyr, furmecyclox, isopyrazam (mixture of syn-epimeric racemate 1RS,4SR,9RS and anti-epimeric racemate 1RS,4SR,9SR), isopyrazam (anti-epimeric racemate 1RS,4SR,9SR), isopyrazam (anti-epimeric enantiomer 1R,4S,9S), isopyrazam (anti-epimeric enantiomer 1S,4R,9R), isopyrazam (syn epimeric racemate 1RS,4SR,9RS), isopyrazam (syn-epimeric enantiomer 1R,4S,9R), isopyrazam (syn-epimeric enantiomer 1S,4R,9S), mepronil, oxycarboxin, penflufen, penthiopyrad, sedaxane, thifluzamide, 1-methyl-N-[2-(1,1,2,2- tetrafluoroethoxy)phenyl]-3-(trifluoromethyl)-1H-pyrazole-4-carboxamide, 3-(difluoromethyl)-1-methyl-N-[2-(1,1,2,2-tetrafluoroethoxy)phenyl]-1H-pyrazole-4-carboxamide, 3-(difluoromethyl)-N-[4-fluoro-2-(1,1,2,3,3,3-hexafluoropropoxy)phenyl]-1-methyl-1H-pyrazole-4-carboxamide, N-[1-(2,4-dichlorophenyl)-1-methoxypropan-2-yl]-3-(difluoromethyl)-1-methyl-1H-pyrazole-4-carboxamide, 5,8-difluoro-N-[2-(2-fluoro-4-{[4-(trifluoromethyl)pyridin-2-yl]oxy}phenyl)ethyl]quinazolin-4-amine, N-[9-(dichloromethylene)-1,2,3,4-tetrahydro-1,4-methanonaphthalen-5-yl]-3-(difluoromethyl)-1-methyl-1H-pyrazole-4-carboxamide, N-[(1S,4R)-9-(dichloromethylene)-1,2,3,4-tetrahydro-1,4-methanonaphthalen-5-yl]-3-(difluoromethyl)-1-methyl-1H-pyrazole-4-carboxamide and N-[(1R,4S)-9-(dichloromethylene)-1,2,3,4-tetrahydro-1,4-methanonaphthalen-5-yl]-3-(difluoromethyl)-1-methyl-1Hpyrazole-4-carboxamide.
(3) inhibitors of the respiratory chain at complex III, for example ametoctradin, amisulbrom, azoxystrobin, cyazofamid, coumethoxystrobin, coumoxystrobin, dimoxystrobin, enestroburin, famoxadone, fenamidone, fenoxystrobin, fluoxastrobin, kresoxim-methyl, metominostrobin, orysastrobin, picoxystrobin, pyraclostrobin, pyrametostrobin, pyraoxystrobin, pyribencarb, triclopyricarb, trifloxystrobin, (2E)-2-(2-{[6-(3-chloro-2-methylphenoxy)-5-fluoropyrimidin-4-yl]oxy}phenyl)-2-(methoxyimino)-N-methylethanamide, (2E)-2-(methoxyimino)-N-methyl-2-(2-{[({(1E)-1-[3-(trifluoromethyl)phenyl]ethylidene}amino)oxy]methyl}phenyl)ethanamide, (2E)-2-(methoxyimino)-N-methyl-2-{2-[(E)-({1-[3-(trifluoromethyl)phenyl]ethoxy}imino)methyl]phenyl}ethanamide, (2E)-2-{2-[({[(1E)-1-(3-{[(E)-1-fluoro-2-phenylethenyl]oxy}phenyl)ethylidene]amino}oxy)methyl]phenyl}-2-(methoxyimino)-N-methylethanamide, (2E)-2-{2-[({[(2E,3E)-4-(2,6-dichlorophenyl)but-3-en-2-ylidene]amino}oxy)methyl]phenyl}-2-(methoxyimino)-N-methylethanamide, 2-chloro-N-(1,1,3-trimethyl-2,3-dihydro-1H-inden-4-yl)pyridine-3-carboxamide, 5-methoxy-2-methyl-4-(2-{[({(1E)-1-[3-(trifluoromethyl)phenyl]ethylidene}amino)oxy]methyl}phenyl)-2,4-dihydro-3H-1,2,4-triazol-3-one, methyl (2E)-2-{2-[({cyclopropyl[(4-methoxyphenyl)imino]methyl}sulfanyl)methyl]phenyl}-3-methoxyprop-2-enoate, N-(3-ethyl-3,5,5-trimethylcyclohexyl)-3-(formylamino)-2-hydroxybenzamide, 2-{2-[(2,5-dimethylphenoxy)methyl]phenyl}-2-methoxy-N-methylacetamide and 5 (2R)-2-{2-[(2,5-dimethylphenoxy)methyl]phenyl}-2-methoxy-N-methylacetamide.
(4) Inhibitors of the mitosis and cell division, for example benomyl, carbendazim, chlorfenazole, diethofencarb, ethaboxam, fluopicolide, fuberidazole, pencycuron, thiabendazole, thiophanate-methyl, thiophanate, zoxamide, 5-chloro-7-(4-methylpiperidin-1-yl)-6-(2,4,6-trifluorophenyl)[1,2,4]triazolo[1,5-a]pyrimidine and 3-chloro-5-(6-chloropyridin-3-yl)-6-methyl-4-(2,4,6-trifluorophenyl)pyridazine.
(5) Compounds capable to have a multisite action, for example bordeaux mixture, captafol, captan, chlorothalonil, copper hydroxide, copper naphthenate, copper oxide, copper oxychloride, copper(2+) sulfate, dichlofluanid, dithianon, dodine, dodine free base, ferbam, fluorofolpet, folpet, guazatine, guazatine acetate, iminoctadine, iminoctadine albesilate, iminoctadine triacetate, mancopper, mancozeb, maneb, metiram, metiram zinc, oxine-copper, propamidine, propineb, sulphur and sulphur preparations including calcium polysulphide, thiram, tolylfluanid, zineb and ziram.
(6) Compounds capable to induce a host defence, for example acibenzolar-S-methyl, isotianil, probenazole and tiadinil.
(7) Inhibitors of the amino acid and/or protein biosynthesis, for example andoprim, blasticidin-S, cyprodinil, kasugamycin, kasugamycin hydrochloride hydrate, mepanipyrim, pyrimethanil and 3-(5-fluoro-3,3,4,4-tetramethyl-3,4-dihydroisoquinolin-1-yl)quinoline.
(8) Inhibitors of the ATP production, for example fentin acetate, fentin chloride, fentin hydroxide andsilthiofam.
(9) Inhibitors of the cell wall synthesis, for example benthiavalicarb, dimethomorph, flumorph, iprovalicarb, mandipropamid, polyoxins, polyoxorim, validamycin A and valifenalate.
(10) Inhibitors of the lipid and membrane synthesis, for example biphenyl, chloroneb, dicloran, edifenphos, etridiazole, iodocarb, iprobenfos, isoprothiolane, propamocarb, propamocarb hydrochloride, prothiocarb, pyrazophos, quintozene, tecnazene and tolclofos-methyl.
(11) Inhibitors of the melanine biosynthesis, for example carpropamid, diclocymet, fenoxanil, phthalide, pyroquilon, tricyclazole and 2,2,2-trifluoroethyl {3-methyl-1-[(4-methylbenzoyl)amino]butan-2-yl}carbamate.
(12) Inhibitors of the nucleic acid synthesis, for example benalaxyl, benalaxyl-M (kiralaxyl), bupirimate, clozylacon, dimethirimol, ethirimol, furalaxyl, hymexazol, metalaxyl, metalaxyl-M (mefenoxam), ofurace, oxadixyl and oxolinic acid.
(13) Inhibitors of the signal transduction, for example chlozolinate, fenpiclonil, fludioxonil, iprodione, procymidone, quinoxyfen 5 and vinclozolin.
(14) Compounds capable to act as an uncoupler, for example binapacryl, dinocap, ferimzone, fluazinam and meptyldinocap.
(15) Further compounds, for example benthiazole, bethoxazin, capsimycin, carvone, chinomethionat, pyriofenone (chlazafenone), cufraneb, cyflufenamid, cymoxanil, cyprosulfamide, dazomet, debacarb, dichlorophen, diclomezine, difenzoquat, difenzoquat methylsulphate, diphenylamine, ecomate, fenpyrazamine, flumetover, fluoroimide, flusulfamide, flutianil, fosetyl-aluminium, fosetyl-calcium, fosetyl-sodium, hexachlorobenzene, irumamycin, methasulfocarb, methyl isothiocyanate, metrafenone, mildiomycin, natamycin, nickel dimethyldithiocarbamate, nitrothal-isopropyl, octhilinone, oxamocarb, oxyfenthiin, pentachlorophenol and salts, phenothrin, phosphorous acid and its salts, propamocarb-fosetylate, propanosine-sodium, proquinazid, pyrimorph, (2E)-3-(4-tert-butylphenyl)-3-(2-chloropyridin-4-yl)-1-(morpholin-4-yl)prop-2-en-1-one, (2Z)-3-(4-tert-butylphenyl)-3-(2-chloropyridin-4-yl)-1-(morpholin-4-yl)prop-2-en-1-one, pyrrolnitrine, tebufloquin, tecloftalam, tolnifanide, triazoxide,trichlamide, zarilamid, (3S,6S,7R,8R)-8-benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoate, 1-(4-{4-[(SR)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone, 1-(4-{4-[(SS)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone,1-(4-{4-[5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone, 1-(4-methoxyphenoxy)-3,3-dimethylbutan-2-yl-1H-imidazole-1-carboxylate, 2,3,5,6-tetrachloro-4-(methylsulfonyl)pyridine, 2,3-dibutyl-6-chlorothieno[2,3-d]pyrimidin-4(3H)-one, 2,6-dimethyl-1H,5H-[1,4]dithiino[2, 3-c:5,6-c']dipyrrole-1,3,5,7(2H,6H)-tetrone, 2-[5-methyl-3-(trifluoromethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanone, 2-[5-methyl-3-(trifluoromethyl)-1Hpyrazol-1-yl]-1-(4-{4-[(5S)-5-phenyl-4,5-dihydro-1,2-oxazol-3-yl]-1,3-thiazol-2-yl}piperidin-1-yl)ethanone, 2-[5-methyl-3-(trifluoromethyl)-1H-pyrazol-1-yl]-1-{4-[4-(5-phenyl-4,5-dihydro-1,2-oxazol-3-yl)-1,3-thiazol-2-yl]piperidin-1-yl}ethanone, 2-butoxy-6-iodo-3-propyl-4H-chromen-4-one, 2-chloro-5-[2-chloro-1-(2,6-difluoro-4-methoxyphenyl)-4-methyl-1H-imidazol-5-yl]pyridine, 2-phenylphenol and salts, 3-(4,4,5-trifluoro-3,3-dimethyl-3,4-dihydroisoquinolin-1-yl)quinoline, 3,4,5-trichloropyridine-2,6-dicarbonitrile, 3-[5-(4-chlorophenyl)-2,3-dimethyl-1,2-oxazolidin-3-yl]pyridine, 3-chloro-5-(4-chlorophenyl)-4-(2,6-difluorophenyl)-6-methylpyridazine, 4-(4-chlorophenyl)-5-(2,6-difluorophenyl)-3,6-dimethylpyridazine, 5-amino-1,3,4-thiadiazole-2-thiol, 5-chloro-N'-phenyl-N'-(prop-2-yn-1-yl)thiophene-2-sulfonohydrazide, 5-fluoro-2-[(4-fluorobenzyl)oxy]pyrimidin-4-amine, 5-fluoro-2-[(4-methylbenzyl)oxy]pyrimidin-4-amine, 5-methyl-6-octyl[1,2,4]triazolo[1,5-a]pyrimidin-7-amine, ethyl (2Z)-3-amino-2-cyano-3-phenylprop-2-enoate, N'-(4-{[3-(4-chlorobenzyl)-1,2,4-thiadiazol-5-yl]oxy}-2,5-dimethylphenyl)-N-ethyl-N-methylimidoformamide, N-(4-chlorobenzyl)-3-[3-methoxy-4-(prop-2-yn-1-yloxy)phenyl]propanamide, N-[(4-chlorophenyl)(cyano)methyl]-3-[3-methoxy-4-(prop-2-yn-1-yloxy)phenyl]propanamide, N-[(5-bromo-3-chloropyridin-2-yl)methyl]-2,4-dichloropyridine-3-carboxamide, N-[1-(5-bromo-3-chloropyridin-2-yl)ethyl]-2,4-dichloropyridine-3-carboxamide, N-[1-(5-bromo-3-chloropyridin-2-yl)ethyl]-2-fluoro-4-iodopyridine-3-carboxamide, N-{(E)-[(cyclopropylmethoxy)imino] [6-(difluoromethoxy)-2,3-difluorophenyl]methyl}-2-phenylacetamide, N-{(Z)-[(cyclopropylmethoxy)imino][6-(difluoromethoxy)-2,3-difluorophenyl]methyl}-2-phenylacetamide, N'-{4-[(3-tert-butyl-4-cyano-1,2-thiazol-5-yl)oxy]-2-chloro-5-methylphenyl}-N-ethyl-N-methylimidoformamide, N-methyl-2-(1-{[5-methyl-3-(trifluoromethyl)-1H-pyrazol-1-yl]acetyl}piperidin-4-yl)-N-(1,2,3,4-tetrahydronaphthalen-1-yl)-1,3-thiazole-4-carboxamide, N-methyl-2-(1-{[5-methyl-3-(trifluoromethyl)-1H-pyrazol-1-yl]acetyl}piperidin-4-yl)-N-[(1R)-1,2,3,4-tetrahydronaphthalen-1-yl]-1,3-thiazole-4-carboxamide, N-methyl-2-(1-{[5-methyl-3-(trifluoromethyl)-1H-pyrazol-1-yl]acetyl }piperidin-4-yl)-N-[(IS)-1,2,3,4-tetrahydronaphthalen- 1-yl]-1,3-thiazole-4-carboxamide, pentyl {6-[(1[(1-methyl-1H-tetrazol-5-yl)(phenyl)methylidene]amino]oxy)methyl]pyridin-2-yl}carbamate, phenazine-1-carboxylic acid, quinolin-8-ol, quinolin-8-ol sulfate (2:1) and tert-butyl {6-[({[(1-methyl-1H-tetrazol-5-yl)(phenyl)methylene]amino}oxy)methyl]pyridin-2-yl}carbamate.

The active ingredients specified herein by their "common name" are known and described, for example, in the Pesticide Manual ("The Pesticide Manual", 14th Ed., British Crop Protection Council 2006) or can be searched in the internet (e.g. http://www.alanwood.net/pesticides).

The composition according to the invention comprising a mixture with a bactericide compound may also be particularly advantageous. Examples of suitable bactericide mixing partners may be selected in the list consisting of bronopol, dichlorophen, nitrapyrin, nickel dimethyldithiocarbamate, kasugamycin, octhilinone, furancarboxylic acid, oxytetracycline, probenazole, streptomycin, tecloftalam, copper sulphate and other copper preparations.

The various aspects of the invention will now be illustrated with reference to the following tables of compounds and examples. The following tables illustrate in a non limiting manner examples of compounds according to the invention.

Abbreviations which are used in the tables include the following:

Me = CH₃

Et = C₂H₅

n-Pr = CH₂CH₂CH₃

tert butyl = C(CH₃)₃ = t-Bu

cyclo-Pr = cyclopropyl

C₆H₅ = phenyl

comm. av. = commercially available

The dotted line in the structures of "A" depicts the bond connecting A to the C(O)NR⁵-moiety.

**Table A**

| | | | | | | | | |
|---|---|---|---|---|---|---|---|---|
| 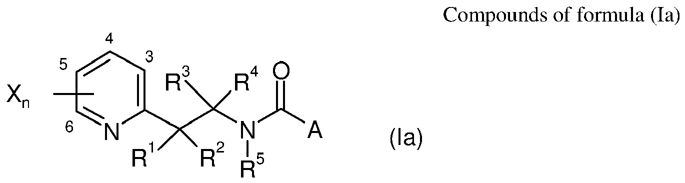 | | | | | | | | |

| **No.** | **Xₙ** | **R¹** | **R²** | **R³** | **R⁴** | **R⁵** | **A** | **cf.** |
|---|---|---|---|---|---|---|---|---|
| Ia-1 | 3-Cl 5-CF₃ | H | H | CH₃ | H | H | 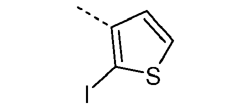 | P10 |
| Ia-2 | 3-Cl 5-CF₃ | H | H | CH₃ | H | H | 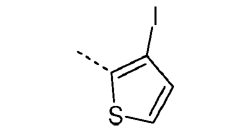 | P10 |
| Ia-3 | 3-Cl 5-CF₃ | H | H | H | H | H | 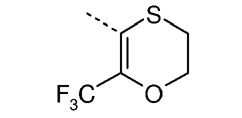 | P9, cpd. V-2 |
| Ia-4 | 3-Cl 5-CF₃ | H | H | H | H | H | 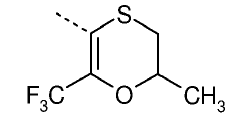 | P9, cpd. V-3 |
| Ia-5 | 3-Cl 5-CF₃ | H | H | H | H | H | 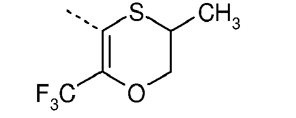 | P9, cpd. V-4 |
| Ia-6 | 3-Cl 5-CF₃ | H | H | H | H | H | 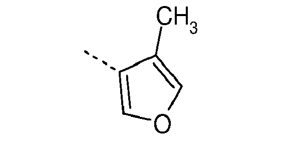 | P9, cpd. B-11 |
| Ia-7 | 3-Cl 5-CF₃ | H | H | H | H | H | 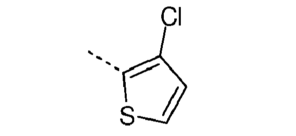 | P9, cpd. C-2 |
| Ia-8 | 3-Cl 5-CF₃ | H | H | H | H | H | 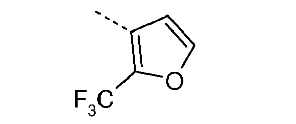 | P9, cpd. B-12 |
| Ia-9 | 3-Cl 5-CF₃ | H | H | H | H | H | 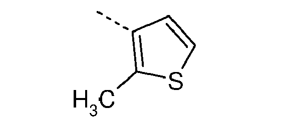 | P9, cpd. D-3 |
| Ia-10 | 3-Cl 5-CF₃ | H | H | H | H | H | 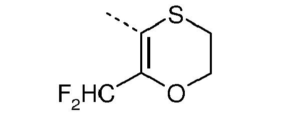 | P9, cpd. V-5 |
| Ia-11 | 3-Cl 5-CF₃ | CN | C(CH₃)₂ | | H | H | 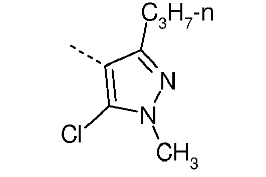 | P12 |
| Ia-12 | 3-Cl 5-CF₃ | CN | C(CH₃)₂ | | H | H | 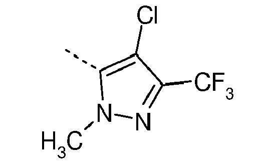 | P12 |
| Ia-13 | 3-Cl 5-CF₃ | H | H | H | H | H | 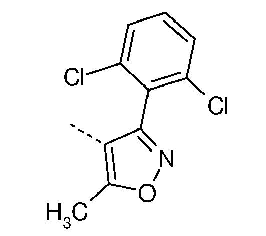 | P9, cpd. M-5 |
| Ia-14 | 3-Cl 5-CF₃ | H | H | H | H | H | 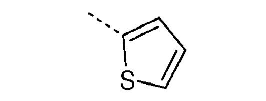 | P9, cpd. C-1 |
| Ia-15 | 3-Cl 5-CF₃ | H | H | H | H | H | 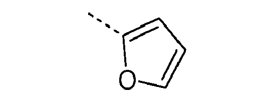 | P9, cpd. A-1 |
| Ia-16 | 3-Me | H | H | CF₃ | H | H | 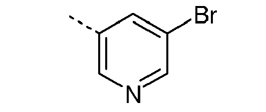 | P11 |
| Ia-17 | 3-Me | H | H | CF₃ | H | H | 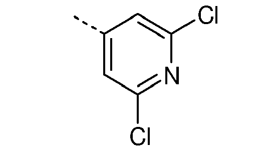 | P11 |
| Ia-18 | 3-Me | H | H | CF₃ | H | H | 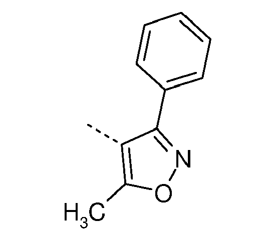 | P11 |
| Ia-19 | 3-Me | H | H | CF₃ | H | H | 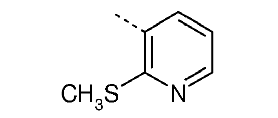 | P11 |
| Ia-20 | 3-Me | H | H | CF₃ | H | H | 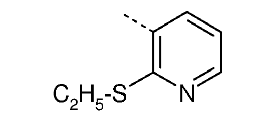 | P11 |
| Ia-21 | 3-Cl 5-CF₃ | H | H | H | H | cyclo-Pr | 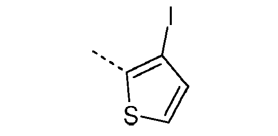 | P9, cpd. C-20 |
| Ia-22 | 3-Br 5-CF₃ | H | H | H | H | H | 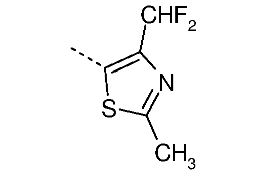 | P9, cpd. H-18 |
| Ia-23 | 3-Cl 5-Cl 6-Me | H | H | H | H | H | 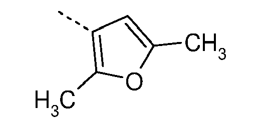 | P9, cpd. B-16 |
| Ia-24 | 3-Cl 5-Cl 6-Me | H | H | H | H | H | 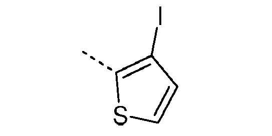 | P9, cpd. C-21 |
| Ia-25 | 3-Cl 5-Cl 6-Me | H | H | H | H | H | 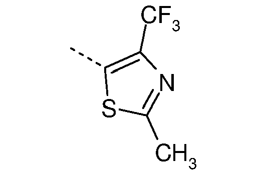 | P9, cpd. H-13 |
| Ia-26 | 3-Cl 5-Cl 6-Me | H | H | H | H | H | 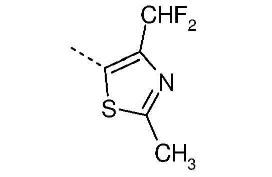 | P9, cpd. H-19 |
| Ia-27 | 3-Cl 5-Br | H | H | H | H | H | 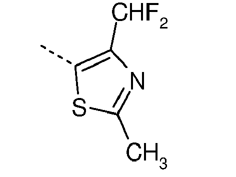 | P9, cpd. H-17 |
| Ia-28 | 5-CF₃ 6-Cl | H | H | H | H | H | 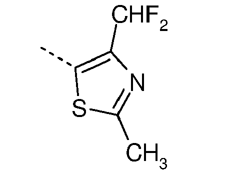 | P9, cpd. H-23 |
| Ia-29 | 3-Cl 5-CF₃ | H | H | H | H | cyclo-Pr | 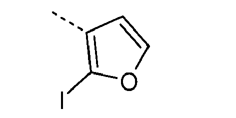 | P9, cpd. B-15 |
| Ia-30 | 3-Cl 5-CF₃ | H | H | H | H | cyclo-Pr | 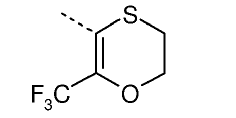 | P9, cpd. V-13 |
| Ia-31 | 3-Cl 5-CF₃ | H | H | H | H | cyclo-Pr | 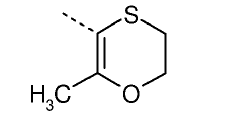 | P9, cpd. V-12 |
| Ia-32 | 3-Cl 5-CF₃ | H | H | H | H | cyclo-Pr | 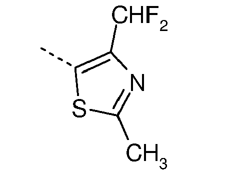 | P9, cpd. H-15 |
| Ia-33 | 3-Cl 5-CF₃ | H | H | H | H | cyclo-Pr | 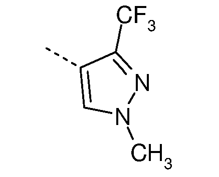 | P9, cpd. J-23 |
| Ia-34 | 3-Cl 5-CF₃ | H | H | H | H | cyclo-Pr | 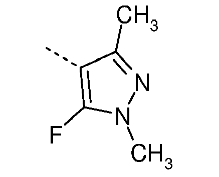 | P9, cpd. J-52 |
| Ia-35 | 3-Cl 5-CF₃ | H | H | H | H | cyclo-Pr | 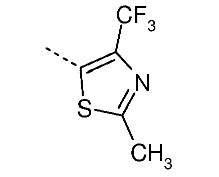 | P9, cpd. H-11 |
| Ia-36 | 3-F 5-F 6-F | H | H | H | H | H | 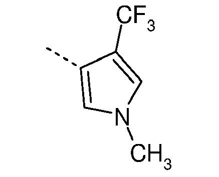 | P9 |
| Ia-37 | 3-F 4-Me 5-F 6-F | H | H | H | H | H | 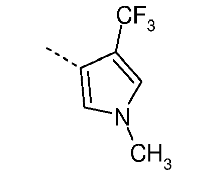 | P9 |
| Ia-38 | 3-F 5-Cl 6-F | H | H | H | H | H | 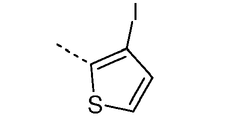 | P9, cpd. C-23 |
| Ia-39 | 3-F 5-Cl 6-F | H | H | H | H | H | 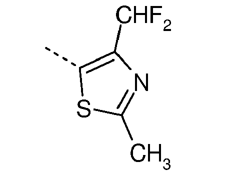 | P9, cpd. H-22 |
| Ia-40 | 3-Cl 5-Cl 6-F | H | H | H | H | H | 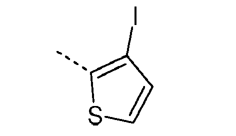 | P9, cpd. C-22 |
| Ia-41 | 3-Cl 5-Cl 6-F | H | H | H | H | H | 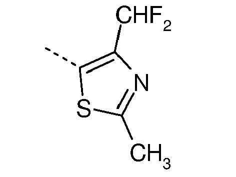 | P9, cpd. H-20 |
| Ia-42 | 3-Cl 5-CF₃ | H | H | H | H | cyclo-Pr | 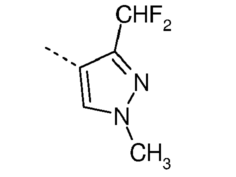 | P9, cpd. J-41 |
| Ia-43 | 3-F 5-F 6-F | H | H | H | H | H | 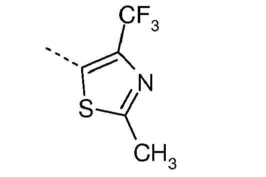 | P9, cpd. H-14 |
| Ia-44 | 3-F 5-F 6-F | H | H | H | H | H | 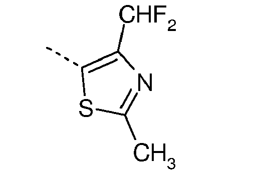 | P9, cpd. H-21 |
| Ia-45 | 3-F 5-F 6-F | H | H | H | H | H | 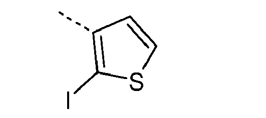 | P9, cpd. D-6 |
| Ia-46 | 3-F 5-F 6-F | H | H | H | H | H | 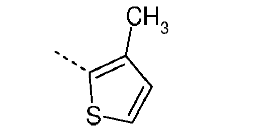 | P9, cpd. C-26 |
| Ia-47 | 3-F 5-F 6-F | H | H | H | H | H | 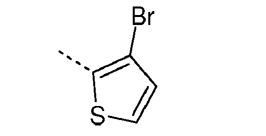 | P9, cpd. C-27 |
| Ia-48 | 3-F 5-F 6-F | H | H | H | H | H | 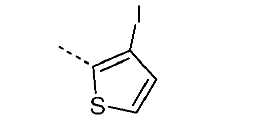 | P9, cpd. C-28 |
| Ia-49 | 3-F 5-F 6-F | H | H | H | H | H | 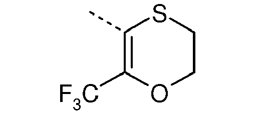 | P9, cpd. V-14 |
| Ia-50 | 3-F 5-F 6-F | H | H | H | H | H | 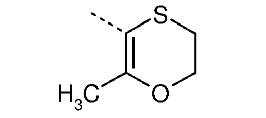 | P9, cpd. V-15 |
| Ia-51 | 3-F 5-F 6-F | H | H | H | H | H | 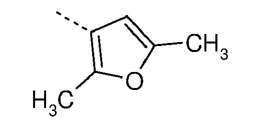 | P9, cpd. B-17 |
| Ia-52 | 3-F 5-F 6-F | H | H | H | H | H | 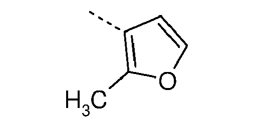 | P9, cpd. B-19 |
| Ia-53 | 3-F 5-F 6-F | H | H | H | H | H | 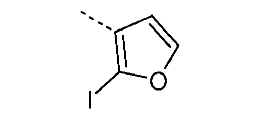 | P9, cpd. B-18 |
| Ia-54 | 3-F 4-Me 5-F 6-F | H | H | H | H | H | 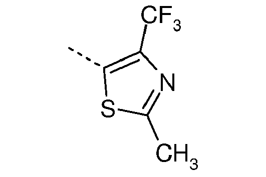 | P9, cpd. H-12 |
| Ia-55 | 3-F 4-Me 5-F 6-F | H | H | H | H | H | 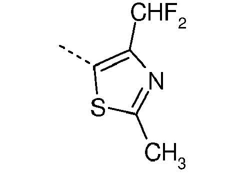 | P9, cpd. H-16 |
| Ia-56 | 3-F 4-Me 5-F 6-F | H | H | H | H | H | 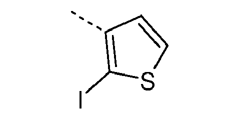 | P9, cpd. D-7 |
| Ia-57 | 3-F 4-Me 5-F 6-F | H | H | H | H | H | 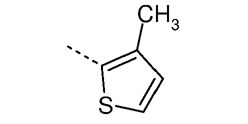 | P9, cpd. C-29 |
| Ia-58 | 3-F 4-Me 5-F 6-F | H | H | H | H | H | 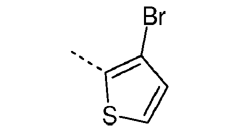 | P9, cpd. C-30 |
| Ia-59 | 3-F 4-Me 5-F 6-F | H | H | H | H | H | 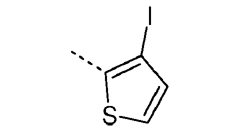 | P9, cpd. C-31 |
| Ia-60 | 3-F 4-Me 5-F 6-F | H | H | H | H | H | 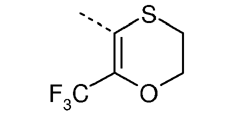 | P9, cpd. V-16 |
| Ia-61 | 3-F 4-Me 5-F 6-F | H | H | H | H | H | 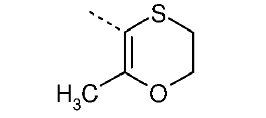 | P9, cpd. V-17 |
| Ia-62 | 3-F 4-Me 5-F 6-F | H | H | H | H | H | 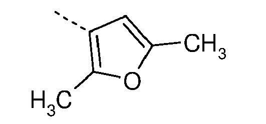 | P9,cpd.B-20 |
| Ia-63 | 3-F 4-Me 5-F 6-F | H | H | H | H | H | 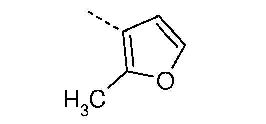 | P9, cpd. B-21 |
| Ia-64 | 3-F 4-Me 5-F 6-F | H | H | H | H | H | 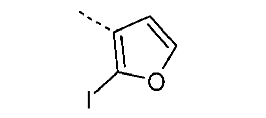 | P9,cpd.B-22 |
| Ia-65 | 5-CF₃ | CH₃ | H | H | H | H | 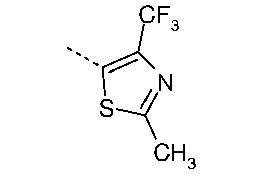 | P10, cpd. 56 |
| Ia-66 | 5-CF₃ | CH₃ | H | H | H | H | 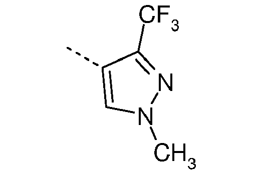 | P10, cpd. 57 |
| Ia-67 | 5-CF₃ | CH₃ | H | H | H | H | 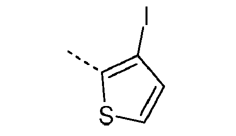 | P10, cpd. 58 |
| Ia-68 | 5-CF₃ 6-Cl | CH₃ | H | H | H | H | 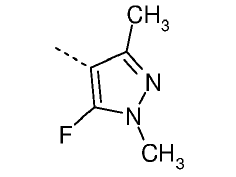 | P10 |
| Ia-69 | 3-Cl 5-Cl | CH₃ | H | H | H | H | 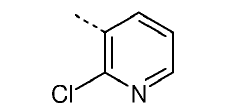 | P11 |
| Ia-70 | 3-Cl 5-CF₃ | NHCO-2-C₆H₄Cl | H | H | H | H | 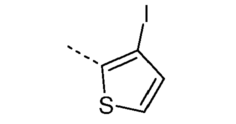 | P10, cpd. 60 |
| Ia-71 | 3-Cl 5-CF₃ | NHCO-2-C₆H₄Cl | H | H | H | H | 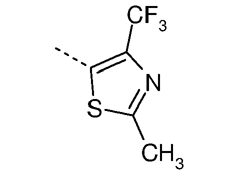 | P10, cpd. 61 |
| Ia-72 | 3-Cl 5-CF₃ | NHCO-2-C₆H₄Cl | H | H | H | H | 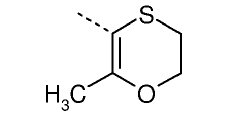 | P10, cpd. 62 |
| Ia-73 | 3-F 5-Cl 6-F | COO-C₄H₉-t | COO-C₄H₉-t | H | H | H | 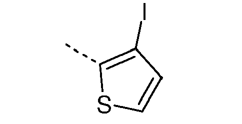 | P11 |
| Ia-74 | 3-Cl 5-CF₃ | H | H | H | H | H | 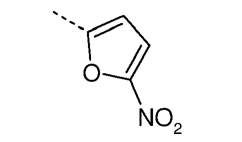 | P9, cpd. A-2 |
| Ia-75 | 3-Cl 5-CF₃ | H | H | H | H | H | 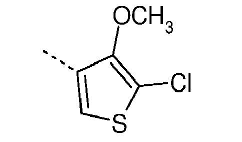 | P9, cpd. D-1 |
| Ia-76 | 3-Cl 5-CF₃ | H | H | H | H | H | 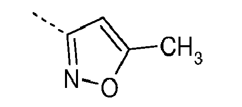 | P9, cpd. L-1 |
| Ia-77 | 3-Cl 5-CF₃ | H | H | H | H | H | 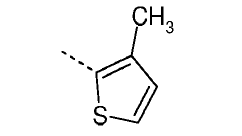 | P9, cpd. C-3 |
| Ia-78 | 3-Cl 5-CF₃ | H | H | H | H | H | 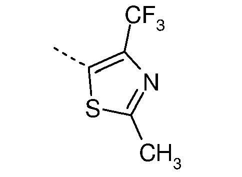 | P9, cpd. H-1 |
| Ia-79 | 3-Cl 5-CF₃ | H | H | H | H | H | 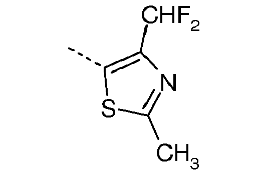 | P9, cpd. H-2 |
| Ia-80 | 3-Cl 5-CF₃ | H | H | H | H | H | 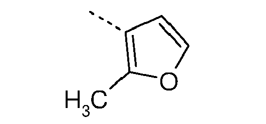 | P9, cpd. B-1 |
| Ia-81 | 3-Cl 5-CF₃ | H | H | H | H | H | 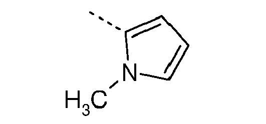 | P9, cpd. F-1 |
| Ia-82 | 3-Cl 5-CF₃ | CH₃ | H | H | H | H | 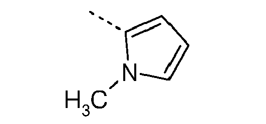 | P10, cpd. 1 |
| Ia-83 | 3-Cl 5-CF₃ | CH₃ | H | H | H | H | 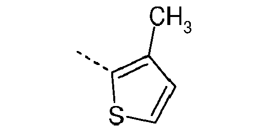 | P10, cpd. 2 |
| Ia-84 | 3-Cl 5-CF₃ | CH₃ | H | H | H | H | 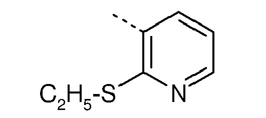 | P10, cpd. 3 |
| Ia-85 | 3-Cl 5-CF₃ | C₂H₅ | H | H | H | H | 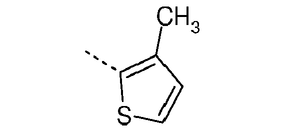 | P10, cpd. 4 |
| Ia-86 | 3-Cl 5-CF₃ | C₂H₅ | H | H | H | H | 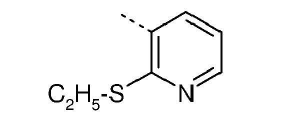 | P10, cpd. 5 |
| Ia-87 | 3-Cl 5-CF₃ | C₂H₅ | H | H | H | H | 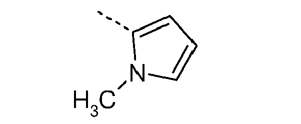 | P10, cpd. 6 |
| Ia-88 | 3-Cl 5-CF₃ | H | H | H | H | H | 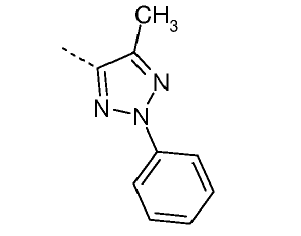 | P9, cpd. O-1 |
| Ia-89 | 3-Cl 5-CF₃ | H | H | H | H | H | 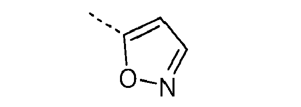 | P9, cpd. N-1 |
| Ia-90 | 3-Cl 5-CF₃ | H | H | H | H | H | 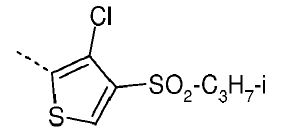 | P9, cpd. C-4 |
| Ia-91 | 3-Cl 5-CF₃ | H | H | H | H | H | 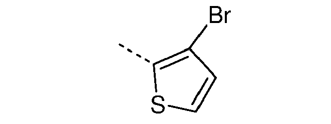 | P9, cpd. C-5 |
| Ia-92 | 3-Cl 5-CF₃ | H | H | H | H | H | 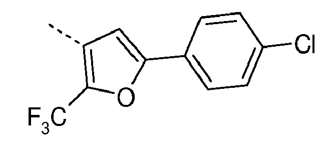 | P9, cpd. B-2 |
| Ia-93 | 3-Cl 5-CF₃ | H | H | H | H | H | 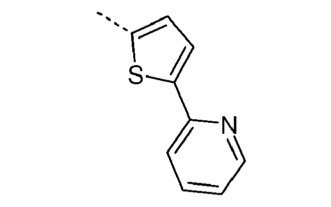 | P9, cpd. C-6 |
| Ia-94 | 3-Cl 5-CF₃ | H | H | H | H | H | 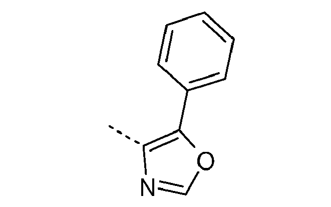 | P9, cpd. G-1 |
| Ia-95 | 3-Cl 5-CF₃ | H | H | H | H | H | 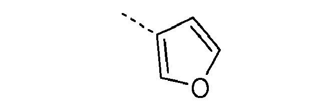 | P9, cpd. B-3 |
| Ia-96 | 3-Cl 5-CF₃ | H | H | H | H | H | 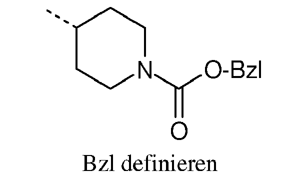 | P9, cpd. T-1 |
| Ia-97 | 3-Cl 5-CF₃ | H | H | H | H | H | 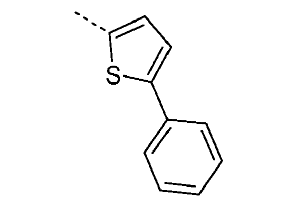 | P9, cpd. C-7 |
| Ia-98 | 3-Cl 5-CF₃ | H | H | H | H | H | 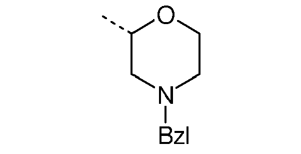 | P9, cpd. U-1 |
| Ia-99 | 3-Cl 5-CF₃ | H | H | H | H | H | 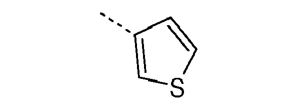 | P9, cpd. D-2 |
| Ia-100 | 3-Cl 5-CF₃ | H | H | H | H | H | 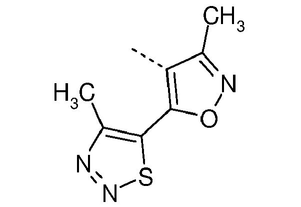 | P9, cpd. M-1 |
| Ia-101 | 3-Cl 5-CF₃ | H | H | H | H | H | 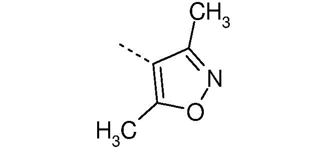 | P9, cpd. M-2 |
| Ia-102 | 3-Cl 5-CF₃ | H | H | H | H | H | 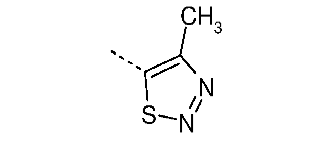 | P9, cpd. P-1 |
| Ia-103 | 3-Cl 5-CF₃ | H | H | H | H | H | 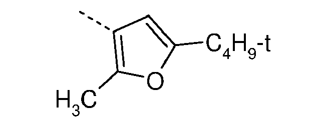 | P9, cpd. B-4 |
| Ia-104 | 3-Cl 5-CF₃ | H | H | H | H | H | 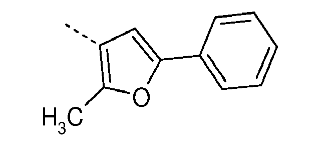 | P9, cpd. B-5 |
| Ia-105 | 3-Cl 5-CF₃ | H | H | H | H | H | 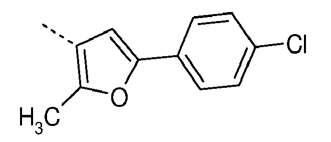 | P9, cpd. B-6 |
| Ia-106 | 3-Cl 5-CF₃ | H | H | H | H | H | 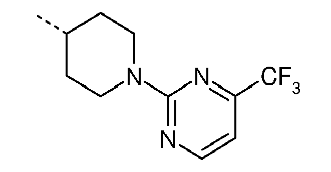 | P9, cpd. T-2 |
| Ia-107 | 3-Cl 5-CF₃ | H | H | H | H | H | 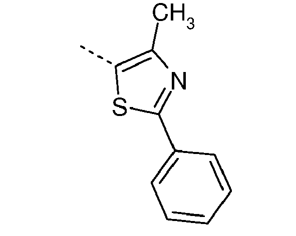 | P9, cpd. H-3 |
| Ia-108 | 3-Cl 5-CF₃ | H | H | H | H | H | 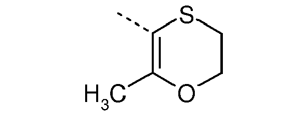 | P9, cpd. V-1 |
| Ia-109 | 3-Cl 5-CF₃ | H | H | H | H | H | 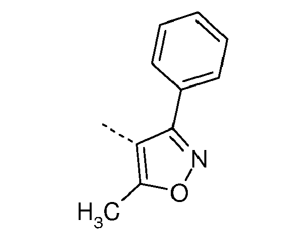 | P9, cpd. M-3 |
| Ia-110 | 3-Cl 5-CF₃ | H | H | H | H | H | 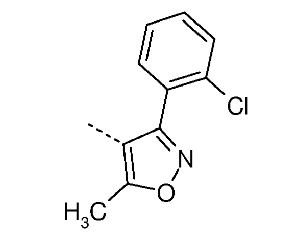 | P9, cpd. M-4 |
| Ia-111 | 3-Cl 5-CF₃ | H | H | H | H | H | 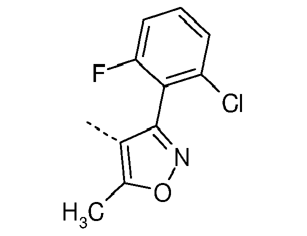 | P9, cpd. M-6 |
| Ia-112 | 3-Cl 5-CF₃ | H | H | H | H | H | 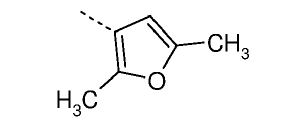 | P9, cpd. B-7 |
| Ia-113 | 3-Cl 5-CF₃ | H | H | H | H | H | 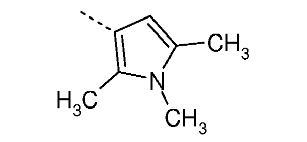 | P9, cpd. E-1 |
| Ia-114 | 3-Cl 5-CF₃ | H | H | H | H | H | 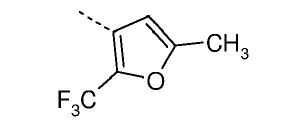 | P9, cpd. B-8 |
| Ia-115 | 3-Cl 5-CF₃ | H | H | H | H | H | 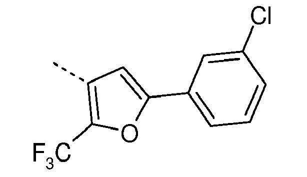 | P9, cpd. B-9 |
| Ia-116 | 3-Cl 5-CF₃ | H | H | H | H | H | 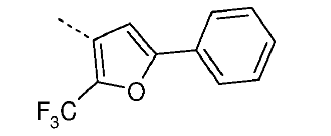 | P9, cpd. B-10 |
| Ia-117 | 3-Cl 5-CF₃ | H | H | H | H | H | 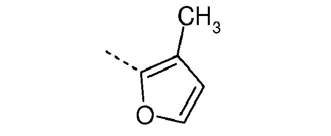 | P9, cpd. A-3 |
| Ia-118 | 3-Cl 5-CF₃ | H | H | H | H | H | 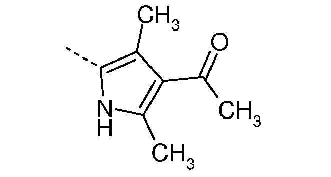 | P9, cpd. F-2 |
| Ia-119 | 3-Cl 5-CF₃ | H | H | H | H | H | 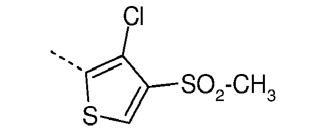 | P9, cpd. C-8 |
| Ia-120 | 3-Cl 5-CF₃ | H | H | H | H | H | 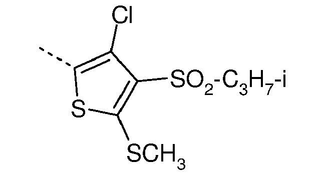 | P9, cpd. C-9 |
| Ia-121 | 3-Cl 5-CF₃ | H | H | H | H | H | 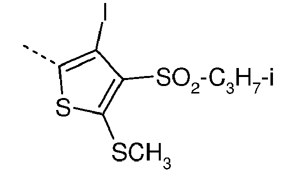 | P9, cpd. C-10 |
| Ia-122 | 3-Cl 5-CF₃ | H | H | H | H | H | 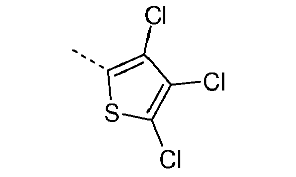 | P9, cpd. C-11 |
| Ia-123 | 3-Cl 5-CF₃ | H | H | H | H | H | 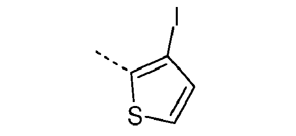 | P9, cpd. C-12 |
| Ia-124 | 3-Cl 5-CF₃ | CH₃ | H | H | H | H | 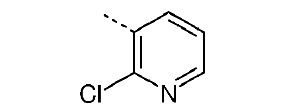 | P10, cpd. 7 |
| Ia-125 | 3-Cl 5-CF₃ | CH₃ | CH₃ | H | H | H | 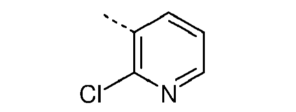 | P10, cpd. 8 |
| Ia-126 | 3-Cl 5-CF₃ | CH₃ | C₂H₅ | H | H | H | 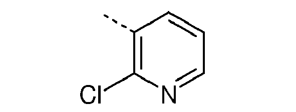 | P10, cpd. 9 |
| Ia-127 | 3-Cl 5-CF₃ | C₃H₇-n | C₃H₇-n | H | H | H | 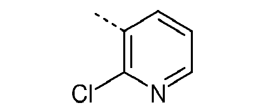 | P10, cpd. 10 |
| Ia-128 | 3-Cl 5-CF₃ | H | H | H | H | H | 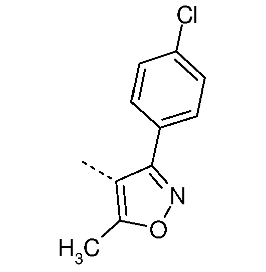 | P9, cpd. M-7 |
| Ia-129 | 3-Cl 5-Cl | H | H | H | H | H | 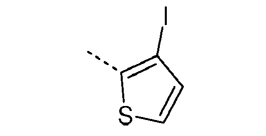 | P9, cpd. C-13 |
| Ia-130 | 3-Cl 5-CF₃ | C₂Hs | H | H | H | H | 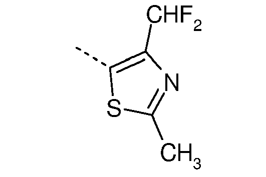 | P10, cpd. 11 |
| Ia-131 | 3-Cl 5-CF₃ | C₂H₅ | H | H | H | H | 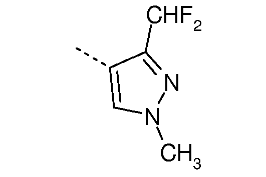 | P10, cpd. 12 |
| Ia-132 | 3-Cl 5-CF₃ | C₂H₅ | H | H | H | H | 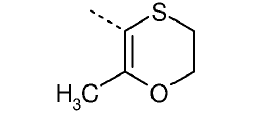 | P10, cpd. 13 |
| Ia-133 | 3-Cl 5-CF₃ | C₂H₅ | H | H | H | H | 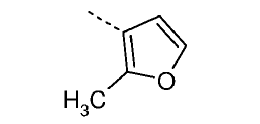 | P10, cpd. 14 |
| Ia-134 | 3-Cl 5-CF₃ | C₂H₅ | H | H | H | H | 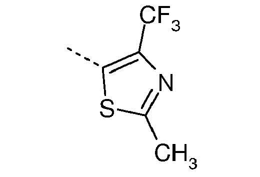 | P10, cpd. 15 |
| Ia-135 | 3-Cl 5-CF₃ | C₂H₅ | H | H | H | H | 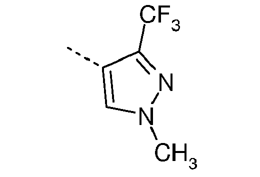 | P10, cpd. 16 |
| Ia-136 | 3-Cl 5-CF₃ | C₃H₇-n | H | H | H | H | 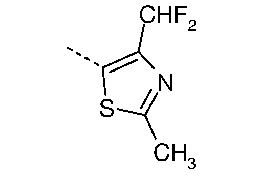 | P10, cpd. 17 |
| Ia-137 | 3-Cl 5-CF₃ | C₃H₇-n | H | H | H | H | 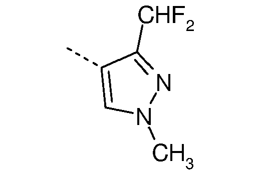 | P10, cpd. 18 |
| Ia-138 | 3-Cl 5-CF₃ | C₃H₇-n | H | H | H | H | 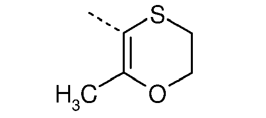 | P10, cpd. 19 |
| Ia-139 | 3-Cl 5-CF₃ | C₃H₇-n | H | H | H | H | 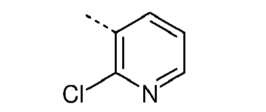 | P10, cpd. 20 |
| Ia-140 | 3-Cl 5-CF₃ | C₃H₇-n | H | H | H | H | 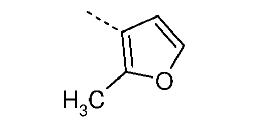 | P10, cpd. 21 |
| Ia-141 | 3-Cl 5-CF₃ | C₃H₇-n | H | H | H | H | 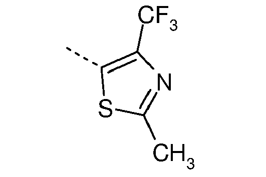 | P10, cpd. 22 |
| Ia-142 | 3-Cl 5-CF₃ | C₃H₇-n | H | H | H | H | 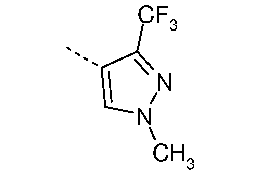 | P10, cpd. 23 |
| Ia-143 | 3-Cl 5-CF₃ | H | (CH₂)₅ | | H | H | 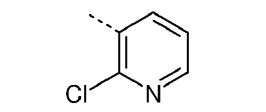 | P12, Table A |
| Ia-144 | 3-Cl 5-Cl | H | H | H | H | H | 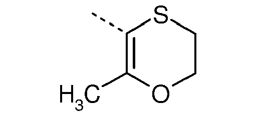 | P9, cpd. V-6 |
| Ia-145 | 3-Cl 5-Cl | H | H | H | H | H | 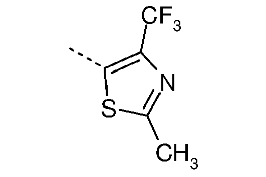 | P9, cpd. H-4 |
| Ia-146 | 3-Cl 5-CF₃ | H | H | H | H | H | 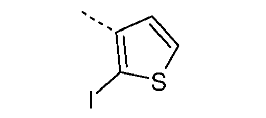 | P9, cpd. D-4 |
| Ia-147 | 3-Cl 5-Cl | H | H | H | H | H | 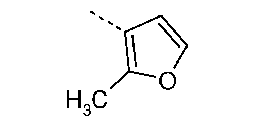 | P9, cpd. B-13 |
| Ia-148 | 3-F 5-Cl | H | H | H | H | H | 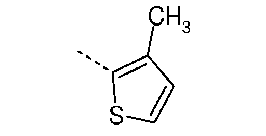 | P9, cpd. C-14 |
| Ia-149 | 3-Cl 5-CF₃ | H | H | H | H | H | 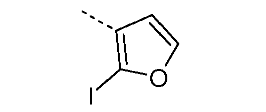 | P9, cpd. B-14 |
| Ia-150 | 3-Cl 5-CF₃ | H | H | H | H | H | 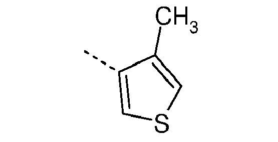 | P9, cpd. D-5 |
| Ia-151 | 3-Cl 5-CF₃ | H | H | H | H | H | 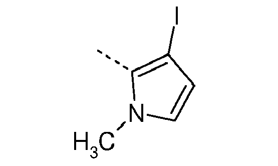 | P9, cpd. F-3 |
| Ia-152 | 3-Cl 5-CF₃ | H | H | H | H | H | 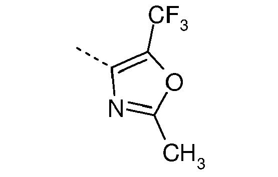 | P9, cpd. G-2 |
| Ia-153 | 3-Cl 5-CF₃ | H | H | H | H | H | 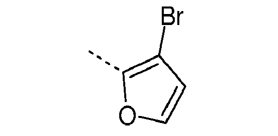 | P9, cpd. A-4 |
| Ia-154 | 3-Cl 5-Cl 6-Cl | H | H | H | H | H | 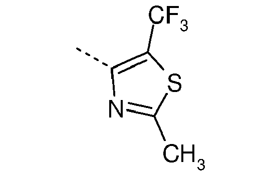 | P9 |
| Ia-155 | 3-Cl 5-Cl 6-Cl | H | H | H | H | H | 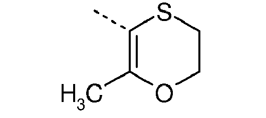 | P9, cpd. V-7 |
| Ia-156 | 3-Cl 5-CF₃ | CH₃ | CH₃ | H | H | H | 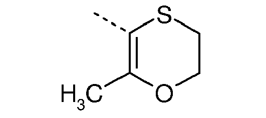 | P10, cpd. 24 |
| Ia-157 | 3-Cl 5-CF₃ | CH₃ | CH₃ | H | H | H | 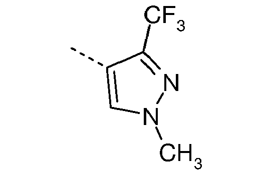 | P10, cpd. 25 |
| Ia-158 | 3-Cl 5-CF₃ | CH₃ | CH₃ | H | H | H | 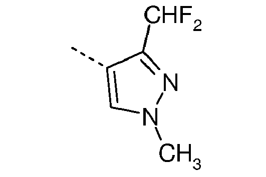 | P10, cpd. 26 |
| Ia-159 | 3-Cl 5-CF₃ | CH₃ | CH₃ | H | H | H | 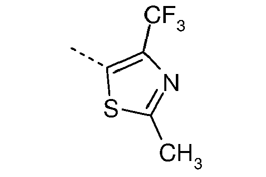 | P10, cpd. 27 |
| Ia-160 | 3-Cl 5-CF₃ | CH₃ | H | H | H | H | 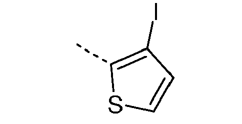 | P10, cpd. 28 |
| Ia-161 | 3-Cl 5-CF₃ | CH₃ | H | H | H | H | 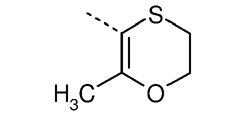 | P10, cpd. 29 |
| Ia-162 | 3-Cl 5-CF₃ | CH₃ | H | H | H | H | 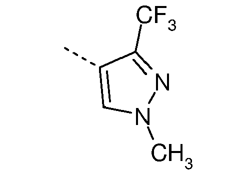 | P10, cpd. 30 |
| Ia-163 | 3-Cl 5-CF₃ | CH₃ | H | H | H | H | 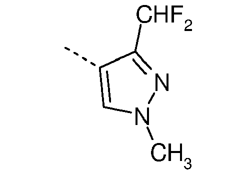 | P10, cpd. 31 |
| Ia-164¹ | 3-Cl 5-CF₃ | H | (CH₂)₄ | | H | H | 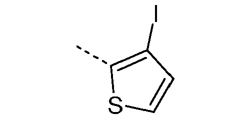 | P12, cpd. B-1, cis-Konfiguration |
| Ia-165 | 3-Cl 5-CF₃ | H | (CH₂)₄ | | H | H | 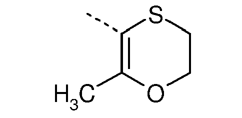 | P12, cpd. C-1 |
| Ia-166 | 3-Cl 5-CF₃ | H | (CH₂)₄ | | H | H | 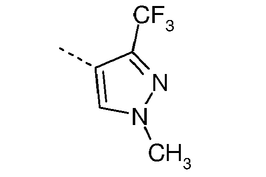 | P12, cpd. E-1 |
| Ia-167 | 3-Cl 5-CF₃ | H | (CH₂)₄ | | H | H | 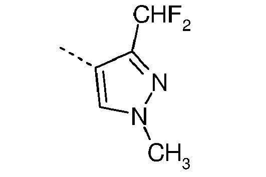 | P12, cpd. F-1 |
| Ia-168 | 3-Cl 5-CF₃ | H | (CH₂)₄ | | H | H | 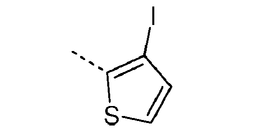 | P12, cpd. B-2 |
| Ia-169² | 3-Cl 5-CF₃ | H | (CH₂)₄ | | H | H | 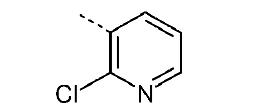 | P12, cpd. A-1, trans-Konfiguration |
| Ia-170¹ | 3-Cl 5-CF₃ | H | (CH₂)₄ | | H | H | 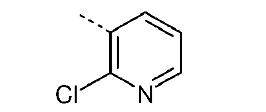 | P12, cpd. A-2, cis-Konfiguration |
| Ia-171 | 3-Cl 5-C H=N OCH₃ | H | H | H | H | H | 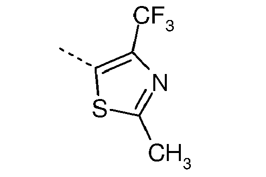 | P9, cpd. H-6 |
| Ia-172 | 3-Cl 5-C H=N OCH₃ | H | H | H | H | H | 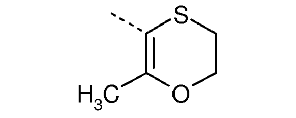 | P9, cpd. V-8 |
| Ia-173 | 3-Cl 5-C H=N OCH₃ | H | H | H | H | H | 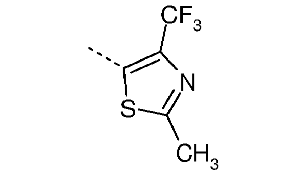 | P9, cpd. H-7 |
| Ia-174 | 3-F 5-Cl | H | H | H | H | H | 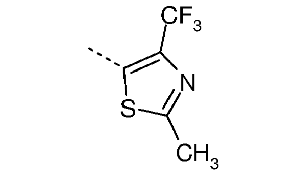 | P9, cpd. H-10 |
| Ia-175 | 3-F 5-Cl | H | H | H | H | H | 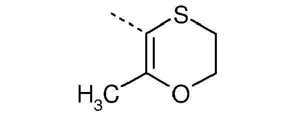 | P9, cpd. V-9 |
| Ia-176 | 3-F 5-Cl | H | H | H | H | H | 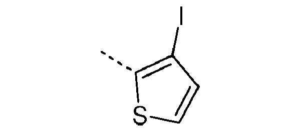 | P9, cpd. C-15 |
| Ia-177 | 3-Cl 5-CF₃ | H | H | C₂H₅ | H | H | 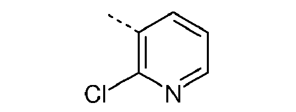 | P10, cpd. 32 |
| Ia-178 | 3-Cl 5-CF₃ | H | H | C₂H₅ | H | H | 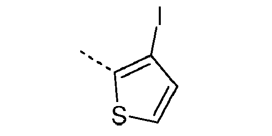 | P10, cpd. 33 |
| Ia-179 | 3-Cl 5-CF₃ | H | H | C₂H₅ | H | H | 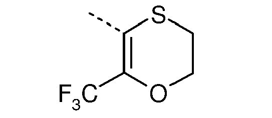 | P10, cpd. 34 |
| Ia-180 | 3-Cl 5-CF₃ | H | H | C₂H₅ | H | H | 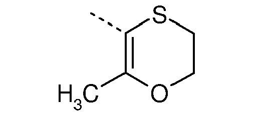 | P10, cpd. 35 |
| Ia-181 | 3-Cl 5-C H=N -OC₃H₇-i | H | H | H | H | H | 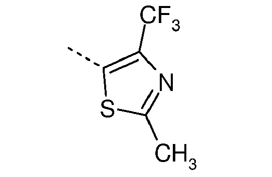 | P9, cpd. H-8 |
| Ia-182 | 3-Br 5-Cl | H | H | H | H | H | 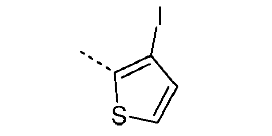 | P9, cpd. C-16 |
| Ia-183 | 5-Cl | H | H | H | H | H | 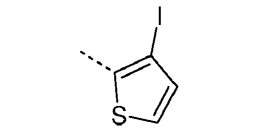 | P9, cpd. C-17 |
| Ia-184 | 3-Cl 6-Cl | H | H | H | H | H | 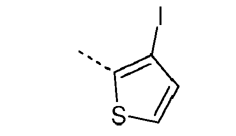 | P9, cpd. C-18 |
| Ia-185 | 3-Cl 6-Cl | H | H | H | H | H | 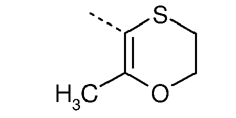 | P9, cpd. V-10 |
| Ia-186 | 3-Cl 6-Cl | H | H | H | H | H | 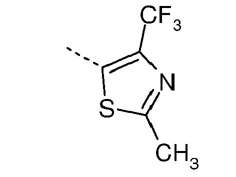 | P9, cpd. H-5 |
| Ia-187 | 5-Me | H | H | H | H | H | 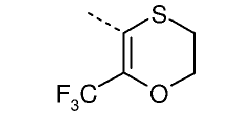 | P9, cpd. V-11 |
| Ia-188 | 5-Me | H | H | H | H | H | 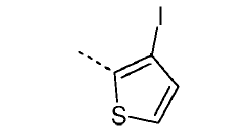 | P9, cpd. C-19 |
| Ia-189 | 3-Cl 5-Cl | CH₃ | H | H | H | H | 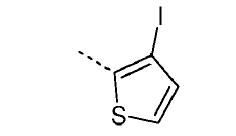 | P11, cpd. A-1 |
| Ia-190 | 3-Cl 5-Cl | CH₃ | H | H | H | H | 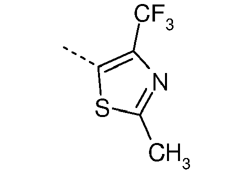 | P11, cpd. B-1 |
| Ia-191 | 3-Cl 5-Cl | CH₃ | H | H | H | H | 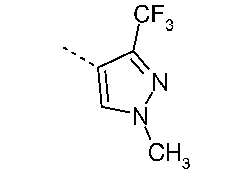 | P11, cpd. C-1 |
| Ia-192 | 3-Cl 5-CF₃ | H | (CH₂)₅ | | H | H | 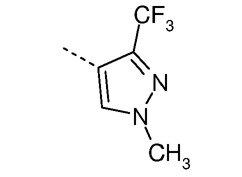 | P12, cpd. E-2 |
| Ia-193 | 3-Cl 5-CF₃ | H | H | C₆H₅ | H | H | 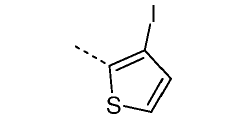 | P10, cpd. 37 |
| Ia-194 | 3-Cl 5-CF₃ | H | H | C₆H₅ | H | H | 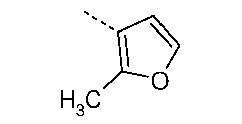 | P10, cpd. 38 |
| Ia-195 | 3-Cl 5-CF₃ | H | H | C₆H₅ | H | H | 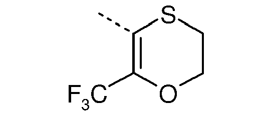 | P10, cpd. 39 |
| Ia-196 | 3-Cl 5-CF₃ | H | H | C₆H₅ | H | H | 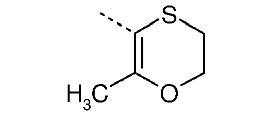 | P10, cpd. 40 |
| Ia-197 | 3-Cl 5-CF₃ | H | H | C₆H₅ | H | H | 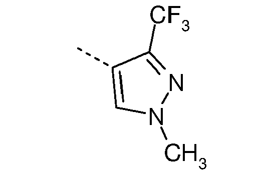 | P10, cpd. 41 |
| Ia-198 | 3-Cl 5-CF₃ | H | H | C₆H₅ | H | H | 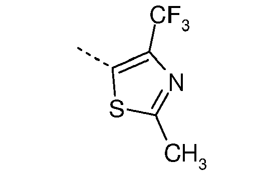 | P10, cpd. 42 |
| Ia-199 | 3-Cl 5-CF₃ | NHCOCH₃ | H | H | H | H | 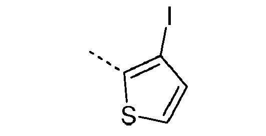 | P10, cpd. 36 |
| Ia-200 | 3-Cl 5-CF₃ | NHCOCH₃ | H | H | H | H | 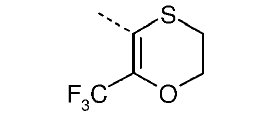 | P10, cpd. 43 |
| Ia-201 | 5-CF ₃ 6-Cl | H | H | H | H | H | 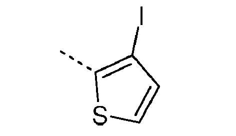 | P9, cpd. C-24 |
| Ia-202 | 5-CF ₃ 6-Cl | H | H | H | H | H | 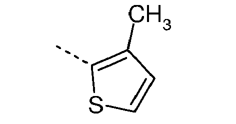 | P9, cpd. C-25 |
| Ia-203 | 3-Cl 5-CF₃ | NHCO-2,6-C₆H₃Cl₂ | H | H | H | H | 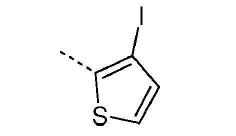 | P10, cpd. 44 |
| Ia-204 | 3-Cl 5-CF₃ | NHCO-2,6-C₆H₃Cl₂ | H | H | H | H | 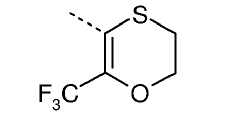 | P10, cpd. 45 |
| Ia-205 | 3-Cl 5-CF₃ | NHCO-2,6-C₆H₃Cl₂ | H | H | H | H | 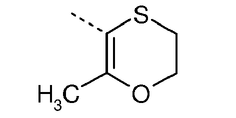 | P10, cpd. 46 |
| Ia-206 | 3-Cl 5-CF₃ | NHCO-2,6-C₆H₃Cl₂ | H | H | H | H | 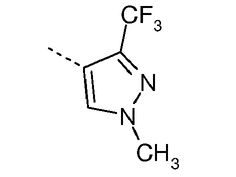 | P10, cpd. 47 |
| Ia-207 | 3-Cl 5-CF₃ | NHCO-2,6-C₆H₃Cl₂ | H | H | H | H | 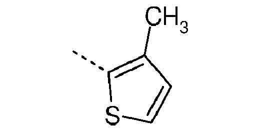 | P10, cpd. 48 |
| Ia-208 | 3-Cl 5-CF₃ | NHCO-2,6-C₆H₃Cl₂ | H | H | H | H | 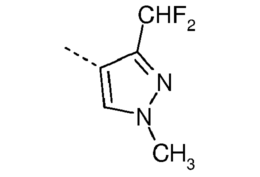 | P10, cpd. 49 |
| Ia-209 | 3-Cl 5-CF₃ | H | H | H | H | H | 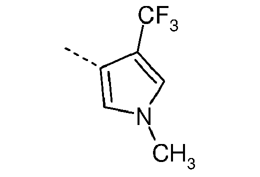 | P9 |
| Ia-210 | 3-Cl 5-CF₃ | H | H | H | H | cyclo-Pr | 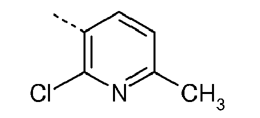 | P9, cpd. R-33 |
| Ia-211 | 5-Cl | CH₃ | H | H | H | H | 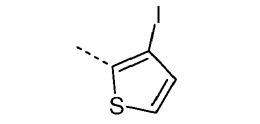 | P11, cpd. A-2 |
| Ia-212 | 5-Cl | CH₃ | H | H | H | H | 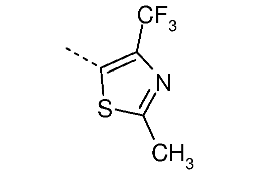 | P11, cpd. B-2 |
| Ia-213 | 3-Cl 5-CF₃ | CH₃ | H | CH₃ | H | H | 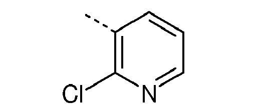 | P10, cpd. 50 |
| Ia-214 | 5-Cl | CH₃ | H | H | H | H | 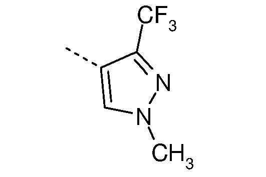 | P11, cpd. C-2 |
| Ia-215 | 3-Cl 5-CF₃ | H | (CH₂)₅ | | H | H | 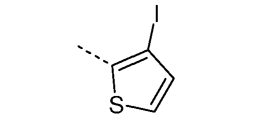 | P12, cpd. B-3 |
| Ia-216 | 3-Cl 5-CF₃ | H | (CH₂)₅ | | H | H | 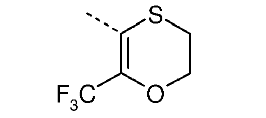 | P12, cpd. D-1 |
| Ia-217 | 3-Cl 5-CF₃ | H | (CH₂)₅ | | H | H | 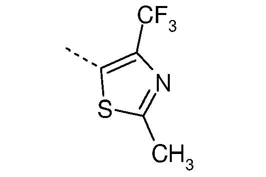 | P12, cpd. G-1 |
| Ia-218 | 5-CF₃ 6-Cl | H | H | H | H | H | 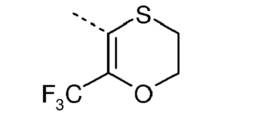 | P9 |
| Ia-219 | 3-Cl 5-CF₃ | CH₃ | H | CH₃ | H | H | 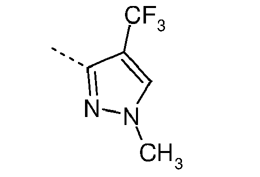 | P10, cpd. 51 |
| Ia-220 | 3-Cl 5-CF₃ | CH₃ | H | CH₃ | H | H | 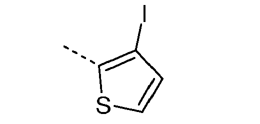 | P10, cpd. 52 |
| Ia-221 | 3-Cl 5-CF₃ | CH₃ | H | CH₃ | H | H | 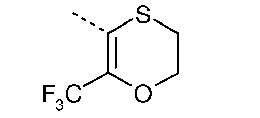 | P10, cpd. 53 |
| Ia-222 | 3-Cl 5-CF₃ | CH₃ | H | CH₃ | H | H | 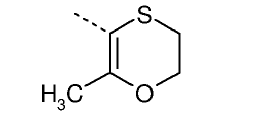 | P10, cpd. 54 |
| Ia-223 | 5-Cl | C₆H₅ | H | H | H | H | 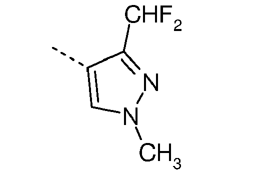 | P11 |
| Ia-224 | 3-F 5-Cl 6-F | COOH | H | H | H | H | 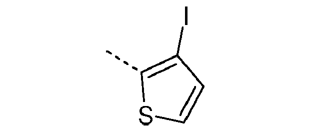 | P11, cpd. A-3 |
| Ia-225 | 5-Cl | (CH₂)₄ | | H | H | H | 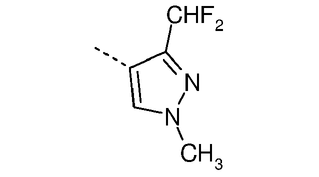 | P11, cpd. C-3 |
| Ia-226 | 3-Cl 5-CF₃ | H | H | H | H | H | 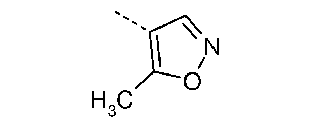 | P9 |
| Ia-227 | 3-Cl 5-CF₃ | H | H | H | H | H | 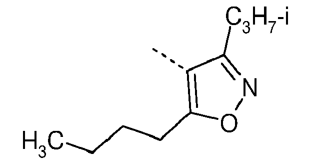 | P9 |
| Ia-228 | 3-Cl 5-CF₃ | H | H | H | H | H | 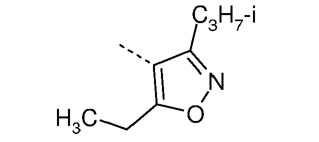 | P9 |
| Ia-229¹ | 3-Cl 5-Cl | H | (CH₂)₄ | | H | H | 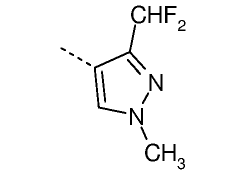 | P13, cpd. A-1, cis-Konfiguration |
| Ia-230 | 3-Cl 5-Cl | H | (CH₂)₄ | | H | H | 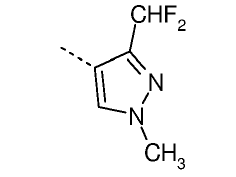 | P13, cpd. A-2 |
| Ia-231 | 3-Cl 5-CF₃ | H | H | CH₃ | H | cyclo-Pr | 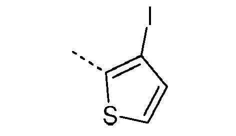 | P10, cpd. 55 |
| Ia-232 | 5-Cl | C₂H₅ | H | H | H | H | 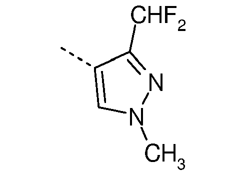 | P11, cpd. C-4 |
| Ia-233 | 3-Cl 5-CF₃ | H | H | CH₃ | H | H | 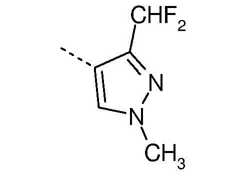 | P10 |
| Ia-234 | 3-Cl 5-CF₃ | H | H | CH₃ | H | H | 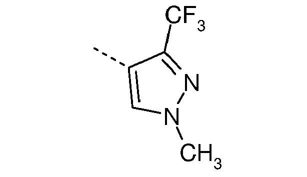 | P10 |
| Ia-235 | 3-Cl 5-CF₃ | C₂H₅ | H | CH₃ | H | H | 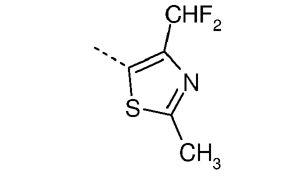 | P10 |
| Ia-236 | 3-Cl 5-CF₃ | C₂H₅ | H | CH₃ | H | H | 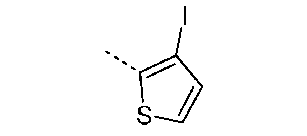 | P10 |
| Ia-237 | 3-Cl 5-CF₃ | C₂H₅ | H | CH₃ | H | H | 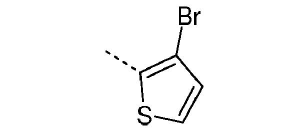 | P10 |
| Ia-238 | 3-Cl 5-CF₃ | H | H | CH₃ | H | H | 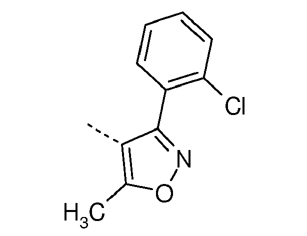 | P10 |
| Ia-239 | 3-Cl 5-CF₃ | C₂H₅ | H | CH₃ | H | H | 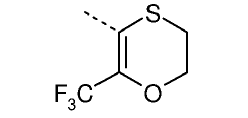 | P10 |
| Ia-240 | 3-Cl 5-CF₃ | CH₂-CH=CH₂ | H | CH₃ | H | H | 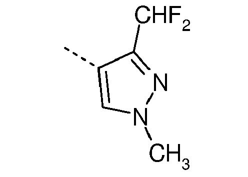 | P10 |
| Ia-241 | 3-Cl 5-CF₃ | C₂H₅ | H | H | H | H | 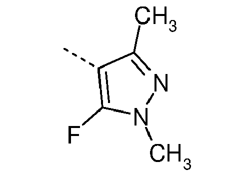 | P10 |
| Ia-242 | 3-Cl 5-CF₃ | C₂H₅ | H | H | H | H | 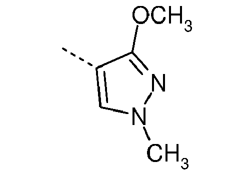 | P10 |
| Ia-243 | 3-Cl 5-CF₃ | H | H | CH₃ | H | H | 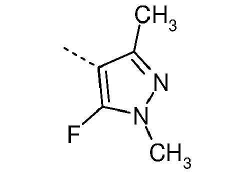 | P10 |
| Ia-244 | 3-Cl 5-CF₃ | H | H | CH₃ | H | H | 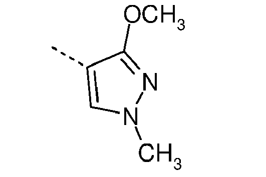 | P10 |
| Ia-245 | 3-Cl 5-C HF₂ | H | H | H | H | H | 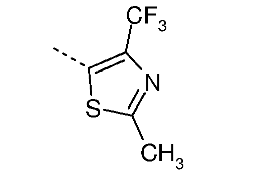 | P9 |
| Ia-246 | 3-Cl 5-Cl | F | F | H | H | H | 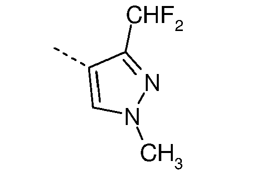 | P11 |
| Ia-247 | 3-Cl 5-Cl | F | F | H | H | H | 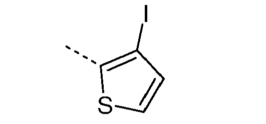 | P11 |
| Ia-248 | 3-Cl 5-CF₃ | F | F | H | H | H | 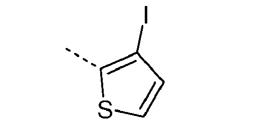 | P10 |
| Ia-249 | 3-Cl 5-CF₃ | F | F | H | H | H | 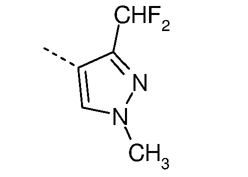 | P10 |
| Ia-250 | 3-Cl 5-Cl | H | CH₂ | | H | H | 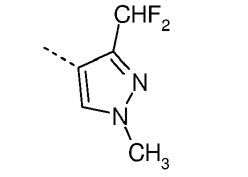 | P13, cpd. A-3 |
| Ia-251 | 3-Cl 5-CF₃ | F | F | H | H | H | 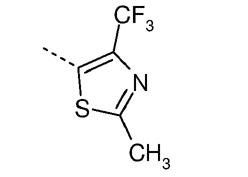 | P10 |
| Ia-25₂₂ | 3-Cl 5-Cl | H | CH₂ | | H | H | 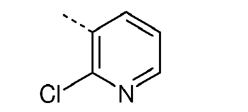 | P13, cpd. B-1, trans-Konfiguration |
| Ia-253² | 3-Cl 5-Cl | H | CH₂ | | H | H | 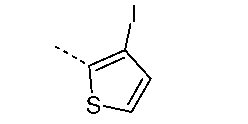 | P13, cpd. C-1, trans-Konfiguration |
| Ia-254 | 3-Cl 5-CF₃ | H | H | H | H | H | 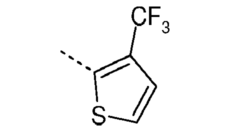 | P9 |
| Ia-255 | 3-Cl 5-CF₃ | H | H | H | H | H | 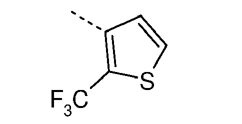 | P9 |
| Ia-256 | 3-Cl 5-CF₃ | CH₂-C≡CH | H | CH₃ | H | H | 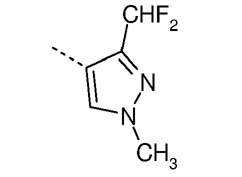 | P10 |
| Ia-257 | 3-Cl 5-CF₃ | H | H | CH₃ | H | H | 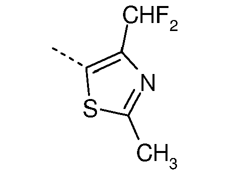 | P10 |
| Ia-258 | 3-Cl 5-CF₃ | H | H | CH₃ | H | H | 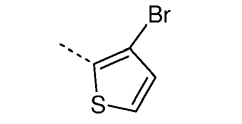 | P10 |
| Ia-259 | 3-Cl 5-CF₃ | H | H | CH₃ | H | H | 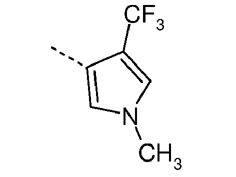 | P10 |
| Ia-260 | 3-Cl 5-CF₃ | H | H | CH₃ | H | H | 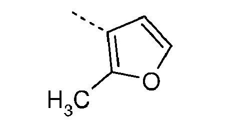 | P10 |
| Ia-261 | 3-Cl 5-CF₃ | H | H | CH₃ | H | H | 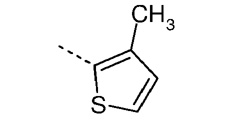 | P10 |
| Ia-262 | 3-Cl 5-CF₃ | CH₂-C≡CH | H | CH₃ | H | H | 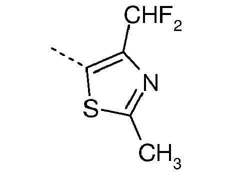 | P10 |
| Ia-263 | 3-Cl 5-CF₃ | CH₂-C≡CH | H | CH₃ | H | H | 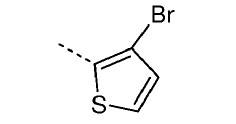 | P10 |
| Ia-264 | 3-Cl 5-CF₃ | CH₂-C≡CH | H | CH₃ | H | H | 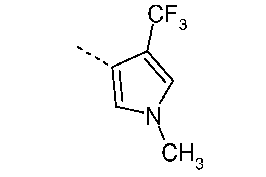 | P10 |
| Ia-265 | 3-Cl 5-CF₃ | CH₂-C≡CH | H | CH₃ | H | H | 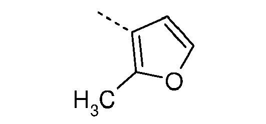 | P10 |
| Ia-266 | 3-Cl 5-CF₃ | CH₂-C≡CH | H | CH₃ | H | H | 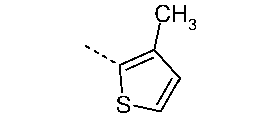 | P10 |
| Ia-267 | 3-Cl 5-CF₃ | CH₂-C≡CH | H | CH₃ | H | H | 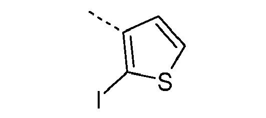 | P10 |
| Ia-268 | 3-Cl 5-CF₃ | H | H | H | H | H | 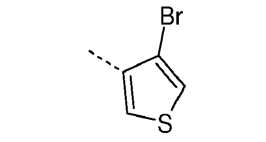 | P9 |
| Ia-269 | 3-Cl 5-CF₃ | H | H | H | H | H | 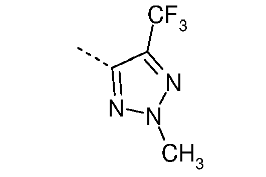 | P9 |
| Ia-270 | 3-Cl 5-CF₃ | H | H | CH₃ | H | H | 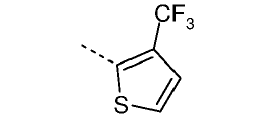 | P10 |
| Ia-271 | 3-Cl 5-CF₃ | H | H | CH₃ | H | H | 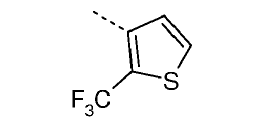 | P10 |
| Ia-272 | 3-Cl 5-Cl | H | CH₂ | | H | H | 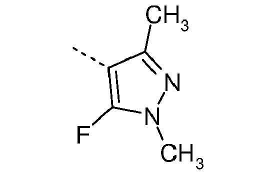 | P13, cpd. A-4 |
| Ia-273 | 3-Cl 5-CF₃ | H | H | CH₃ | H | H | 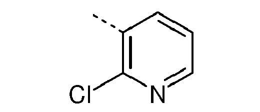 | P10 |
| Ia-274 | 3-Cl 5-CF₃ | H | H | CH₃ | H | H | 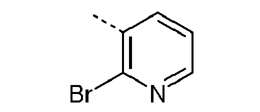 | P10 |
| Ia-275 | 3-Cl 5-CF₃ | H | H | CH₃ | H | H | 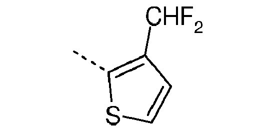 | P10 |
| Ia-276 | 3-Cl 5-CF₃ | H | H | CH₃ | H | H | 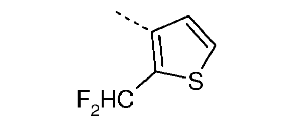 | P10 |
| Ia-277 | 3-Cl 5-CF₃ | H | H | CH₃ | H | H | 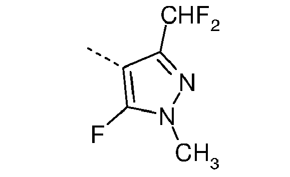 | P10 |
| Ia-278 | 3-Cl 5-CF₃ | C₂H₅ | H | H | H | H | 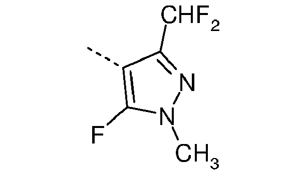 | P10 |
| Ia-279 | 3-Me | H | H | CF₃ | H | H | 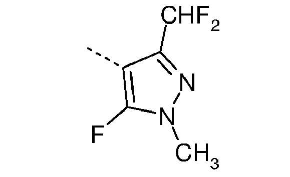 | P11 |
| Ia-280 | 3-Cl 5-CF₃ | C₂H₅ | H | H | H | H | 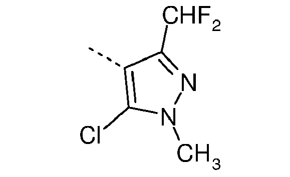 | P10 |
| Ia-281 | 3-Me | H | H | CF₃ | H | H | 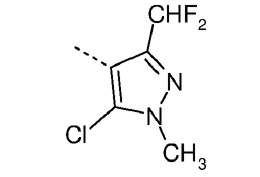 | P11 |
| Ia-282 | 3-Cl 5-CF₃ | H | H | H | H | H | 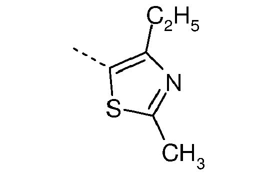 | P9 |
| Ia-283 | 3-Cl 5-CF₃ | H | H | CH₃ | H | H | 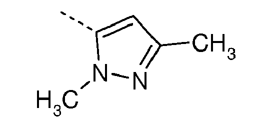 | P10 |
| Ia-284 | 3-Cl 5-CF₃ | H | H | CH₃ | H | H | 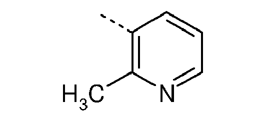 | P10 |
| Ia-285 | 3-Cl 5-CF₃ | H | H | CH₃ | H | cyclo-Pr | 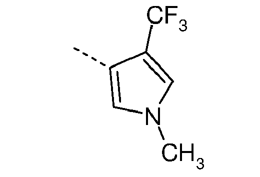 | EP 2218717 CAS-No. 1321534-55-7 |
| Ia-286 | 3-Cl 5-CF₃ | H | H | CH₃ | H | cyclo-Pr | 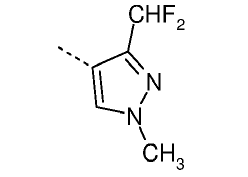 | EP 2218717 CAS-No. 1321534-91-1 |
| Ia-287 | 3-Cl 5-CF₃ | H | H | CH₃ | H | cyclo-Pr | 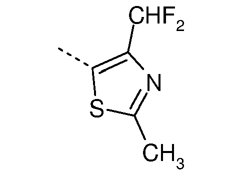 | EP 2218717 CAS-No. 1321534-58-0 |
| Ia-288 | 3-Cl 5-CF₃ | H | H | CH₃ | H | cyclo-Pr | 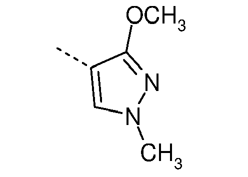 | EP 2218717 CAS-No. 1321534-66-0 |
| Ia-289 | 3-Cl 5-CF₃ | H | H | CH₃ | H | H | 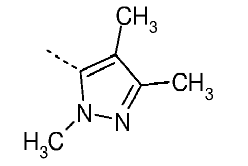 | P10 |
| Ia-290 | 3-Cl 5-CF₃ | C₂H₅ | H | H | H | H | 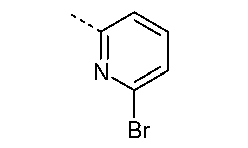 | P10 |
| Ia-291 | 3-Me | H | H | CF₃ | H | H | 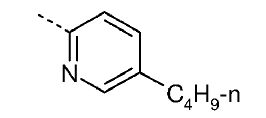 | P10 |
| Ia-292 | 3-Cl 5-CF₃ | H | H | CH₃ | H | H | 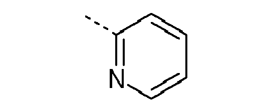 | P10 |
| Ia-293 | 3-Cl 5-CF₃ | CH₂-cyclo-Pr | H | CH₃ | H | H | 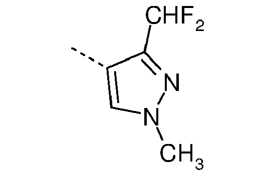 | |
| Ia-294 | 3-Cl 5-CF₃ | CH₂-cyclo-Pr | H | CH₃ | H | H | 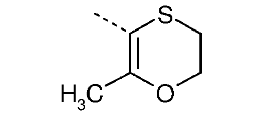 | |
| Ia-295 | 3-Cl 5-CF₃ | CH₂-cyclo-Pr | H | CH₃ | H | H | 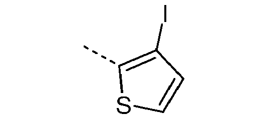 | |
| Ia-296 | 3-Cl 5-CF₃ | CH₂-cyclo-Pr | H | CH₃ | H | H | 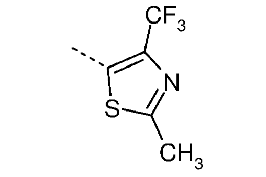 | |
| Ia-297 | 3-Cl 5-CF₃ | CH₂-cyclo-Pr | H | CH₃ | H | H | 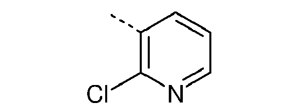 | |
| Ia-298 | 3-Cl 5-CF₃ | CH₂-cyclo-Pr | H | CH₃ | H | H | 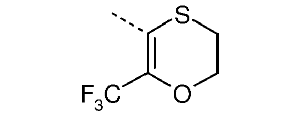 | |
| Ia-299 | 3-Cl 5-Cl | H | H | H | H | H | 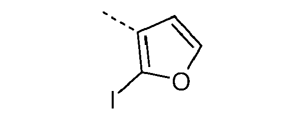 | |
| Ia-300 | 3-Cl 5-CF₃ | H | H | CH₃ | H | H | 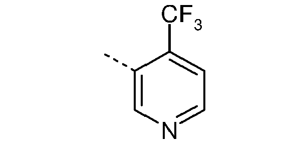 | |
| Ia-301 | 3-Cl 5-CF₃ | H | H | CH₃ | H | H | 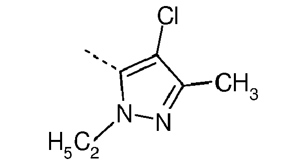 | |
| Ia-302 | 3-Cl 5-CF₃ | H | H | CH₃ | H | H | 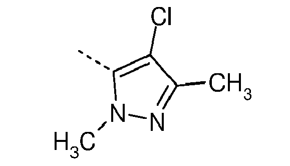 | |
| Ia-303 | 3-Cl 5-CF₃ | CH₃ | H | H | H | H | 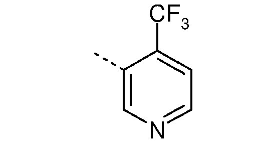 | |
| Ia-304 | 3-Cl 5-CF₃ | CH₃ | H | H | H | H | 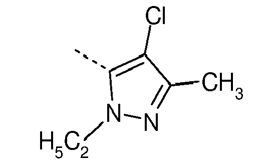 | |
| Ia-305 | 3-Cl 5-CF₃ | CH₃ | H | H | H | H | 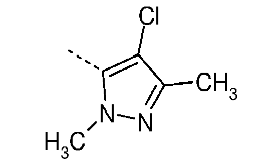 | |
| Ia-306 | 3-Cl 5-CF₃ | CH₃ | H | H | H | H | 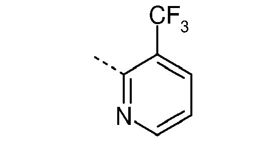 | |
| Ia-307 | 3-Cl 5-CF₃ | CH₃ | H | H | H | H | 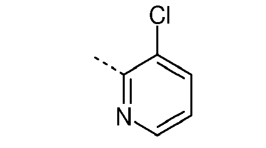 | |
| Ia-308 | 3-Cl 5-CF₃ | CH₃ | H | H | H | H | 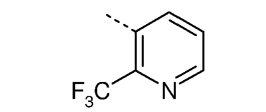 | |

| | | | | | | | | |
|---|---|---|---|---|---|---|---|---|
| ¹cis-Isomer ²trans-Isomer **1H-NMR data** ¹H-NMR-data were determined with a Bruker Avance 400 equipped with a flow cell (60 µl volume) or with a Bruker AVIII 400 equipped with 1.7 mm cryo-CPTCI probe head or with a Bruker AVII 600 (600.13 MHz) equipped with a cyroTCI probe head or with a Bruker AVIII 600 (601.6 MHz) equipped with a cryo CPMNP probe head with tetramethylsilane as reference (0.0) and the solvents CD₃CN, CDCl₃, [D₆]-DMSO. NMR-data of selected examples are listed in classic format (chemical shift δ, multiplicity, number of hydrogen atoms) or as NMR-peak-lists. NMR-peak-lists: If NMR-data of selected examples are provided in form of ¹H-NMR-peak lists, then for every peak first the chemical shift δ in ppm and then, separated by a blank, the intensity oft the signal in round brackets is listed. The peak list of an example is therefore listed as: δ₁ (intensity₁); δ₂ (intensity₂); ... ; δᵢ (intensity;);...; δₙ (intensityₙ). The solvent, in which the NMR-spectrum was measured is specified in squared brackets. | | | | | | | | |

**Table B**

| | | | | | | | | |
|---|---|---|---|---|---|---|---|---|
| Compounds of formula (Ib) | | | | | | | | |
| 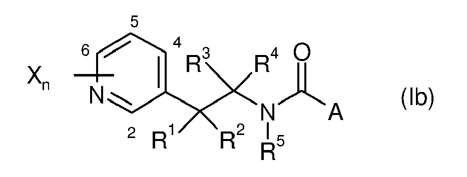 | | | | | | | | |

| **No.** | **Xₙ** | **R¹** | **R²** | **R³** | **R⁴** | **R⁵** | **A** | **cf.** |
|---|---|---|---|---|---|---|---|---|
| Ib-1 | 6-CF₃ | H | H | H | H | H | 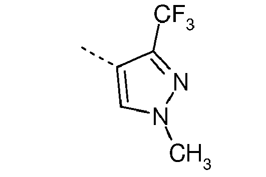 | P14, cpd. A-1 |
| Ib-2 | 6-OMe | H | H | H | H | H | 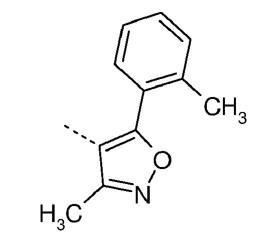 | P14 |
| Ib-3 | 6-OMe | H | H | H | H | H | 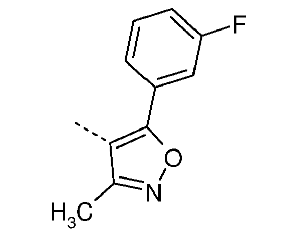 | P14 |
| Ib-4 | 6-OMe | H | H | H | H | H | 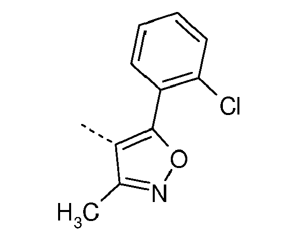 | P14 |
| Ib-5 | 6-OMe | H | H | H | H | H | 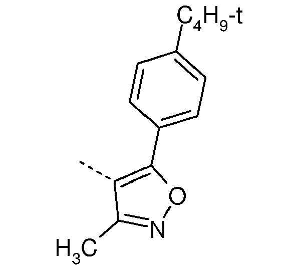 | P14 |
| Ib-6 | 6-Cl | H | H | H | H | H | 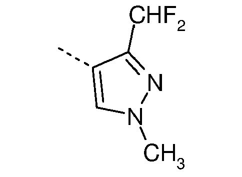 | P14, cpd. A-2 |
| Ib-7 | 6-Cl | H | H | H | H | H | 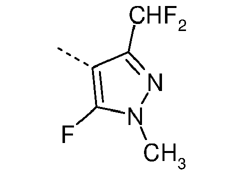 | P14 |
| Ib-8 | 6-Cl | H | H | H | H | H | 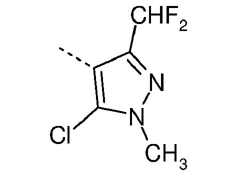 | P14 |

**Table C**

| | | | | | | | | |
|---|---|---|---|---|---|---|---|---|
| Compounds of formula (Ic) | | | | | | | | |
| 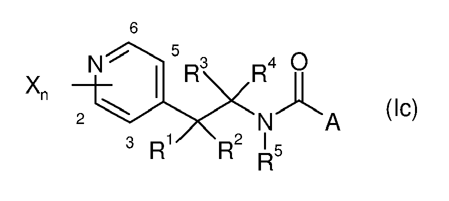 | | | | | | | | |

| **No.** | **Xₙ** | **R¹** | **R²** | **R³** | **R⁴** | **R⁵** | **A** | **cf.** |
|---|---|---|---|---|---|---|---|---|
| Ic-1 | 3-Cl | H | H | H | H | H | 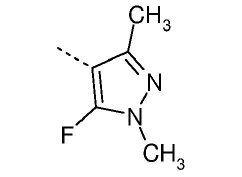 | P15 |
| Ic-2 | 2-F 3-F 5-F 6-F | H | H | H | H | H | 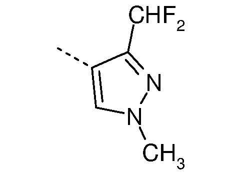 | P15, cpd. A-1 |
| Ic-3 | 2-F 3-F 5-F 6-F | H | H | H | H | H | 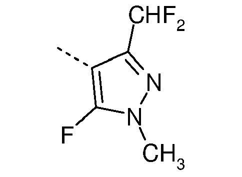 | P15 |
| Ic-4 | 2-F 3-F 5-F 6-F | H | H | H | H | H | 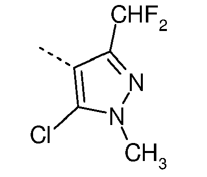 | P15 |

| | | | | | | | | |
|---|---|---|---|---|---|---|---|---|
| The following tables D to F illustrate in a non limiting manner intermediates of formula (II) which can be used for the preparation of compounds of formula (I). | | | | | | | | |

**Table D**

| | | | | | | | | |
|---|---|---|---|---|---|---|---|---|
| Compounds of formula (IIa) | | | | | | | | |
| 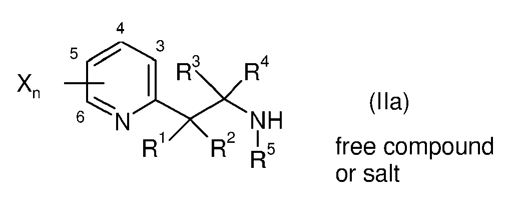 | | | | | | | | |

| **No.** | **Xₙ** | **R¹** | **R²** | **R³** | **R⁴** | **R⁵** | **salt** | **Reference or phys.chem. data** |
|---|---|---|---|---|---|---|---|---|
| IIa-1 | 3-Cl 5-CF₃ | C₂H₅ | H | H | H | H | HCl-salt | P2 |
| IIa-2 | 3-Cl 5-CF₃ | CH₃ | H | H | H | H | | |
| IIa-3 | 5-CF₃ | CH₃ | H | H | H | H | HCl-salt | NMR |
| IIa-4 | 5-CF₃ 6-Cl | CH₃ | H | H | H | H | | |
| IIa-5 | 5-Cl | CH₃ | H | H | H | H | | P11 |
| IIa-6 | 5-Cl | CH₃ | H | H | H | H | | |
| IIa-7 | 3-Cl 5-CF₃ | n-Pr | H | H | H | H | | |
| IIa-8 | 5-Cl | C₆H₅ | H | H | H | H | | |
| IIa-9 | 3-Cl 5-CF₃ | NHC(O)-CH₃ | H | H | H | H | | P2 |
| IIa-10 | 3-Cl 5-CF₃ | NHC(O)-2-C₆H₄Cl | H | H | H | H | | |
| IIa-11 | 3-Cl 5-CF₃ | NHC(O)-2,6-C₆H₃Cl₂ | H | H | H | H | | |
| IIa-12 | 3-Cl 5-CF₃ | OH | H | COOC₂H₅ | H | H | | |
| IIa-13 | 3-Cl 5-CF₃ | OH | H | H | H | H | | P2 |
| IIa-14 | 3-Cl 5-CF₃ | F | F | H | H | H | | |
| IIa-15 | 3-Cl 5-Cl | F | F | H | H | H | | |
| IIa-16 | 3-Cl 5-CF₃ | CH₃ | H | CH₃ | H | H | | |
| IIa-17 | 3-Cl 5-CF₃ | CH₂-cyclo-Pr | H | CH₃ | H | H | | |
| IIa-18 | 3-Cl 5-CF₃ | CH₂-CH=CH₂ | H | CH₃ | H | H | | |
| IIa-19 | 3-Cl 5-CF₃ | CH₂-C≡CH | H | CH₃ | H | H | | |
| IIa-20 | 3-Cl 5-CF₃ | H | H | C₂H₅ | H | H | | P2 |
| IIa-21 | 3-Cl 5-CF₃ | H | H | CH₃ | H | H | | |
| IIa-22 | 5-Cl | H | H | CH₃ | H | H | | |
| IIa-23 | 6-Cl 5-CF₃ | H | H | CH₃ | H | H | | |
| IIa-24 | 3-Cl 5-CF₃ | H | H | CH₃ | H | cyclo-Pr | HCl salt | P2 P10 NMR |
| IIa-25 | 3-Cl 5-CF₃ | H | H | C₆H₅ | H | H | | P2 |
| IIa-26 | 3-Cl 5-CF₃ | H | H | COOH | H | H | | comm. av. CAS-No. 317377-64-3 NMR |
| IIa-27 | 3-Cl | H | H | COOMe | H | H | | P1 |
| | 5-CF₃ | | | | | | | comm. av. CAS-No. 1008232-30-1 |
| IIa-28 | 3-Cl 5-CF₃ | H | H | COOEt | H | H | | according to P1 |
| IIa-29 | 3-Cl 5-CF₃ | H | H | CONHMe | H | H | | according to P1 |
| IIa-30 | 3-Cl 5-CF₃ | H | H | CONHEt | H | H | | according to P1 NMR |
| IIa-31 | 5-CF₃ | CH₂-CH₂ | | H | H | H | | |
| IIa-32 | 5-Cl | CH₂-CH₂ | | H | H | H | - | |
| IIa-33 | 5-Cl | CH₂-CH₂ | | H | H | H | - | |
| IIa-34 | 5-Cl | (CH₂)₄ | | H | H | H | - | |
| IIa-35 | 3-Cl 5-Cl | H | CH₂ | | H | COO-C₄H₉-t | - | P5 |
| IIa-36 | 5-Cl | H | (CH₂)₄ | | H | H | - | P5 |
| IIa-37 | 3-Cl 5-CF₃ | H | (CH₂)₄ | | H | H | - | P4 |
| IIa-38 | 3-Cl 5-CF₃ | H | (CH₂)₅ | | H | H | - | P4 |
| IIa-39 | 3-Cl 5-CF₃ | H | H | H | H | cyclo-Pr | | P9 |
| | | | | | | | | |
| **No. IIa-3, Solvent: <[D₆]-DMSO >, Spektrometer: 399.95MHz** 8.9425 (4.53); 8.5297 (0.55); 8.3132 (0.37); 8.3081 (0.36); 8.2919 (0.35); 8.2871 (0.34); 8.2274 (2.43); 8.2220 (2.47); 8.2066 (2.63); 8.2014 (2.57); 7.9233 (3.03); 7.9035 (1.80); 7.8274 (0.51); 7.6396 (4.25); 7.6190 (3.98); 7.3355 (0.94); 7.2080 (1.11); 7.0806 (0.95); 3.9056 (6.21); 3.5088 (0.47); 3.3780 (460.23); 3.3398 (2.99); 3.3213 (1.08); 3.3038 (0.56); 3.2802 (0.50); 3.2660 (0.94); 3.2515 (1.14); 3.2344 (1.49); 3.2202 (1.32); 3.2154 (1.20); 3.2005 (0.91); 3.1668 (2.48); 3.1300 (0.34); 3.1160 (1.18); 3.1012 (1.89); 3.0859 (1.76); 3.0701 (1.36); 3.0550 (0.71); 2.6777 (1.01); 2.6732 (1.39); 2.6687 (1.05); 2.6173 (0.39); 2.5383 (15.01); 2.5244 (34.14); 2.5127 (88.01); 2.5087 (171.64); 2.5041 (220.28); 2.4996 (165.79); 2.4953 (85.87); 2.3354 (1.00); 2.3308 (1.41); 2.3264 (1.01); 2.0215 (0.61); 1.4956 (2.91); 1.2936 (16.00); 1.2762 (15.82); -0.0002 (1.03) | | | | | | | | |
| **No. IIa-24, Solvent: <[D₆]-DMSO >, Spektrometer: 399.95MHz** 9.1652 (0.94); 9.0728 (1.01); 8.9503 (6.07); 8.9477 (5.98); 8.5288 (6.13); 8.5250 (6.03); 3.9453 (1.35); 3.9054 (5.68); 3.5449 (1.73); 3.5325 (1.70); 3.5062 (2.40); 3.4935 (2.26); 3.3867 (0.60); 3.3436 (264.78); 3.3065 (3.05); 3.2849 (2.60); 3.2674 (2.09); 3.2459 (1.91); 3.1667 (0.97); 2.8120 (1.66); 2.6810 (0.39); 2.6769 (0.81); 2.6723 (1.13); 2.6678 (0.85); 2.5426 (0.72); 2.5257 (3.49); 2.5121 (66.26); 2.5078 (134.10); 2.5032 (177.17); 2.4987 (132.59); 2.4943 (67.64); 2.3390 (0.41); 2.3344 (0.84); 2.3300 (1.15); 2.3255 (0.86); 1.3351 (0.36); 1.3158 (15.96); 1.2994 (16.00); 1.2490 (0.33); 1.2342 (0.32); 0.9477 (0.49); 0.9389 (0.89); 0.9174 (6.40); 0.9081 (5.02); 0.8945 (1.34); 0.8853 (0.82); 0.8753 (0.70); 0.8534 (0.52); 0.8376 (0.78); 0.8323 (0.56); 0.8224 (1.44); 0.8071 (4.83); 0.7889 (4.82); 0.7662 (0.83); 0.7508 (0.37); 0.0080 (0.39); -0.0002 (12.25); -0.0085 (0.53) | | | | | | | | |
| **No. IIa-26, Solvent: <[D₆]-DMSO >, Spektrometer: 399.95MHz** 8.8717 (15.40); 8.8694 (15.54); 8.4864 (15.85); 8.4825 (15.76); 8.3229 (0.39); 8.2695 (0.42); 8.0777 (0.43); 7.7065 (0.38); 4.1514 (4.63); 4.1387 (3.79); 3.9052 (16.00); 3.6150 (5.91); 3.6033 (6.10); 3.5730 (8.21); 3.5613 (8.15); 3.3562 (72.65); 3.1669 (6.89); 2.6767 (3.03); 2.6721 (4.09); 2.6677 (3.09); 2.5254 (12.41); 2.5119 (232.26); 2.5076 (463.05); 2.5031 (607.50); 2.4986 (454.95); 2.4944 (234.67); 2.3343 (2.90); 2.3298 (3.98); 2.3253 (2.98); 1.2582 (0.35); 1.2486 (0.52); 1.2353 (0.57); 0.8750 (0.45); 0.0079 (0.73); -0.0002 (20.80); -0.0083 (0.88) | | | | | | | | |
| **No. IIa-30, Solvent: <[D₆]-DMSO >, Spektrometer: 399.95MHz** 8.8681 (3.65); 8.8491 (0.41); 8.6288 (0.37); 8.4069 (3.72); 8.4024 (3.41); 8.1943 (0.35); 8.1890 (0.35); 7.9500 (0.92); 7.9366 (1.46); 7.9233 (0.80); 5.5399 (0.79); 3.9050 (5.32); 3.7490 (1.58); 3.7358 (1.88); 3.7276 (1.96); 3.7144 (1.76); 3.4459 (0.40); 3.3410 (284.43); 3.2952 (1.33); 3.2820 (1.17); 3.2585 (1.65); 3.2454 (1.60); 3.1740 (1.00); 3.1609 (0.95); 3.1525 (0.32); 3.1282 (0.50); 3.1135 (1.08); 3.1028 (2.50); 3.0955 (2.31); 3.0819 (4.30); 3.0652 (3.50); 3.0508 (2.32); 3.0456 (2.22); 3.0329 (0.97); 3.0182 (0.41); 2.6765 (0.85); 2.6719 (1.15); 2.6673 (0.85); 2.5252 (4.14); 2.5118 (68.50); 2.5074 (134.85); 2.5029 (175.39); 2.4983 (128.97); 2.4939 (64.49); 2.3341 (1.17); 2.3296 (1.48); 2.3251 (1.19); 1.2350 (0.41); 1.0955 (0.63); 1.0853 (0.53); 1.0773 (1.24); 1.0591 (0.66); 1.0381 (0.44); 1.0300 (0.52); 1.0205 (0.63); 1.0059 (7.75); 0.9879 (16.00); 0.9699 (7.79); 0.9536 (1.46); 0.9356 (0.72); 0.9119 (0.48); 0.8940 (0.60); 0.8761 (0.33); -0.0002 (9.34); -0.0084 (0.44) | | | | | | | | |

**Table E**

| | | | | | | | | |
|---|---|---|---|---|---|---|---|---|
| Compounds of formula (IIb) | | | | | | | | |
| 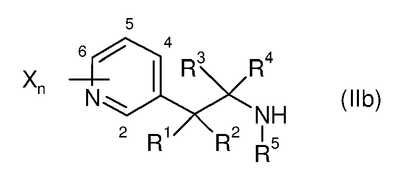 | | | | | | | | |

| **No.** | **Xn** | **R¹** | **R²** | **R³** | **R⁴** | **R⁵** | **optical information** | **Reference of synthesis procedure or phys. chem. data** |
|---|---|---|---|---|---|---|---|---|
| IIb-1 | 6-Cl | H | H | COOH | H | H | | comm. av. CAS-No. 188916-07-6 |
| IIb-2 | 5-Cl 6-OMe | H | H | COOH | H | H | | comm. av. CAS-No. 1270318-19-8 |
| IIb-3 | 5-Cl 6-OMe | H | H | COOMe | H | H | - | according to P1 |
| IIb-4 | 5-Cl 6-OMe | H | H | COOMe | H | H | R-enantiomer | comm. av. CAS-No. 1212838-31-7 |
| Ilb-5 | 5-Cl 6-OMe | H | H | COOMe | H | H | S-enantiomer | comm. av. CAS-No. 1213663-26-3 |
| IIb-6 | 5-Cl 6-OMe | H | H | COOEt | H | H | - | according to P1 |
| IIb-7 | 5-Cl 6-OMe | H | H | CONHMe | H | H | - | according to P1 |
| IIb-8 | 5-Cl 6-OMe | H | H | CONHEt | H | H | - | according to P1 |
| IIb-9 | 6-Cl | H | H | H | H | H | - | P4 |
| IIb-10 | 6-CF₃ | H | H | H | H | H | - | according to P4 comm. av. CAS-No. 765287-34-1 |
| IIb-11 | 2-Cl 4-Cl | H | H | H | H | H | - | according to P4 |
| IIb-12 | 6-Cl | CH₃ | H | H | H | H | | |
| IIb-13 | 6-Cl | CH₃ | CH₃ | H | H | H | - | comm. av. CAS-No. 1060812-06-7 |
| IIb-14 | 6-CH₃ | CH₃ | CH₃ | H | H | H | - | comm. av. CAS-No. 1060806-41-8 |
| IIb-15 | 6-OMe | CH₃ | CH₃ | H | H | H | - | comm. av. CAS-No. 1060807-33-1 |
| IIb-16 | 6-Cl | F | F | H | H | H | - | comm. av. CAS-No. 1204235-05-1 |
| IIb-17 | 6-CF₃ | H | H | CH₃ | H | H | - | comm. av. CAS-No. 910414-32-3 |
| IIb-18 | 2-Cl | H | H | CH₃ | H | H | - | comm. av. CAS-No. 910413-05-7 |
| IIb-19 | 6-Cl | H | H | CH₃ | H | H | | comm. av. CAS-No. 910398-62-8 |
| IIb-20 | 2-OMe 5-Br | H | H | C₂H₅ | H | H | - | comm. av. CAS-No. 910407-42-0 |
| IIb-21 | 6-CF₃ | H | H | C₂H₅ | H | H | - | comm. av. CAS-No. 910386-05-9 |
| IIb-22 | 6-Cl | H | H | C₂H₅ | H | H | - | comm. av. CAS-No. 910387-17-6 |
| IIb-23 | 5-CF3 | CH₂-CH₂ | | H | H | H | - | comm. av. CAS-No. 1245915-95-0 |
| IIb-24 | 6-Cl | CH₂-CH₂ | | H | H | H | - | comm. av. CAS-No. 1060811-84-8 |
| IIb-25 | 2-Br | CH₂-CH₂ | | H | H | H | - | comm. av. CAS-No. 1060811-52-0 |
| IIb-26 | 4-F | CH₂-CH₂ | | H | H | H | - | comm. av. CAS-No. 1060809-53-1 |
| IIb-27 | 6-CF₃ | CH₂-CH₂ | | H | H | H | - | comm. av. CAS-No. 1060811-04-2 |
| IIb-28 | 6-Br | CH₂-CH₂ | | H | H | H | - | comm. av. CAS-No. 1060811-50-8 |
| IIb-29 | 2-OMe | CH₂-CH₂ | | H | H | H | - | comm. av. CAS-No. 1060807-12-6 |
| IIb-30 | 4-CH₃ | CH₂-CH₂ | | H | H | H | - | comm. av. CAS-No. 1060804-94-5 |
| IIb-31 | 5-F | H | (CH₂)₃ | | H | H | | comm. av. CAS-No. 1247729-51-6 |
| IIb-32 | 5-F | H | (CH₂)₄ | | H | H | | comm. av. CAS-No. 1249152-50-8 |
| IIb-33 | 5-F | H | (CH₂)₅ | | H | H | | comm. av. CAS-No. 1247860-65-6 |

**Table F**

| | | | | | | | | |
|---|---|---|---|---|---|---|---|---|
| Compounds of formula (IIc) | | | | | | | | |
| 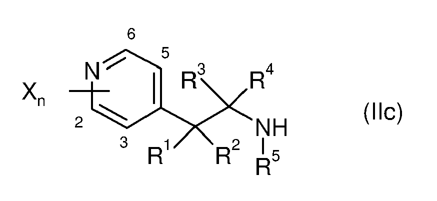 | | | | | | | | |

| **No.** | **Xn** | **R¹** | **R²** | **R³** | **R⁴** | **R⁵** | **Reference of synthesis procedure or phys.chem. data** | **BCS-Nr.** |
|---|---|---|---|---|---|---|---|---|
| IIc-1 | 2-F 3-F 5-F 6-F | H | H | H | H | H | P8 | |
| IIc-2 | 2-Me 5-Cl | H | H | H | H | H | according to P8 | |
| IIc-3 | 2-F 5-F | H | H | H | H | H | according to P8 | |
| IIc-4 | 2-F 3-F 5-F 6-F | H | H | COOH | H | H | comm. av. CAS-No. 1270314-19-6 | |
| IIc-5 | 2-F 3-F 5-F 6-F | H | (CH₂)₄ | | H | H | according to P5 | |
| IIc-6 | 3-F 5-F | H | H | H | H | H | comm. av. CAS-No. 1314907-15-7 | |
| IIc-7 | 2-CF₃ 3-OMe | H | H | H | H | H | comm. av. CAS-No. 1211539-23-9 | |
| IIc-8 | 2-CF₃ | H | H | H | H | H | comm. av. CAS-No. 1060811-11-1 | |
| IIc-9 | 3-Cl 5-Cl | H | H | H | H | H | comm. av. CAS-No. 910391-44-5 | |
| IIc-10 | 3-Cl | H | H | H | H | H | comm. av. CAS-No. 910410-77-4 | |
| IIc-11 | 2-OMe | H | H | H | H | H | comm. av. CAS-No. 764708-27-2 | |
| IIc-12 | 2-Me | H | H | H | H | H | comm. av. CAS-No. 625438-03-1 | |
| IIc-13 | 2-Cl | H | H | H | H | H | comm. av. CAS-No. 910388-12-4 | |
| IIc-14 | 3-Cl 5-Cl | H | H | CH₃ | H | H | comm. av. CAS-No. 910389-65-0 | |
| IIc-15 | 3-F 5-Fl | H | H | CH₃ | H | H | comm. av. CAS-No. 910402-24-3 | |
| IIc-16 | 3-Cl 6-Cl | H | H | CH₃ | H | H | comm. av. CAS-No. 910407-17-9 | |
| IIc-17 | 3-F | H | H | CH₃ | H | H | comm. av. CAS-No. 910389-92-3 | |
| IIc-18 | 3-Cl | H | H | CH₃ | H | H | comm. av. CAS-No. 910389-80-9 | |
| IIc-19 | 2-OMe | CH₃ | CH₃ | H | H | H | comm. av. CAS-No. 1060807-35-3 | |
| IIc-20 | 2-OMe | CH₂-CH₂ | | H | H | H | comm. av. CAS-No. 1060807-10-4 | |
| IIc-21 | 2-Me | CH₂-CH₂ | | H | H | H | comm. av. CAS-No. 1060806-23-6 | |
| IIc-22 | 2-CF₃ | CH₂-CH₂ | | H | H | H | comm. av. CAS-No. 1060811-06-4 | |
| IIc-23 | 2-Cl | CH₂-CH₂ | | H | H | H | comm. av. CAS-No. 1060811-85-9 | |
| IIc-24 | 3-Cl 5-Cl | H | H | COOH | H | H | comm. av. CAS-No. 1270326-34-5 | |
| IIc-25 | 2-Me | CH₃ | CH₃ | H | H | H | comm. av. CAS-No. 1060806-42-9 | |
| IIc-26 | 2-Cl | CH₃ | CH₃ | H | H | H | comm. av. CAS-No. 1060812-07-8 | |

## Claims

1. Use of a compound of formula (I) 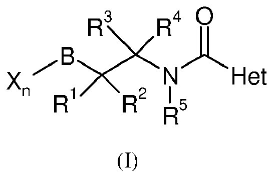 wherein
B represents 2-pyridyl, 3-pyridyl, or 4-pyridyl,
X is selected from the group consisting of halogen, nitro, cyano, hydroxy, amino, -SH, -SF₅, -CHO, -OCHO, -NHCHO, -COOH, -CONH₂, -CONH(OH), -OCONH₂, (hydroxyimino)-C₁-C₆-alkyl, C₁-C₈-alkyl, C₁-C₈-halogenoalkyl having 1 to 5 halogen atoms, C₂-C₈-alkenyl, C₂-C₈-alkynyl, C₁-C₈-alkylamino, di-(C₁-C₈-alkyl)amino, C₁-C₈-alkoxy, C₁-C₈-halogenoalkoxy having 1 to 5 halogen atoms,, C₂-C₈-alkenyloxy, C₂-C₈-halogenoalkenyloxy having 1 to 5 halogen atoms, C₃-C₈-alkynyloxy, C₃-C₈-halogenoalkynyloxy having 1 to 5 halogen atoms, C₃-C₈-cycloalkyl, C₃-C₈-halogenocycloalkyl having 1 to 5 halogen atoms, C₁-C₈-alkylcarbonyl, C₁-C₈-halogenoalkylcarbonyl having 1 to 5 halogen atoms, -CONH(C₁-C₈-alkyl), -CON(C₁-C₈-alkyl)₂, -CONH(OC₁-C₈-alkyl), -CON(OC₁-C₈-alkyl)(C₁-C₈-alkyl), C₁-C₈-alkoxycarbonyl, C₁-C₈-halogenoalkoxycarbonyl having 1 to 5 halogen atoms, C₁-C₈-alkylcarbonyloxy, C₁-C₈-halogenoalkylcarbonyloxy having 1 to 5 halogen atoms, C₁-C₈-alkylcarbonylamino, C₁-C₈-halogenoalkylcarbonylamino having 1 to 5 halogen atoms, -OCONH(C₁-C₈-alkyl), -OCON(C₁-C₈-alkyl)₂, -OCONH(OC₁-C₈-alkyl), -OCO(OC₁-C₈-alkyl), -S-C₁-C₈-alkyl, -S-C₁-C₈-halogenoalkyl having 1 to 5 halogen atoms, -S(O)-C₁-C₈-alkyl, -S(O)-C₁-C₈-halogenoalkyl having 1 to 5 halogen atoms, -S(O)₂-C₁-C₈-alkyl, -S(O)₂-C₁-C₈-halogenoalkyl having 1 to 5 halogen atoms, (C₁-C₆-alkoxyimino)-C₁-C₆-alkyl, (C₂-C₆-alkenyloxyimino)-C₁-C₆-alkyl, (C₃-C₆-alkynyloxyimino)-C₁-C₆-alkyl, (benzyloxyimino)-C₁-C₆-alkyl, benzyloxy, -S-benzyl, benzylamino, phenoxy, -S-phenyl and phenylamino;
n is 1, 2, 3 or 4 and if n is 2, 3, or 4, then the substituents X may be the same or different;
R¹ and R² are the same or different and are selected from the group consisting of hydrogen, halogen, cyano, hydroxy, amino, -SH, -CHO, -OCHO, -NHCHO, -COOH, -CONH₂, -CONH(OH), -OCONH₂, a (hydroxyimino)-C₁-C₆-alkyl group, C₁-C₆-alkyl, C₂-C₆-alkenyl, C₂-C₆-alkynyl, C₁-C₆-alkylamino, di-(C₁-C₆-alkyl)amino, C₁-C₆-alkoxy, C₁-C₆-halogenoalkyl having 1 to 5 halogen atoms, C₁-C₆-halogenoalkoxy having 1 to 5 halogen atoms,, C₂-C₆-alkenyloxy, C₂-C₆-halogenoalkenyloxy having 1 to 5 halogen atoms, C₃-C₆-alkynyloxy, C₃-C₆-halogenoalkynyloxy having 1 to 5 halogen atoms, C₃-C₆-cycloalkyl, C₃-C₆-halogenocycloalkyl having 1 to 5 halogen atoms, C₃-C₆-cycloalkyl-C₁-C₆-alkyl, C₃-C₆-halogenocycloalkyl-C₁-C₆-alkyl having 1 to 5 halogen atoms, C₁-C₆-alkylcarbonyl, C₁-C₆-halogenoalkylcarbonyl having 1 to 5 halogen atoms, -CONH(C₁-C₆-alkyl), -CON(C₁-C₆-alkyl)₂, -CONH(OC₁-C₆-alkyl), -CON(OC₁-C₆-alkyl)(C₁-C₆-alkyl), C₁-C₆-alkoxycarbonyl, a C₁-C₆-halogenoalkoxycarbonyl having 1 to 5 halogen atoms, -OC(O)-C₁-C₆-alkyl, -OC(O)-C₁-C₆-halogenoalkyl having 1 to 5 halogen atoms, -NHC(O)-C₁-C₆-alkyl, -NHC(O)-C₁-C₆-halogenoalkyl having 1 to 5 halogen atoms, -OCONH(C₁-C₆-alkyl), -OCON(C₁-C₆-alkyl)₂, -OCONH(OC₁-C₆-alkyl), OCO(OC₁-C₆-alkyl), -S-C₁-C₆-alkyl, -S-C₁-C₆-halogenoalkyl having 1 to 5 halogen atoms, -S(O)-C₁-C₆-alkyl, -S(O)-C₁-C₆-halogenoalkyl having 1 to 5 halogen atoms, -S(O)₂-C₁-C₆-alkyl, -S(O)₂-C₁-C₆-halogenoalkyl having 1 to 5 halogen atoms, benzyl, benzyloxy, -S-benzyl, -S(O)-benzyl, -S(O)₂-benzyl, benzylamino, phenoxy, -S-phenyl, -S(O)-phenyl, -S(O)₂-phenyl, phenylamino, phenylcarbonylamino, 2,6-dichlorophenyl-carbonylamino, 2-chlorophenyl-carbonylamino and phenyl;
R³ and R⁴ are the same or different and are selected from the group consisting of hydrogen, halogen, cyano, hydroxy, amino, -SH, -CHO, -COOH, -CONH₂, -CONH(OH), -OCONH₂, (hydroxyimino)-C₁-C₆-alkyl, C₁-C₆-alkyl, C₂-C₆-alkenyl, C₂-C₆-alkynyl, C₁-C₆-alkylamino, di-(C₁-C₆-alkyl)amino, C₁-C₆-alkoxy, hydroxy-C₁-C₆-alkyl, C₁-C₆-alkoxy-C₁-C₆-alkyl, C₁-C₆-halogenoalkyl having 1 to 5 halogen atoms, C₁-C₆-halogenoalkoxy having 1 to 5 halogen atoms, C₂-C₆-alkenyloxy, C₂-C₆-halogenoalkenyloxy having 1 to 5 halogen atoms, C₃-C₆-alkynyloxy, C₃-C₆-halogenoalkynyloxy having 1 to 5 halogen atoms, C₃-C₆-cycloalkyl, C₃-C₆-halogenocycloalkyl having 1 to 5 halogen atoms, C₁-C₆-alkylcarbonyl, C₁-C₆-halogenoalkylcarbonyl having 1 to 5 halogen atoms, -CONH(C₁-C₆-alkyl), -CON(C₁-C₆-alkyl)₂, -CONH(OC₁-C₆-alkyl), -CON(OC₁-C₆-alkyl)(C₁-C₆-alkyl), C₁-C₆-alkoxycarbonyl, C₁-C₆-halogenoalkoxycarbonyl having 1 to 5 halogen atoms, -OC(O)-C₁-C₆-alkyl, -OC(O)-C₁-C₆-halogenoalkyl having 1 to 5 halogen atoms, -NHC(O)-C₁-C₆-alkyl, -NHC(O)-C₁-C₆-halogenoalkyl having 1 to 5 halogen atoms, -OCONH(C₁-C₆-alkyl), -OCON(C₁-C₆-alkyl)₂, -OCONH(OC₁-C₆-alkyl), OCO(OC₁-C₆-alkyl), -S-C₁-C₆-alkyl, -S-C₁-C₆-halogenoalkyl having 1 to 5 halogen atoms, -S(O)-C₁-C₆-alkyl, -S(O)-C₁-C₆-halogenoalkyl having 1 to 5 halogen atoms, -S(O)₂-C₁-C₆-alkyl, -S(O)₂-C₁-C₆-halogenoalkyl having 1 to 5 halogen atoms, benzyl, benzyloxy, -S-benzyl, -S(O)-benzyl, -S(O)₂-benzyl, benzylamino, phenoxy, -S-phenyl, -S(O)-phenyl, -S(O)₂-phenyl, phenylamino, phenylcarbonylamino, 2-chlorophenyl-carbonylamino, 2,6-dichlorophenyl-carbonylamino and phenyl;
R⁵ is selected from the group consisting of hydrogen, cyano, -CHO, -OH, C₁-C₆-alkyl, C₁-C₆-halogenoalkyl having 1 to 5 halogen atoms, C₁-C₆-alkoxy, C₁-C₆-halogenoalkoxy having 1 to 5 halogen atoms, C₃-C₇-cycloalkyl, C₃-C₇-halogenocycloalkyl having 1 to 5 halogen atoms, C₃-Crcycloalkyl-C₁-C₆-alkyl, -CONH(C₁-C₆-alkyl), C₂-C₆-alkenyl, C₂-C₆-alkynyl, Cₗ-C₆-alkoxy-Ci-C6-alkyl, C₃-C₇-cycloalkyl-C₁-C₆-alkyl, cyano-C₁-C₆-alkyl, amino-C₁-C₆-alkyl, C₁-C₆-alkylamino-C₁-C₆-alkyl, di-(C₁-C₆-alkyl)amino-C₁-C₆-alkyl, C₁-C₆-alkylcarbonyl, C₁-C₆-halogenoalkylcarbonyl having 1 to 5 halogen atoms, C₁-C₆-alkoxycarbonyl, C₁-C₆-benzyloxycarbonyl, C₁-C₆-alkoxy-C₁-C₆-alkylcarbonyl, -S-C₁-C₆-alkyl, -S-C₁-C₆-halogenoalkyl having 1 to 5 halogen atoms, -S(O)₂-C₁-C₆-alkyl, and -S(O)₂-C₁-C₆-halogenoalkyl having 1 to 5 halogen atoms;
with the proviso, that when B = 2-pyridyl, then
Het may not be pyrazinyl (Het ≠ Het-29), and
when B = 2-pyridyl and Het = pyridyl or pyrazolyl, then
at least one of R¹, R², R³, R⁴ and R⁵ is not a hydrogen atom, and
R¹ and R² may also together with the carbon atom to which they are bonded form a 3-, 4-, 5- or 6-membered carbocycle, or
R² and R³ may together with the carbon atoms to which they are bonded form a 3-, 4-, 5-, 6- or 7-membered carbocycle, and
with the further proviso, that when B = 3-pyridyl, then
R¹ and R² may also together with the carbon atom to which they are bonded form a 3-, 4-, 5- or 6-membered carbocycle, or
R³ and R⁴ may also together with the carbon atoms to which they are bonded form a 3-, 4-, 5- or 6-membered carbocycle, and
with the further proviso, that when B = 4-pyridyl, then
R¹ and R² may also together with the carbon atom to which they are bonded form a 3-, 4-, 5- or 6-membered carbocycle, or
R³ and R⁴ may also together with the carbon atoms to which they are bonded form a 3-, 4-, 5- or 6-membered carbocycle;
Het represents a heterocycle of the formula (Het-1) 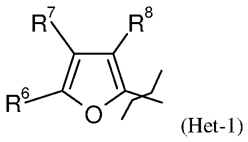 in which
R⁶ and R⁷ may be the same or different and are selected from the group consisting of hydrogen, halogen, amino, nitro, C₁-C₄-alkyl and C₁-C₄-halogenoalkyl having 1 to 5 halogen atoms, and
R⁸ is selected from the group consisting of hydrogen, halogen, nitro, C₁-C₄-alkyl and C₁-C₄-halogenoalkyl having 1 to 5 halogen atoms, or
Het represents a heterocycle of the formula (Het-2) 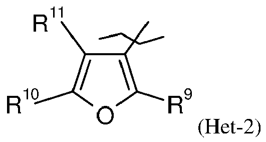 in which
R⁹ is selected from the group consisting of hydrogen, halogen, C₁-C₄-alkyl and C₁-C₄-halogenoalkyl having 1 to 5 halogen atoms, and
R¹⁰ and R¹¹ may be the same or different and are selected from the group consisting of hydrogen, halogen, amino, C₁-C₄-alkyl, C₁-C₄-halogenoalkyl having 1 to 5 halogen atoms and phenyl (optionally substituted by halogen or C₁-C₄-alkyl), or
Het represents a heterocycle of the formula (Het-3) 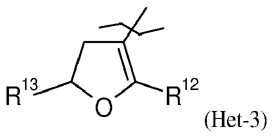 in which
R¹² is selected from the group consisting of halogen, C₁-C₄-alkyl and C₁-C₄-halogenoalkyl having 1 to 5 halogen atoms, and
R¹³ is selected from the group consisting of hydrogen, C₁-C₄-alkyl and C₁-C₄-halogenoalkyl having 1 to 5 halogen atoms, or
Het represents a heterocycle of the formula (Het-4) 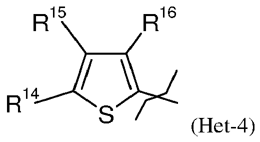 in which
R¹⁴ and R¹⁵ may be the same or different and are selected from the group consisting of hydrogen, halogen, C₁-C₄-alkyl, C₁-C₄-halogenoalkyl having 1 to 5 halogen atoms, -S-Ci-C4₋alkyl, -S(O)₂-C₁-C₄-alkyl, phenyl optionally substituted by halogen or C₁-C₄-alkyl and pyridyl (optionally substituted by halogen or C₁-C₄-alkyl), and
R¹⁶ is selected from the group consisting of hydrogen, halogen, cyano, C₁-C₄-alkyl, C₁-C₄-halogenoalkyl having 1 to 5 halogen atoms and C₁-C₄-halogenoalkoxy having 1 to 5 halogen atoms, or
Het represents a heterocycle of the formula (Het-5) 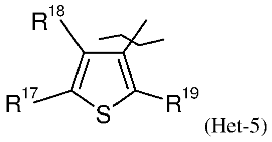 in which
R¹⁷ and R¹⁸ may be the same or different and are selected from the group consisting of hydrogen, halogen, C₁-C₄-alkyl, C₁-C₄-alkyloxy and C₁-C₄-halogenoalkyl having 1 to 5 halogen atoms, and
R¹⁹ is selected from the group consisting of hydrogen, halogen, C₁-C₄-alkyl and C₁-C₄-halogenoalkyl having 1 to 5 atoms, or
Het represents a heterocycle of the formula (Het-6) 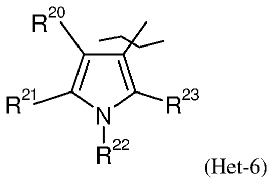 in which
R²⁰ is selected from the group consisting of hydrogen, halogen, cyano, C₁-C₄-alkyl and C₁-C₄-halogenoalkyl having 1 to 5 halogen atoms, and
R²¹ and R²³ may be the same or different and are selected from the group consisting of hydrogen, halogen, C₁-C₄-alkyl and C₁-C₄-halogenoalky having 1 to 5 halogen atoms, and
R²² is selected from the group consisting of hydrogen, cyano, C₁-C₄-alkyl, C₁-C₄-halogenoalkyl having 1 to 5 halogen atoms, C₁-C₄-alkoxy-C₁-C₄-alkyl, hydroxyl-C₁-C₄-alkyl, -S(O)₂-C₁-C₄-alkyl, -S(O)₂-N(C₁-C₄-alkyl)₂, C₁-C₆-alkylcarbonyl, -S(O)₂-phenyl (optionally substituted by halogen or C₁-C₄-alkyl) and benzoyl (optionally substituted by halogen or C₁-C₄-alkyl), or
Het represent a heterocycle of the formula (Het-7) 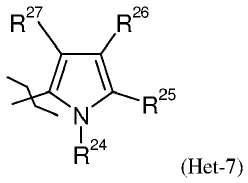 in which
R²⁴ is selected from the group consisting of hydrogen, cyano, C₁-C₄-alkyl, C₁-C₄-halogenoalkyl having 1 to 5 halogen atoms, C₁-C₄-alkoxy-C₁-C₄-alkyl, hydroxy-C₁-C₄-alkyl, -S(O)₂-C₁-C₄-alkyl, -S(O)₂-N(C₁-C₄-alkyl)₂, C₁-C₆-alkylcarbonyl, -S(O)₂-phenyl (optionally substituted by halogen or C₁-C₄-alkyl) and benzoyl (optionally substituted by halogen or a C₁-C₄-alkyl), and
R²⁵, R²⁶ and R²⁷ may be the same or different and are selected from the group consisting of hydrogen, halogen, cyano, C₁-C₄-alkyl, C₁-C₄-halogenalkyl having 1 to 5 halogen atoms and C₁-C₄-alkylcarbonyl, or
Het represents a heterocycle of the formula (Het-8) 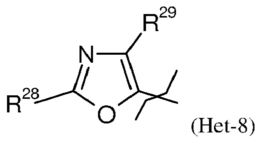 in which
R²⁸ is selected from the group consisting of hydrogen and C₁-C₄-alkyl, and
R²⁹ is selected from the group consisting of halogen, C₁-C₄-alkyl and C₁-C₄-halogenoalkyl having 1 to 5 halogen atoms, or
Het represent a heterocycle of the formula (Het-9) 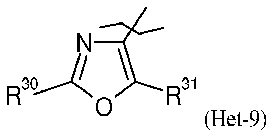 in which
R³⁰ is selected from the group consisting of hydrogen and C₁-C₄-alkyl, and
R³¹ is selected from the group consisting of halogen, C₁-C₄-alkyl, C₁-C₄-halogenoalkyl having 1 to 5 halogen atoms and phenyl (optionally substituted by halogen or C₁-C₄-alkyl), or
Het represent a heterocycle of the formula (Het-10) 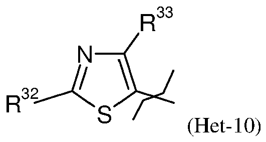 in which
R³² is selected from the group consisting of hydrogen, halogen, amino, cyano, C₁-C₄-alkylamino, di-(C₁-C₄-alkyl)amino, C₁-C₄-alkyl, C₁-C₄-halogenoalkyl having 1 to 5 halogen atoms and phenyl (optionally substituted by halogen or C₁-C₄-alkyl), and
R³³ is selected from the group consisting of halogen, C₁-C₄-alkyl and C₁-C₄-halogenoalkyl having 1 to 5 halogen atoms, or
Het represents a heterocycle of the formula (Het-11) 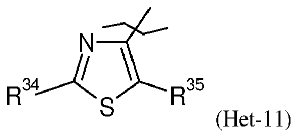 in which
R³⁴ is selected from the group consisting of hydrogen, halogen, amino, cyano, C₁-C₄-alkylamino, di-(C₁-C₄-alkyl)amino, C₁-C₄-alkyl and C₁-C₄-halogenoalkyl having 1 to 5 halogen atoms, and
R³⁵ is selected from the group consisting of halogen, C₁-C₄-alkyl and C₁-C₄-halogenoalkyl having 1 to 5 halogen atoms, or
Het represents a heterocycle of the formula (Het-12) 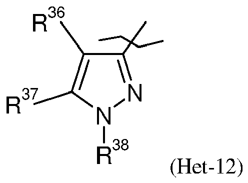 in which
R³⁶ is selected from the group consisting of hydrogen, halogen, cyano, nitro, C₁-C₄-alkyl, C₁-C₄-halogenoalkyl having 1 to 5 halogen atoms, C₃-C₆-cycloalkyl, C₁-C₄-alkoxy, C₁-C₄-halogenoalkoxy having 1 to 5 halogen atoms, -S-C₁-C₄-alkyl, -S-C₁-C₄-halogenoalkyl having 1 to 5 halogen atoms, aminocarbonyl and aminocarbonyl-C₁-C₄-alkyl, and
R³⁷ ist selected from the group consisting of hydrogen, halogen, cyano, nitro, C₁-C₄-alkyl, C₁-C₄-alkoxy and -S-C₁-C₄-alkyl, and
R³⁸ is selected from the group consisting of hydrogen, phenyl, C₁-C₄-alkyl, C₁-C₄-halogenoalkyl having 1 to 5 halogen atoms, hydroxy-C₁-C₄-alkyl, C₂-C₆-alkenyl, C₃-C₆-cycloalkyl, C₁-C₄-alkylthio-C₁-C₄-alkyl, C₁-C₄-halogenoalkylthio-C₁-C₄-alkyl having 1 to 5 halogen atoms, C₁-C₄-alkoxy-C₁-C₄-alkyl and C₁-C₄-halogenoalkoxy-C₁-C₄-alkyl having 1 to 5 halogen atoms, or
Het represents a heterocycle of the formula (Het-13) 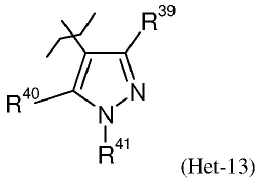 in which
R³⁹ is selected from the group consisting of hydrogen, halogen, cyano, nitro, C₁-C₄-alkyl, C₁-C₄-halogenoalkyl having 1 to 5 halogen atoms, C₃-C₆-cycloalkyl, C₁-C₄-alkoxy, C₁-C₄-halogenoalkoxy having 1 to 5 halogen atoms, -S-C₁-C₄-alkyl, -S-C₁-C₄-halogenoalkyl having 1 to 5 atoms, aminocarbonyl and aminocarbonyl-C₁-C₄-alkyl, and
R⁴⁰ is selected from the group consisting of hydrogen, halogen, cyano, C₁-C₄-alkyl, C₁-C₄-alkoxy, C₁-C₄-halogenoalkoxy having 1 to 5 halogen atoms and -S-C₁-C₄-alkyl, and
R⁴¹ is selected from the group consisting of hydrogen, C₁-C₄-alkyl, C₁-C₄-halogenoalkyl having 1 to 5 halogen atoms, hydroxy-C₁-C₄-alkyl, C₂-C₆-alkenyl, C₃-C₆-cycloalkyl, C₁-C₄-alkylthio-C₁-C₄-alkyl, C₁-C₄-halogenoalkylthio-C₁-C₄-alkyl having 1 to 5 halogen atoms, C₁-C₄-alkoxy-C₁-C₄- alkyl, C₁-C₄-halogenoalkoxy-C₁-C₄-alkyl having 1 to 5 halogen atoms and phenyl (optionally substituted by halogen, C₁-C₄-alkyl, C₁-C₄-alkoxy-C₁-C₄-alkyl or nitro), or
Het represents a heterocycle of the formula (Het-14) 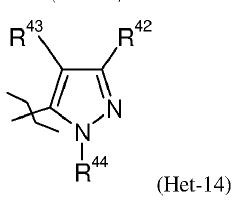 in which
R⁴² is selected from the group consisting of hydrogen, halogen, cyano, nitro, C₁-C₄-alkyl, C₁-C₄-halogenoalkyl having 1 to 5 halogen atoms, C₃-C₆-cycloalkyl, C₁-C₄-alkoxy, C₁-C₄-halogenoalkoxy having 1 to 5 halogen atoms, -S-C₁-C₄-alkyl, -S-C₁-C₄-halogenoalkyl having 1 to 5 halogen atoms, aminocarbonyl and aminocarbonyl-C₁-C₄-alkyl, and
R⁴³ is selected from the group consisting of hydrogen, halogen, cyano, C₁-C₄-alkyl, C₁-C₄-alkoxy, -S-C₁-C₄-alkyl and C₁-C₄-halogenoalkyl having 1 to 5 halogen atoms, and
R⁴⁴ is selected from the group consisting of hydrogen, phenyl, benzyl, C₁-C₄-alkyl, C₁-C₄-halogenoalkyl having 1 to 5 halogen atoms, hydroxy-C₁-C₄-alkyl, C₂-C₆-alkenyl, C₃-C₆-cycloalkyl, C₁-C₄-alkylthio-C₁-C₄-alkyl, C₁-C₄-halogenoalkylthio-C₁-C₄-alkyl having 1 to 5 halogen atoms, C₁-C₄-alkoxy-C₁-C₄-alkyl and C₁-C₄-halogenoalkoxy-C₁-C₄-alkyl having 1 to 5 halogen atoms, or
Het represents a heterocycle of the formula (Het-15) 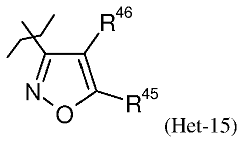 in which
R⁴⁵ and R⁴⁶ may be the same or different and are selected from the group consisting of hydrogen, halogen, C₁-C₄-alkyl and C₁-C₄-halogenoalkyl having 1 to 5 halogen atoms, or
Het represents a heterocycle of the formula (Het-16) 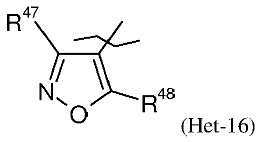 in which
R⁴⁷ and R⁴⁸ may be the same or different and are selected from the group consisting of hydrogen, halogen, C₁-C₄-alkyl, C₁-C₄-halogenoalkyl having 1 to 5 halogen atoms, phenyl (optionally substituted by halogen or a C₁-C₄-alkyl), and heterocyclyl like pyridyl, pyrimidinyl and thiadiazolyl (each optionally substituted by halogen or C₁-C₄-alkyl), or
Het represents a heterocycle of the formula (Het-17) 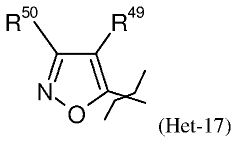 in which
R⁴⁹ and R⁵⁰ may be the same or different and are selected from the group consisting of hydrogen, halogen, C₁-C₄-alkyl and C₁-C₄-halogenoalkyl having 1 to 5 halogen atoms, or
Het represents a heterocycle of the formula (Het-18) 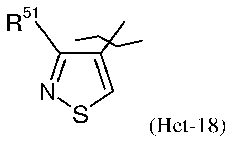 in which
R⁵¹ is selected from the group consisting of halogen, C₁-C₄-alkyl and C₁-C₄-halogenoalkyl having 1 to 5 halogen atoms, or
Het represents a heterocycle of the formula (Het-19) 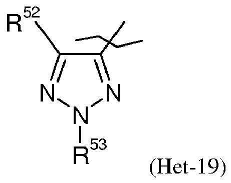 in which
R⁵² is selected from the group consisting of halogen, C₁-C₄-alkyl and C₁-C₄-halogenoalkyl having 1 to 5 halogen atoms, and
R⁵³ is selected from the group consisting of hydrogen, C₁-C₄-alkyl, C₁-C₄-halogenoalkyl having 1 to 5 halogen atoms and phenyl (optionally substituted by halogen or C₁-C₄-alkyl), or
Het represents a heterocycle of the formula (Het-20) 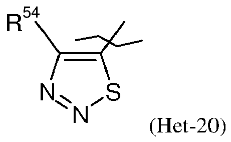 in which
R⁵⁴ is selected from the group consisting of halogen, C₁-C₄-alkyl and C₁-C₄-halogenoalkyl having 1 to 5 halogen atoms, or
Het represents a heterocycle of the formula (Het-21) 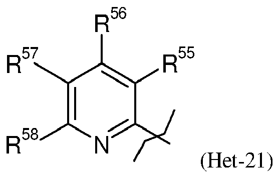 in which
R⁵⁵ is selected from the group consisting of hydrogen, halogen, hydroxy, cyano, C₁-C₄-alkyl, C₁-C₄-halogenoalkyl having 1 to 5 halogen atoms, C₁-C₄-alkoxy, -S-C₁-C₄-alkyl, -S-C₁-C₄-halogenoalkyl having 1 to 5 halogen atoms and C₁-C₄-halogenoalkoxy having 1 to 5 halogen atoms, and
R⁵⁶, R⁵⁷ and R⁵⁸, which may be the same or different, are selected from the group consisting of hydrogen, halogen, cyano, C₁-C₄-alkyl, C₁-C₄-halogenoalkyl having 1 to 5 halogen atoms, C₁-C₄-alkoxy, -S-C₁-C₄-alkyl, C₁-C₄-halogenoalkoxy having 1 to 5 halogen atoms, -S(O)-C₁-C₄-alkyl and -S(O)₂-C₁-C₄-alkyl, or
Het represents a heterocycle of the formula (Het-22) 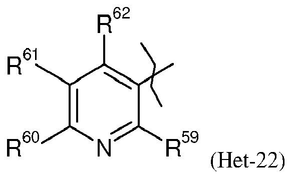 in which
R⁵⁹ is selected from the group consisting of hydrogen, halogen, hydroxy, cyano, C₁-C₄-alkyl, C₁-C₄-halogenoalkyl having 1 to 5 halogen atoms, C₁-C₄ alkoxy, -S-C₁-C₅-alkyl, -S-C₂-C₅-alkenyl, -S-C₁-C₄-halogenoalkyl having 1 to 5 halogen atoms, C₁-C₄-halogenoalkoxy having 1 to 5 halogen atoms, phenyloxy (optionally substituted by halogen or C₁-C₄-alkyl) and -S-phenyl (optionally substituted by halogen or C₁-C₄-alkyl), and
R⁶⁰, R⁶¹ and R⁶², which may the same or different, are selected from the group consisting of hydrogen, halogen, cyano, C₁-C₄-alkyl, C₁-C₄-halogenoalkyl having 1 to 5 halogen atoms, C₁-C₄-alkoxy, -S-C₁-C₄-alkyl, C₁-C₄-halogenoalkoxy having 1 to 5 halogen atoms, -S(O)-C₁-C₄-alkyl, -S(O)₂-C₁-C₄-alkyl, N-morpholine optionally substituted by halogen or C₁-C₄-alkyl, and thienyl (optionally substituted by halogen or a C₁-C₄-alkyl), or
Het represents a heterocycle of the formula (Het-23) 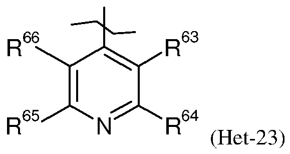 in which
R⁶³, R⁶⁴, R⁶⁵ and R⁶⁶, which may be the same or different, are selected from the group consisting of hydrogen, halogen, hydroxy, cyano, C₁-C₄-alkyl, C₁-C₄-halogenoalkyl having 1 to 5 halogen atoms, C₁-C₄-alkoxy, -S-C₁-C₄-alkyl, -S-C₁-C₄-halogenoalkyl having 1 to 5 halogen atoms, C₁-C₄-halogenoalkoxy having 1 to 5 halogen atoms, -S(O)-C₁-C₄-alkyl and -S(O)₂-C₁-C₄-alkylsulphonyl, or
Het represents a heterocycle of the formula (Het-24) 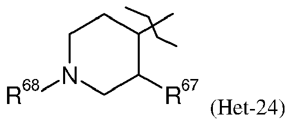 in which
R⁶⁷ is selected from the group consisting of hydrogen, halogen, C₁-C₄-alkyl and C₁-C₄-halogenoalkyl having 1 to 5 halogen atoms, and
R⁶⁸ is selected from the group consisting of hydrogen, C₁-C₄-alkyl, C₁-C₄-halogenoalkyl having 1 to 5 halogen atoms, C₁-C₆-alkoxycarbonyl, benzyl (optionally substituted by 1 to 3 halogen atoms), benzyloxycarbonyl (optionally substituted by 1 to 3 halogen atoms), and heterocyclyl like pyridyl and pyrimidinyl (each optionally substituted by halogen, C₁-C₄-alkyl, C₁-C₄-halogenoalkyl having 1 to 5 halogen atoms), or
Het represents a heterocycle of the formula (Het-25) 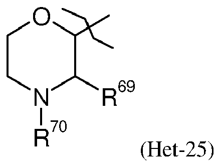 in which
R⁶⁹ is selected from the group consisting of hydrogen, halogen, hydroxy, cyano, C₁-C₄-alkyl, C₁-C₄-halogenoalkyl having 1 to 5 halogen atoms, C₁-C₄-alkoxy, -S-C₁-C₄-alkyl, -S-C₁-C₄-halogenoalkyl having 1 to 5 halogen atoms and C₁-C₄-halogenoalkoxy having 1 to 5 halogen atoms, and
R⁷⁰ is selected from the group consisting of hydrogen, C₁-C₄-alkyl, C₁-C₄-halogenoalkyl having 1 to 5 halogen atoms, and benzyl, or
Het represents a heterocycle of the formula (Het-26) 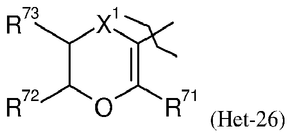 in which
X¹ is selected from the group consisting of sulphur, -SO-, -SO₂- and -CH₂-, and
R⁷¹ is selected from the group consisting of C₁-C₄-alkyl and C₁-C₄-halogenoalkyl having 1 to 5 halogen atoms, and
R⁷² and R⁷³ may be the same or different and are selected from the group consisting of hydrogen and C₁-C₄-alkyl, or
Het represents a heterocycle of the formula (Het-27) 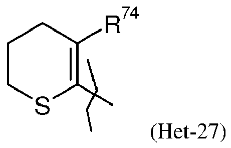 in which
R⁷⁴ is selected from the group consisting of C₁-C₄-alkyl and C₁-C₄-halogenoalkyl having 1 to 5 halogen atoms, or
Het represents a heterocycle of the formula (Het-28) 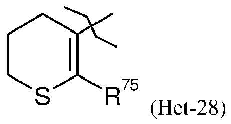 in which
R⁷⁵ is selected from the group consisting of C₁-C₄-alkyl and C₁-C₄-halogenoalkyl having 1 to 5 halogen atoms, or
Het represents a heterocycle of the formula (Het-29) 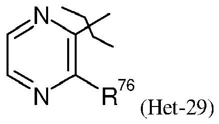 in which
R⁷⁶ is selected from the group consisting of hydrogen, halogen, C₁-C₄-alkyl and C₁-C₄-halogenoalkyl having 1 to 5 halogen atoms
against nematodes.
